# EUROPEAN PATENT APPLICATION

(11) **EP 3 865 575 A1**
(43) Date of publication of application: **18.08.2021**
(21) Application number: 21157083.3
(22) Date of filing: 30.10.2014
(51) Int. Cl.: C12N 15/09, A01K 67/027, C12N 1/15, C12N 1/19, C12N 1/21, C12N 5/10, C12N 15/90, C12N 15/85, C12N 15/10

(54) **VECTOR FOR NUCLEIC ACID INSERTION**

(30) Priority: 06.11.2013 JP 2013230349
(62) Divisional of application: 14860703.9
(71) Applicant: Hiroshima University, Higashihiroshima-shi Hiroshima 739-8511 (JP)
(72) Inventor: YAMAMOTO, Takashi, Hiroshima 739-8526 (JP); SUZUKI, Kenichi, Hiroshima 739-8526 (JP); SAKUMA, Tetsushi, Hiroshima 739-8526 (JP); SAKANE, Yuto, Hiroshima 739-8526 (JP)
(74) Representative: Boult Wade Tennant LLP

(57) **Abstract**

The present invention provides the following: a vector for inserting a desired nucleic acid into a predetermined site of a nucleic acid comprising a region formed of a first nucleotide sequence, the predetermined site, and a region composed of a second nucleotide sequence, in the stated order in the 5'-to-3' direction, wherein the vector comprises a region formed of the first nucleotide sequence, the desired nucleic acid, and the second nucleotide sequence in the stated order in the 5'-to-3' direction; a kit that includes this vector; a method of inserting a nucleic acid comprising a step for introducing this vector into a cell; a cell acquired by this method; and an organism comprising this cell.

SEQUENCE LISTING

<110>  HIROSHIMA UNIVERSITY

<120>  A vector for inserting a nucleic acid

<130>  NLW/EOR/P147377EP01

<150>  JP 2013-230349
<151>  2013-11-06

<160>  96

<170>  PatentIn version 3.5

<210>  1
<211>  8465
<212>  DNA
<213>  Artificial Sequence

<220>
<223>  Left TALEN


<220>
<221>  CDS
<222>  (5131)..(8433)

<400>  1
agccatctgt tgtttgcccc tcccccgtgc cttccttgac cctggaaggt gccactccca      60
ctgtcctttc ctaataaaat gaggaaattg catcacaaca ctcaacccta tctcggtcta     120
ttcttttgat ttataaggga ttttgccgat ttcggcctat tggttaaaaa atgagctgat     180
ttaacaaaaa tttaacgcga attaattctg tggaatgtgt gtcagttagg gtgtggaaag     240
tccccaggct ccccagcagg cagaagtatg caaagcatgc atctcaatta gtcagcaacc     300
aggtgtggaa agtccccagg ctccccagca ggcagaagta tgcaaagcat gcatctcaat     360
tagtcagcaa ccatagtccc gcccctaact ccgcccatcc cgcccctaac tccgcccagt     420
tccgcccatt ctccgcccca tggctgacta atttttttta tttatgcaga ggccgaggcc     480
gcctctgcct ctgagctatt ccagaagtag tgaggaggct tttttggagg cctaggcttt     540
tgcaaaaagc tcccgggagc ttgtatatcc attttcggat ctgatcagca cgtgatgaaa     600
aagcctgaac tcaccgcgac gtctgtcgag aagtttctga tcgaaaagtt cgacagcgtt     660
tccgacctga tgcagctctc ggagggcgaa gaatctcgtg ctttcagctt cgatgtagga     720
gggcgtggat atgtcctgcg ggtaaatagc tgcgccgatg gtttctacaa agatcgttat     780
gtttatcggc actttgcatc ggccgcgctc ccgattccgg aagtgcttga cattggggaa     840
ttcagcgaga gcctgaccta ttgcatctcc cgccgtgcac agggtgtcac gttgcaagac     900
ctgcctgaaa ccgaactgcc cgctgttctg cagccggtcg cggaggccat ggatgcgatc     960
gctgcggccg atcttagcca gacgagcggg ttcggcccat tcggaccgca aggaatcggt    1020
caatacacta catggcgtga tttcatatgc gcgattgctg atccccatgt gtatcactgg    1080
caaactgtga tggacgacac cgtcagtgcg tccgtcgcgc aggctctcga tgagctgatg    1140
ctttgggccg aggactgccc cgaagtccgg cacctcgtgc acgcggattt cggctccaac    1200
aatgtcctga cggacaatgg ccgcataaca gcggtcattg actggagcga ggcgatgttc    1260
ggggattccc aatacgaggt cgccaacatc ttcttctgga ggccgtggtt ggcttgtatg    1320
gagcagcaga cgcgctactt cgagcggagg catccggagc ttgcaggatc gccgcggctc    1380
cgggcgtata tgctccgcat tggtcttgac caactctatc agagcttggt tgacggcaat    1440
ttcgatgatg cagcttgggc gcagggtcga tgcgacgcaa tcgtccgatc cggagccggg    1500
actgtcgggc gtacacaaat cgcccgcaga agcgcggccg tctggaccga tggctgtgta    1560
gaagtactcg ccgatagtgg aaaccgacgc cccagcactc gtccgagggc aaaggaatag    1620
cacgtgctac gagatttcga ttccaccgcc gccttctatg aaaggttggg cttcggaatc    1680
gttttccggg acgccggctg gatgatcctc cagcgcgggg atctcatgct ggagttcttc    1740
gcccacccca acttgtttat tgcagcttat aatggttaca aataaagcaa tagcatcaca    1800
aatttcacaa ataaagcatt tttttcactg cattctagtt gtggtttgtc caaactcatc    1860
aatgtatctt atcatgtctg tataccgtcg acctctagct agagcttggc gtaatcatgg    1920
tcattaccaa tgcttaatca gtgaggcacc tatctcagcg atctgtctat ttcgttcatc    1980
catagttgcc tgactccccg tcgtgtagat aactacgata cgggagggct taccatctgg    2040

ccccagcgct gcgatgatac cgcgagaccc acgctcaccg gctccggatt tatcagcaat 2100
aaaccagcca gccggaaggg ccgagcgcag aagtggtcct gcaactttat ccgcctccat 2160
ccagtctatt aattgttgcc gggaagctag agtaagtagt tcgccagtta atagtttgcg 2220
caacgttgtt gccatcgcta caggcatcgt ggtgtcacgc tcgtcgtttg gtatggcttc 2280
attcagctcc ggttcccaac gatcaaggcg agttacatga tcccccatgt tgtgcaaaaa 2340
agcggttagc tccttcggtc ctccgatcgt tgtcagaagt aagttggccg cagtgttatc 2400
actcatggtt atggcagcac tgcataattc tcttactgtc atgccatccg taagatgctt 2460
ttctgtgact ggtgagtact caaccaagtc attctgagaa tagtgtatgc ggcgaccgag 2520
ttgctcttgc ccggcgtcaa tacgggataa taccgcgcca catagcagaa ctttaaaagt 2580
gctcatcatt ggaaaacgtt cttcggggcg aaaactctca aggatcttac cgctgttgag 2640
atccagttcg atgtaaccca ctcgtgcacc caactgatct tcagcatctt ttactttcac 2700
cagcgtttct gggtgagcaa aaacaggaag gcaaaatgcc gcaaaaaagg gaataagggc 2760
gacacggaaa tgttgaatac tcatattctt cctttttcaa tattattgaa gcatttatca 2820
gggttattgt ctcatgagcg gatacatatt tgaatgtatt tagaaaaata aacaaatagg 2880
ggtcagtgtt acaaccaatt aaccaattct gaacattatc gcgagcccat ttatacctga 2940
atatggctca taacacccct tgctcatgac caaaatccct taacgtgagt tacgcgcgcg 3000
tcgttccact gagcgtcaga ccccgtagaa aagatcaaag gatcttcttg agatcctttt 3060
tttctgcgcg taatctgctg cttgcaaaca aaaaaaccac cgctaccagc ggtggtttgt 3120
ttgccggatc aagagctacc aactcttttt ccgaaggtaa ctggcttcag cagagcgcag 3180
ataccaaata ctgttcttct agtgtagccg tagttagccc accacttcaa gaactctgta 3240
gcaccgccta catacctcgc tctgctaatc ctgttaccag tggctgctgc cagtggcgat 3300
aagtcgtgtc ttaccgggtt ggactcaaga cgatagttac cggataaggc gcagcggtcg 3360
ggctgaacgg ggggttcgtg cacacagccc agcttggagc gaacgaccta caccgaactg 3420
agatacctac agcgtgagct atgagaaagc gccacgcttc ccgaagggag aaaggcggac 3480
aggtatccgg taagcggcag ggtcggaaca ggagagcgca cgagggagct tccaggggga 3540
aacgcctggt atctttatag tcctgtcggg tttcgccacc tctgacttga gcgtcgattt 3600
ttgtgatgct cgtcaggggg gcggagccta tggaaaaacg ccagcaacgc ggccttttta 3660
cggttcctgg ccttttgctg gccttttgct cacatgttct ttcctgcgtt atcccctgat 3720
tctgtggata accgtattac cgcctttgag tgagctgata ccgctcgccg cagccgaacg 3780
accgagcgca gcgagtcagt gagcgaggaa gcggaaggcg agagtaggga actgccaggc 3840
atcaaactaa gcagaaggcc cctgacggat ggcctttttg cgtttctaca aactctttct 3900
gtgttgtaaa acgacggcca gtcttaagct cgggccccct gggcggttct gataacgagt 3960
aatcgttaat ccgcaaataa cgtaaaaacc cgcttcggcg ggttttttta tggggggagt 4020
ttagggaaag agcatttgtc agaatattta agggcgcctg tcactttgct tgatatatga 4080
gaattattta accttataaa tgagaaaaaa gcaacgcact ttaaataaga tacgttgctt 4140
tttcgattga tgaacaccta taattaaact attcatctat tatttatgat tttttgtata 4200
tacaatattt ctagtttgtt aaagagaatt aagaaaataa atctcgaaaa taataaaggg 4260
aaaatcagtt tttgatatca aaattataca tgtcaacgat aatacaaaat ataatacaaa 4320
ctataagatg ttatcagtat ttattatcat ttagaataaa ttttgtgtcg cccttaattg 4380
tgagcggata acaattacga gcttcatgca cagtggcgtt gacattgatt attgactagt 4440
tattaatagt aatcaattac ggggtcatta gttcatagcc catatatgga gttccgcgtt 4500
acataactta cggtaaatgg cccgcctggc tgaccgccca acgacccccg cccattgacg 4560
tcaataatga cgtatgttcc catagtaacg ccaataggga ctttccattg acgtcaatgg 4620
gtggagtatt tacggtaaac tgcccacttg gcagtacatc aagtgtatca tatgccaagt 4680
acgcccccta ttgacgtcaa tgacggtaaa tggcccgcct ggcattatgc ccagtacatg 4740
accttatggg actttcctac ttggcagtac atctacgtat tagtcatcgc tattaccatg 4800
gtgatgcggt tttggcagta catcaatggg cgtggatagc ggtttgactc acggggattt 4860
ccaagtctcc accccattga cgtcaatggg agtttgtttt ggcaccaaaa tcaacgggac 4920
tttccaaaat gtcgtaacaa ctccgcccca ttgacgcaaa tgggcggtag gcgtgtacgg 4980
tgggaggtct atataagcag agctctctgg ctaactagag aacccactgc ttactggctt 5040
atcgaaatta atacgactca ctatagggaa gcttcttgtt ctttttgcag aagctcagaa 5100
taaacgctca actttggcct cgaggccacc atg gct tcc tcc cct cca aag aaa 5154
Met Ala Ser Ser Pro Pro Lys Lys
1               5

aag aga aag gtt gcg gcc gct gac tac aag gat gac gac gat aaa agt 5202
Lys Arg Lys Val Ala Ala Ala Asp Tyr Lys Asp Asp Asp Asp Lys Ser
    10                  15                  20

tgg aag gac gca agt ggt tgg tct aga atg cat gcg gcc ccg cga cgg 5250
Trp Lys Asp Ala Ser Gly Trp Ser Arg Met His Ala Ala Pro Arg Arg
25                  30                  35                  40

cgt gct gcg caa ccc tcc gac gct tcg ccg gcc gcg cag gtg gat cta 5298
Arg Ala Ala Gln Pro Ser Asp Ala Ser Pro Ala Ala Gln Val Asp Leu
                45                  50                  55

cgc acg ctc ggc tac agt cag cag cag caa gag aag atc aaa ccg aag 5346
Arg Thr Leu Gly Tyr Ser Gln Gln Gln Gln Glu Lys Ile Lys Pro Lys
            60                  65                  70

gtg cgt tcg aca gtg gcg cag cac cac gag gca ctg gtg ggc cat ggg 5394
Val Arg Ser Thr Val Ala Gln His His Glu Ala Leu Val Gly His Gly
        75                  80                  85

ttt aca cac gcg cac atc gtt gcg ctc agc caa cac ccg gca gcg tta 5442
Phe Thr His Ala His Ile Val Ala Leu Ser Gln His Pro Ala Ala Leu
    90                  95                  100

ggg acc gtc gct gtc acg tat cag cac ata atc acg gcg ttg cca gag 5490
Gly Thr Val Ala Val Thr Tyr Gln His Ile Ile Thr Ala Leu Pro Glu
105                 110                 115                 120

gcg aca cac gaa gac atc gtt ggc gtc ggc aaa cag tgg tcc ggc gca 5538
Ala Thr His Glu Asp Ile Val Gly Val Gly Lys Gln Trp Ser Gly Ala
                125                 130                 135

cgc gcc ctg gag gcc ttg ctc acg gat gcg ggg gag ttg aga ggt ccg 5586
Arg Ala Leu Glu Ala Leu Leu Thr Asp Ala Gly Glu Leu Arg Gly Pro
            140                 145                 150

ccg tta cag ttg gac aca ggc caa ctt gtg aag att gca aaa cgt ggc 5634
Pro Leu Gln Leu Asp Thr Gly Gln Leu Val Lys Ile Ala Lys Arg Gly
        155                 160                 165

ggc gtg acc gca atg gag gca gtg cat gca tcg cgc aat gcg ctc acg 5682
Gly Val Thr Ala Met Glu Ala Val His Ala Ser Arg Asn Ala Leu Thr
    170                 175                 180

gga gca ccc ctc aac ctg acc cca gac cag gtt gtg gcc atc gcc agc 5730
Gly Ala Pro Leu Asn Leu Thr Pro Asp Gln Val Val Ala Ile Ala Ser
185                 190                 195                 200

aac ata ggt ggc aag cag gcc ctc gaa acc gtc cag aga ctg tta ccg 5778
Asn Ile Gly Gly Lys Gln Ala Leu Glu Thr Val Gln Arg Leu Leu Pro
                205                 210                 215

gtt ctc tgc cag gac cac ggc ctg acc ccg gaa cag gtg gtt gca atc 5826
Val Leu Cys Gln Asp His Gly Leu Thr Pro Glu Gln Val Val Ala Ile
            220                 225                 230

gcg tca cac gat ggg gga aag cag gcc cta gaa acc gtt cag cga ctc 5874
Ala Ser His Asp Gly Gly Lys Gln Ala Leu Glu Thr Val Gln Arg Leu
        235                 240                 245

ctg ccc gtc ctg tgc cag gcc cac ggc ctg acc ccc gac cag gtt gtc 5922
Leu Pro Val Leu Cys Gln Ala His Gly Leu Thr Pro Asp Gln Val Val
    250                 255                 260

gct att gct agt aac ggc gga ggc aaa cag gcg ctg gaa aca gtt cag 5970
Ala Ile Ala Ser Asn Gly Gly Gly Lys Gln Ala Leu Glu Thr Val Gln
265                 270                 275                 280

cgc ctc ttg ccg gtc ttg tgt cag gcc cac ggc ctg acc ccc gcc cag 6018
Arg Leu Leu Pro Val Leu Cys Gln Ala His Gly Leu Thr Pro Ala Gln
                285                 290                 295

gtt gtc gct att gct agt aac ggc gga ggc aaa cag gcg ctg gaa aca     6066
Val Val Ala Ile Ala Ser Asn Gly Gly Gly Lys Gln Ala Leu Glu Thr
            300                 305                 310

gtt cag cgc ctc ttg ccg gtc ttg tgt cag gac cac ggc ctg acc ccg     6114
Val Gln Arg Leu Leu Pro Val Leu Cys Gln Asp His Gly Leu Thr Pro
        315                 320                 325

gac cag gtg gtt gca atc gcg tca cac gat ggg gga aag cag gcc cta     6162
Asp Gln Val Val Ala Ile Ala Ser His Asp Gly Gly Lys Gln Ala Leu
    330                 335                 340

gaa acc gtt cag cga ctc ctg ccc gtc ctg tgc cag gac cac ggc ctg     6210
Glu Thr Val Gln Arg Leu Leu Pro Val Leu Cys Gln Asp His Gly Leu
345                 350                 355                 360

acc ccc gaa cag gtt gtc gct att gct agt aac ggc gga ggc aaa cag     6258
Thr Pro Glu Gln Val Val Ala Ile Ala Ser Asn Gly Gly Gly Lys Gln
                365                 370                 375

gcg ctg gaa aca gtt cag cgc ctc ttg ccg gtc ttg tgt cag gcc cac     6306
Ala Leu Glu Thr Val Gln Arg Leu Leu Pro Val Leu Cys Gln Ala His
            380                 385                 390

ggc ctg acc ccc gac cag gtt gtc gct att gct agt aac ggc gga ggc     6354
Gly Leu Thr Pro Asp Gln Val Val Ala Ile Ala Ser Asn Gly Gly Gly
        395                 400                 405

aaa cag gcg ctg gaa aca gtt cag cgc ctc ttg ccg gtc ttg tgt cag     6402
Lys Gln Ala Leu Glu Thr Val Gln Arg Leu Leu Pro Val Leu Cys Gln
    410                 415                 420

gcc cac ggc ctg acc cca gcc caa gtt gtc gcg att gca agc aac aac     6450
Ala His Gly Leu Thr Pro Ala Gln Val Val Ala Ile Ala Ser Asn Asn
425                 430                 435                 440

gga ggc aaa caa gcc tta gaa aca gtc cag aga ttg ttg ccg gtg ctg     6498
Gly Gly Lys Gln Ala Leu Glu Thr Val Gln Arg Leu Leu Pro Val Leu
                445                 450                 455

tgc caa gac cac ggc ctg acc ccg gac cag gtg gtt gca atc gcg tca     6546
Cys Gln Asp His Gly Leu Thr Pro Asp Gln Val Val Ala Ile Ala Ser
            460                 465                 470

cac gat ggg gga aag cag gcc cta gaa acc gtt cag cga ctc ctg ccc     6594
His Asp Gly Gly Lys Gln Ala Leu Glu Thr Val Gln Arg Leu Leu Pro
        475                 480                 485

gtc ctg tgc cag gac cac ggc ctg acc ccc gaa cag gtt gtc gct att     6642
Val Leu Cys Gln Asp His Gly Leu Thr Pro Glu Gln Val Val Ala Ile
    490                 495                 500

gct agt aac ggc gga ggc aaa cag gcg ctg gaa aca gtt cag cgc ctc     6690
Ala Ser Asn Gly Gly Gly Lys Gln Ala Leu Glu Thr Val Gln Arg Leu
505                 510                 515                 520

ttg ccg gtc ttg tgt cag gcc cac ggc ctg acc cca gac caa gtt gtc     6738
Leu Pro Val Leu Cys Gln Ala His Gly Leu Thr Pro Asp Gln Val Val
                525                 530                 535

gcg att gca agc aac aac gga ggc aaa caa gcc tta gaa aca gtc cag     6786
Ala Ile Ala Ser Asn Asn Gly Gly Lys Gln Ala Leu Glu Thr Val Gln

            540                 545                 550

aga ttg ttg cct gtg ctg tgc caa gcc cac ggc ctg acc ccg gcc cag 6834
Arg Leu Leu Pro Val Leu Cys Gln Ala His Gly Leu Thr Pro Ala Gln
        555                 560                 565

gtg gtt gca atc gcg tca cac gat ggg gga aag cag gcc cta gaa acc 6882
Val Val Ala Ile Ala Ser His Asp Gly Gly Lys Gln Ala Leu Glu Thr
    570                 575                 580

gtt cag cga ctc ctg ccc gtc ctg tgc cag gac cac ggc ctg acc cca 6930
Val Gln Arg Leu Leu Pro Val Leu Cys Gln Asp His Gly Leu Thr Pro
585                 590                 595                 600

gac cag gtt gtg gcc atc gcc agc aac ata ggt ggc aag cag gcc ctc 6978
Asp Gln Val Val Ala Ile Ala Ser Asn Ile Gly Gly Lys Gln Ala Leu
                605                 610                 615

gaa acc gtc cag aga ctg tta ccg gtt ctc tgc cag gac cac ggc ctg 7026
Glu Thr Val Gln Arg Leu Leu Pro Val Leu Cys Gln Asp His Gly Leu
            620                 625                 630

acc ccg gaa cag gtg gtt gca atc gcg tca cac gat ggg gga aag cag 7074
Thr Pro Glu Gln Val Val Ala Ile Ala Ser His Asp Gly Gly Lys Gln
        635                 640                 645

gcc cta gaa acc gtt cag cga ctc ctg ccc gtc ctg tgc cag gcc cac 7122
Ala Leu Glu Thr Val Gln Arg Leu Leu Pro Val Leu Cys Gln Ala His
    650                 655                 660

ggc ctg acc ccc gac cag gtt gtc gct att gct agt aac ggc gga ggc 7170
Gly Leu Thr Pro Asp Gln Val Val Ala Ile Ala Ser Asn Gly Gly Gly
665                 670                 675                 680

aaa cag gcg ctg gaa aca gtt cag cgc ctc ttg ccg gtc ttg tgt cag 7218
Lys Gln Ala Leu Glu Thr Val Gln Arg Leu Leu Pro Val Leu Cys Gln
                685                 690                 695

gcc cac ggc ctg acc cca gcc caa gtt gtc gcg att gca agc aac aac 7266
Ala His Gly Leu Thr Pro Ala Gln Val Val Ala Ile Ala Ser Asn Asn
            700                 705                 710

gga ggc aaa caa gcc tta gaa aca gtc cag aga ttg ttg ccg gtg ctg 7314
Gly Gly Lys Gln Ala Leu Glu Thr Val Gln Arg Leu Leu Pro Val Leu
        715                 720                 725

tgc caa gac cac ggc ctg acc cca gac caa gtt gtc gcg att gca agc 7362
Cys Gln Asp His Gly Leu Thr Pro Asp Gln Val Val Ala Ile Ala Ser
    730                 735                 740

aac aac gga ggc aaa caa gcc tta gaa aca gtc cag aga ttg ttg ccg 7410
Asn Asn Gly Gly Lys Gln Ala Leu Glu Thr Val Gln Arg Leu Leu Pro
745                 750                 755                 760

gtg ctg tgc caa gac cac ggc ctg acc cca gaa caa gtt gtc gcg att 7458
Val Leu Cys Gln Asp His Gly Leu Thr Pro Glu Gln Val Val Ala Ile
                765                 770                 775

gca agc aac aac gga ggc aaa caa gcc tta gaa aca gtc cag aga ttg 7506
Ala Ser Asn Asn Gly Gly Lys Gln Ala Leu Glu Thr Val Gln Arg Leu
            780                 785                 790

ttg ccg gtg ctg tgc caa gcc cac ggc ctg acc cca gac cag gtt gtg 7554

Leu Pro Val Leu Cys Gln Ala His Gly Leu Thr Pro Asp Gln Val Val
        795                 800                 805

gcc atc gcc agc aac ata ggt ggc aag cag gcc ctc gaa acc gtc cag        7602
Ala Ile Ala Ser Asn Ile Gly Gly Lys Gln Ala Leu Glu Thr Val Gln
    810                 815                 820

aga ctg tta ccg gtt ctc tgc cag gcc cac ggc ctg acg cct gag cag        7650
Arg Leu Leu Pro Val Leu Cys Gln Ala His Gly Leu Thr Pro Glu Gln
825                 830                 835                 840

gta gtg gct att gca tcc aac ata ggg ggc aga ccc gca ctg gag tca        7698
Val Val Ala Ile Ala Ser Asn Ile Gly Gly Arg Pro Ala Leu Glu Ser
                845                 850                 855

atc gtg gcc cag ctt tcg agg ccg gac ccc gcg ctg gcc gca ctc act        7746
Ile Val Ala Gln Leu Ser Arg Pro Asp Pro Ala Leu Ala Ala Leu Thr
            860                 865                 870

aat gat cat ctt gta gcg ctg gcc tgc ctc ggc gga cgt cct gcc atg        7794
Asn Asp His Leu Val Ala Leu Ala Cys Leu Gly Gly Arg Pro Ala Met
        875                 880                 885

gat gca gtg aaa aag gga ttg ccg cac gcg ccg gaa ttg atc aga tcc        7842
Asp Ala Val Lys Lys Gly Leu Pro His Ala Pro Glu Leu Ile Arg Ser
    890                 895                 900

cag cta gtg aaa tct gaa ttg gaa gag aag aaa tct gaa ctt aga cat        7890
Gln Leu Val Lys Ser Glu Leu Glu Glu Lys Lys Ser Glu Leu Arg His
905                 910                 915                 920

aaa ttg aaa tat gtg cca cat gaa tat att gaa ttg att gaa atc gca        7938
Lys Leu Lys Tyr Val Pro His Glu Tyr Ile Glu Leu Ile Glu Ile Ala
                925                 930                 935

aga aat tca act cag gat aga atc ctt gaa atg aag gtg atg gag ttc        7986
Arg Asn Ser Thr Gln Asp Arg Ile Leu Glu Met Lys Val Met Glu Phe
            940                 945                 950

ttt atg aag gtt tat ggt tat cgt ggt aaa cat ttg ggt gga tca agg        8034
Phe Met Lys Val Tyr Gly Tyr Arg Gly Lys His Leu Gly Gly Ser Arg
        955                 960                 965

aaa cca gac gga gca att tat act gtc gga tct cct att gat tac ggt        8082
Lys Pro Asp Gly Ala Ile Tyr Thr Val Gly Ser Pro Ile Asp Tyr Gly
    970                 975                 980

gtg atc gtt gat act aag gca tat tca gga ggt tat aat ctt cca att        8130
Val Ile Val Asp Thr Lys Ala Tyr Ser Gly Gly Tyr Asn Leu Pro Ile
985                 990                 995                 1000

ggt caa gca gat gaa  atg caa aga tat gtc  gaa gag aat caa aca        8175
Gly Gln Ala Asp Glu  Met Gln Arg Tyr Val  Glu Glu Asn Gln Thr
                1005                1010                1015

aga aac aag cat atc  aac cct aat gaa tgg  tgg aaa gtc tat cca        8220
Arg Asn Lys His Ile  Asn Pro Asn Glu Trp  Trp Lys Val Tyr Pro
                1020                1025                1030

tct tca gta aca gaa  ttt aag ttc ttg ttt  gtg agt ggt cat ttc        8265
Ser Ser Val Thr Glu  Phe Lys Phe Leu Phe  Val Ser Gly His Phe
                1035                1040                1045

aaa gga aac tac aaa  gct cag ctt aca aga  ttg aat cat atc act      8310
Lys Gly Asn Tyr Lys  Ala Gln Leu Thr Arg  Leu Asn His Ile Thr
                1050                 1055                 1060

aat tgt aat gga gct  gtt ctt agt gta gaa  gag ctt ttg att ggt      8355
Asn Cys Asn Gly Ala  Val Leu Ser Val Glu  Glu Leu Leu Ile Gly
                1065                 1070                 1075

gga gaa atg att aaa  gct ggt aca ttg aca  ctt gag gaa gtg aga      8400
Gly Glu Met Ile Lys  Ala Gly Thr Leu Thr  Leu Glu Glu Val Arg
                1080                 1085                 1090

agg aaa ttt aat aac  ggt gag ata aac ttt  taa aaaatcagcc           8443
Arg Lys Phe Asn Asn  Gly Glu Ile Asn Phe
                1095                 1100

tcgactgtgc cttctagttg cc                                           8465


<210>  2
<211>  1100
<212>  PRT
<213>  Artificial Sequence

<220>
<223>  Synthetic Construct

<400>  2

Met Ala Ser Ser Pro Pro Lys Lys Lys Arg Lys Val Ala Ala Ala Asp
1               5                   10                  15

Tyr Lys Asp Asp Asp Asp Lys Ser Trp Lys Asp Ala Ser Gly Trp Ser
            20                  25                  30

Arg Met His Ala Ala Pro Arg Arg Arg Ala Ala Gln Pro Ser Asp Ala
        35                  40                  45

Ser Pro Ala Ala Gln Val Asp Leu Arg Thr Leu Gly Tyr Ser Gln Gln
    50                  55                  60

Gln Gln Glu Lys Ile Lys Pro Lys Val Arg Ser Thr Val Ala Gln His
65                  70                  75                  80

His Glu Ala Leu Val Gly His Gly Phe Thr His Ala His Ile Val Ala
                85                  90                  95

Leu Ser Gln His Pro Ala Ala Leu Gly Thr Val Ala Val Thr Tyr Gln
            100                 105                 110

His Ile Ile Thr Ala Leu Pro Glu Ala Thr His Glu Asp Ile Val Gly
        115                 120                 125

Val Gly Lys Gln Trp Ser Gly Ala Arg Ala Leu Glu Ala Leu Leu Thr
    130                 135                 140

Asp Ala Gly Glu Leu Arg Gly Pro Pro Leu Gln Leu Asp Thr Gly Gln
145                 150                 155                 160

Leu Val Lys Ile Ala Lys Arg Gly Gly Val Thr Ala Met Glu Ala Val
                165                 170                 175

His Ala Ser Arg Asn Ala Leu Thr Gly Ala Pro Leu Asn Leu Thr Pro

            180                 185                 190

Asp Gln Val Val Ala Ile Ala Ser Asn Ile Gly Gly Lys Gln Ala Leu
        195                 200                 205

Glu Thr Val Gln Arg Leu Leu Pro Val Leu Cys Gln Asp His Gly Leu
    210                 215                 220

Thr Pro Glu Gln Val Val Ala Ile Ala Ser His Asp Gly Gly Lys Gln
225                 230                 235                 240

Ala Leu Glu Thr Val Gln Arg Leu Leu Pro Val Leu Cys Gln Ala His
                245                 250                 255

Gly Leu Thr Pro Asp Gln Val Val Ala Ile Ala Ser Asn Gly Gly Gly
            260                 265                 270

Lys Gln Ala Leu Glu Thr Val Gln Arg Leu Leu Pro Val Leu Cys Gln
        275                 280                 285

Ala His Gly Leu Thr Pro Ala Gln Val Val Ala Ile Ala Ser Asn Gly
    290                 295                 300

Gly Gly Lys Gln Ala Leu Glu Thr Val Gln Arg Leu Leu Pro Val Leu
305                 310                 315                 320

Cys Gln Asp His Gly Leu Thr Pro Asp Gln Val Val Ala Ile Ala Ser
                325                 330                 335

His Asp Gly Gly Lys Gln Ala Leu Glu Thr Val Gln Arg Leu Leu Pro
            340                 345                 350

Val Leu Cys Gln Asp His Gly Leu Thr Pro Glu Gln Val Val Ala Ile
        355                 360                 365

Ala Ser Asn Gly Gly Gly Lys Gln Ala Leu Glu Thr Val Gln Arg Leu
    370                 375                 380

Leu Pro Val Leu Cys Gln Ala His Gly Leu Thr Pro Asp Gln Val Val
385                 390                 395                 400

Ala Ile Ala Ser Asn Gly Gly Gly Lys Gln Ala Leu Glu Thr Val Gln
                405                 410                 415

Arg Leu Leu Pro Val Leu Cys Gln Ala His Gly Leu Thr Pro Ala Gln
            420                 425                 430

Val Val Ala Ile Ala Ser Asn Asn Gly Gly Lys Gln Ala Leu Glu Thr
        435                 440                 445

Val Gln Arg Leu Leu Pro Val Leu Cys Gln Asp His Gly Leu Thr Pro
    450                 455                 460

Asp Gln Val Val Ala Ile Ala Ser His Asp Gly Gly Lys Gln Ala Leu
465                 470                 475                 480

Glu Thr Val Gln Arg Leu Leu Pro Val Leu Cys Gln Asp His Gly Leu
                485                 490                 495

Thr Pro Glu Gln Val Val Ala Ile Ala Ser Asn Gly Gly Gly Lys Gln
            500                 505                 510

Ala Leu Glu Thr Val Gln Arg Leu Leu Pro Val Leu Cys Gln Ala His

515                 520                 525

Gly Leu Thr Pro Asp Gln Val Val Ala Ile Ala Ser Asn Asn Gly Gly
    530                 535                 540

Lys Gln Ala Leu Glu Thr Val Gln Arg Leu Leu Pro Val Leu Cys Gln
545                 550                 555                 560

Ala His Gly Leu Thr Pro Ala Gln Val Val Ala Ile Ala Ser His Asp
                565                 570                 575

Gly Gly Lys Gln Ala Leu Glu Thr Val Gln Arg Leu Leu Pro Val Leu
            580                 585                 590

Cys Gln Asp His Gly Leu Thr Pro Asp Gln Val Val Ala Ile Ala Ser
        595                 600                 605

Asn Ile Gly Gly Lys Gln Ala Leu Glu Thr Val Gln Arg Leu Leu Pro
    610                 615                 620

Val Leu Cys Gln Asp His Gly Leu Thr Pro Glu Gln Val Val Ala Ile
625                 630                 635                 640

Ala Ser His Asp Gly Gly Lys Gln Ala Leu Glu Thr Val Gln Arg Leu
                645                 650                 655

Leu Pro Val Leu Cys Gln Ala His Gly Leu Thr Pro Asp Gln Val Val
            660                 665                 670

Ala Ile Ala Ser Asn Gly Gly Gly Lys Gln Ala Leu Glu Thr Val Gln
        675                 680                 685

Arg Leu Leu Pro Val Leu Cys Gln Ala His Gly Leu Thr Pro Ala Gln
    690                 695                 700

Val Val Ala Ile Ala Ser Asn Asn Gly Gly Lys Gln Ala Leu Glu Thr
705                 710                 715                 720

Val Gln Arg Leu Leu Pro Val Leu Cys Gln Asp His Gly Leu Thr Pro
                725                 730                 735

Asp Gln Val Val Ala Ile Ala Ser Asn Asn Gly Gly Lys Gln Ala Leu
            740                 745                 750

Glu Thr Val Gln Arg Leu Leu Pro Val Leu Cys Gln Asp His Gly Leu
        755                 760                 765

Thr Pro Glu Gln Val Val Ala Ile Ala Ser Asn Asn Gly Gly Lys Gln
    770                 775                 780

Ala Leu Glu Thr Val Gln Arg Leu Leu Pro Val Leu Cys Gln Ala His
785                 790                 795                 800

Gly Leu Thr Pro Asp Gln Val Val Ala Ile Ala Ser Asn Ile Gly Gly
                805                 810                 815

Lys Gln Ala Leu Glu Thr Val Gln Arg Leu Leu Pro Val Leu Cys Gln
            820                 825                 830

Ala His Gly Leu Thr Pro Glu Gln Val Val Ala Ile Ala Ser Asn Ile
        835                 840                 845

Gly Gly Arg Pro Ala Leu Glu Ser Ile Val Ala Gln Leu Ser Arg Pro

850                 855                 860

Asp Pro Ala Leu Ala Ala Leu Thr Asn Asp His Leu Val Ala Leu Ala
865                 870                 875                 880

Cys Leu Gly Gly Arg Pro Ala Met Asp Ala Val Lys Lys Gly Leu Pro
                885                 890                 895

His Ala Pro Glu Leu Ile Arg Ser Gln Leu Val Lys Ser Glu Leu Glu
            900                 905                 910

Glu Lys Lys Ser Glu Leu Arg His Lys Leu Lys Tyr Val Pro His Glu
        915                 920                 925

Tyr Ile Glu Leu Ile Glu Ile Ala Arg Asn Ser Thr Gln Asp Arg Ile
    930                 935                 940

Leu Glu Met Lys Val Met Glu Phe Phe Met Lys Val Tyr Gly Tyr Arg
945                 950                 955                 960

Gly Lys His Leu Gly Gly Ser Arg Lys Pro Asp Gly Ala Ile Tyr Thr
                965                 970                 975

Val Gly Ser Pro Ile Asp Tyr Gly Val Ile Val Asp Thr Lys Ala Tyr
            980                 985                 990

Ser Gly Gly Tyr Asn Leu Pro Ile  Gly Gln Ala Asp Glu  Met Gln Arg
        995                 1000                1005

Tyr Val  Glu Glu Asn Gln Thr  Arg Asn Lys His Ile  Asn Pro Asn
    1010                1015                1020

Glu Trp  Trp Lys Val Tyr Pro  Ser Ser Val Thr Glu  Phe Lys Phe
    1025                1030                1035

Leu Phe  Val Ser Gly His Phe  Lys Gly Asn Tyr Lys  Ala Gln Leu
    1040                1045                1050

Thr Arg  Leu Asn His Ile Thr  Asn Cys Asn Gly Ala  Val Leu Ser
    1055                1060                1065

Val Glu  Glu Leu Leu Ile Gly  Gly Glu Met Ile Lys  Ala Gly Thr
    1070                1075                1080

Leu Thr  Leu Glu Glu Val Arg  Arg Lys Phe Asn Asn  Gly Glu Ile
    1085                1090                1095

Asn Phe
    1100


<210>  3
<211>  8159
<212>  DNA
<213>  Artificial Sequence

<220>
<223>  Right TALEN


<220>
<221>  CDS
<222>  (5131)..(8127)

<400> 3
agccatctgt tgtttgcccc tcccccgtgc cttccttgac cctggaaggt gccactccca 60
ctgtcctttc ctaataaaat gaggaaattg catcacaaca ctcaacccta tctcggtcta 120
ttcttttgat ttataaggga ttttgccgat ttcggcctat tggttaaaaa atgagctgat 180
ttaacaaaaa tttaacgcga attaattctg tggaatgtgt gtcagttagg gtgtggaaag 240
tccccaggct ccccagcagg cagaagtatg caaagcatgc atctcaatta gtcagcaacc 300
aggtgtggaa agtccccagg ctccccagca ggcagaagta tgcaaagcat gcatctcaat 360
tagtcagcaa ccatagtccc gcccctaact ccgcccatcc cgcccctaac tccgcccagt 420
tccgcccatt ctccgcccca tggctgacta atttttttta tttatgcaga ggccgaggcc 480
gcctctgcct ctgagctatt ccagaagtag tgaggaggct tttttggagg cctaggcttt 540
tgcaaaaagc tcccgggagc ttgtatatcc attttcggat ctgatcagca cgtgatgaaa 600
aagcctgaac tcaccgcgac gtctgtcgag aagtttctga tcgaaaagtt cgacagcgtt 660
tccgacctga tgcagctctc ggagggcgaa gaatctcgtg ctttcagctt cgatgtagga 720
gggcgtggat atgtcctgcg ggtaaatagc tgcgccgatg gtttctacaa agatcgttat 780
gtttatcggc actttgcatc ggccgcgctc ccgattccgg aagtgcttga cattggggaa 840
ttcagcgaga gcctgaccta ttgcatctcc cgccgtgcac agggtgtcac gttgcaagac 900
ctgcctgaaa ccgaactgcc cgctgttctg cagccggtcg cggaggccat ggatgcgatc 960
gctgcggccg atcttagcca gacgagcggg ttcggcccat tcggaccgca aggaatcggt 1020
caatacacta catggcgtga tttcatatgc gcgattgctg atccccatgt gtatcactgg 1080
caaactgtga tggacgacac cgtcagtgcg tccgtcgcgc aggctctcga tgagctgatg 1140
ctttgggccg aggactgccc cgaagtccgg cacctcgtgc acgcggattt cggctccaac 1200
aatgtcctga cggacaatgg ccgcataaca gcggtcattg actggagcga ggcgatgttc 1260
ggggattccc aatacgaggt cgccaacatc ttcttctgga ggccgtggtt ggcttgtatg 1320
gagcagcaga cgcgctactt cgagcggagg catccggagc ttgcaggatc gccgcggctc 1380
cgggcgtata tgctccgcat tggtcttgac caactctatc agagcttggt tgacggcaat 1440
ttcgatgatg cagcttgggc gcagggtcga tgcgacgcaa tcgtccgatc cggagccggg 1500
actgtcgggc gtacacaaat cgcccgcaga agcgcggccg tctggaccga tggctgtgta 1560
gaagtactcg ccgatagtgg aaaccgacgc cccagcactc gtccgagggc aaaggaatag 1620
cacgtgctac gagatttcga ttccaccgcc gccttctatg aaaggttggg cttcggaatc 1680
gttttccggg acgccggctg gatgatcctc cagcgcgggg atctcatgct ggagttcttc 1740
gcccacccca acttgtttat tgcagcttat aatggttaca aataaagcaa tagcatcaca 1800
aatttcacaa ataaagcatt tttttcactg cattctagtt gtggtttgtc caaactcatc 1860
aatgtatctt atcatgtctg tataccgtcg acctctagct agagcttggc gtaatcatgg 1920
tcattaccaa tgcttaatca gtgaggcacc tatctcagcg atctgtctat ttcgttcatc 1980
catagttgcc tgactccccg tcgtgtagat aactacgata cgggagggct taccatctgg 2040
ccccagcgct gcgatgatac cgcgagaacc acgctcaccg gctccggatt tatcagcaat 2100
aaaccagcca gccggaaggg ccgagcgcag aagtggtcct gcaactttat ccgcctccat 2160
ccagtctatt aattgttgcc gggaagctag agtaagtagt tcgccagtta atagtttgcg 2220
caacgttgtt gccatcgcta caggcatcgt ggtgtcacgc tcgtcgtttg gtatggcttc 2280
attcagctcc ggttcccaac gatcaaggcg agttacatga tcccccatgt tgtgcaaaaa 2340
agcggttagc tccttcggtc ctccgatcgt tgtcagaagt aagttggccg cagtgttatc 2400
actcatggtt atggcagcac tgcataattc tcttactgtc atgccatccg taagatgctt 2460
ttctgtgact ggtgagtact caaccaagtc attctgagaa tagtgtatgc ggcgaccgag 2520
ttgctcttgc ccggcgtcaa tacgggataa taccgcgcca catagcagaa ctttaaaagt 2580
gctcatcatt ggaaaacgtt cttcggggcg aaaactctca aggatcttac cgctgttgag 2640
atccagttcg atgtaaccca ctcgtgcacc caactgatct tcagcatctt ttactttcac 2700
cagcgtttct gggtgagcaa aaacaggaag gcaaaatgcc gcaaaaaagg gaataagggc 2760
gacacggaaa tgttgaatac tcatattctt cctttttcaa tattattgaa gcatttatca 2820
gggttattgt ctcatgagcg gatacatatt tgaatgtatt tagaaaaata aacaaatagg 2880
ggtcagtgtt acaaccaatt aaccaattct gaacattatc gcgagcccat ttatacctga 2940
atatggctca taacacccct tgctcatgac caaaatccct taacgtgagt tacgcgcgcg 3000
tcgttccact gagcgtcaga ccccgtagaa aagatcaaag gatcttcttg agatcctttt 3060
tttctgcgcg taatctgctg cttgcaaaca aaaaaaccac cgctaccagc ggtggtttgt 3120
ttgccggatc aagagctacc aactcttttt ccgaaggtaa ctggcttcag cagagcgcag 3180
ataccaaata ctgttcttct agtgtagccg tagttagccc accacttcaa gaactctgta 3240
gcaccgccta catacctcgc tctgctaatc ctgttaccag tggctgctgc cagtggcgat 3300
aagtcgtgtc ttaccgggtt ggactcaaga cgatagttac cggataaggc gcagcggtcg 3360
ggctgaacgg ggggttcgtg cacacagccc agcttggagc gaacgaccta caccgaactg 3420
agatacctac agcgtgagct atgagaaagc gccacgcttc ccgaagggag aaaggcggac 3480
aggtatccgg taagcggcag ggtcggaaca ggagagcgca cgagggagct tccaggggga 3540
aacgcctggt atctttatag tcctgtcggg tttcgccacc tctgacttga gcgtcgattt 3600
ttgtgatgct cgtcaggggg gcggagccta tggaaaaacg ccagcaacgc ggccttttta 3660

cggttcctgg ccttttgctg gccttttgct cacatgttct ttcctgcgtt atcccctgat 3720
tctgtggata accgtattac cgcctttgag tgagctgata ccgctcgccg cagccgaacg 3780
accgagcgca gcgagtcagt gagcgaggaa gcggaaggcg agagtaggga actgccaggc 3840
atcaaactaa gcagaaggcc cctgacggat ggcctttttg cgtttctaca aactctttct 3900
gtgttgtaaa acgacggcca gtcttaagct cgggccccct gggcggttct gataacgagt 3960
aatcgttaat ccgcaaataa cgtaaaaacc cgcttcggcg ggttttttta tggggggagt 4020
ttagggaaag agcatttgtc agaatattta agggcgcctg tcactttgct tgatatatga 4080
gaattattta accttataaa tgagaaaaaa gcaacgcact ttaaataaga tacgttgctt 4140
tttcgattga tgaacaccta taattaaact attcatctat tatttatgat tttttgtata 4200
tacaatattt ctagtttgtt aaagagaatt aagaaaataa atctcgaaaa taataaaggg 4260
aaaatcagtt tttgatatca aaattataca tgtcaacgat aatacaaaat ataatacaaa 4320
ctataagatg ttatcagtat ttattatcat ttagaataaa ttttgtgtcg cccttaattg 4380
tgagcggata acaattacga gcttcatgca cagtggcgtt gacattgatt attgactagt 4440
tattaatagt aatcaattac ggggtcatta gttcatagcc catatatgga gttccgcgtt 4500
acataactta cggtaaatgg cccgcctggc tgaccgccca acgacccccg cccattgacg 4560
tcaataatga cgtatgttcc catagtaacg ccaataggga ctttccattg acgtcaatgg 4620
gtggagtatt tacggtaaac tgcccacttg gcagtacatc aagtgtatca tatgccaagt 4680
acgcccccta ttgacgtcaa tgacggtaaa tggcccgcct ggcattatgc ccagtacatg 4740
accttatggg actttcctac ttggcagtac atctacgtat tagtcatcgc tattaccatg 4800
gtgatgcggt tttggcagta catcaatggg cgtggatagc ggtttgactc acggggattt 4860
ccaagtctcc accccattga cgtcaatggg agtttgtttt ggcaccaaaa tcaacgggac 4920
tttccaaaat gtcgtaacaa ctccgcccca ttgacgcaaa tgggcggtag gcgtgtacgg 4980
tgggaggtct atataagcag agctctctgg ctaactagag aacccactgc ttactggctt 5040
atcgaaatta atacgactca ctatagggaa gcttcttgtt ctttttgcag aagctcagaa 5100
taaacgctca actttggcct cgaggccacc atg gct tcc tcc cct cca aag aaa 5154
Met Ala Ser Ser Pro Pro Lys Lys
1               5

aag aga aag gtt gcg gcc gct gac tac aag gat gac gac gat aaa agt 5202
Lys Arg Lys Val Ala Ala Ala Asp Tyr Lys Asp Asp Asp Asp Lys Ser
    10                  15                  20

tgg aag gac gca agt ggt tgg tct aga atg cat gcg gcc ccg cga cgg 5250
Trp Lys Asp Ala Ser Gly Trp Ser Arg Met His Ala Ala Pro Arg Arg
25                  30                  35                  40

cgt gct gcg caa ccc tcc gac gct tcg ccg gcc gcg cag gtg gat cta 5298
Arg Ala Ala Gln Pro Ser Asp Ala Ser Pro Ala Ala Gln Val Asp Leu
                45                  50                  55

cgc acg ctc ggc tac agt cag cag cag caa gag aag atc aaa ccg aag 5346
Arg Thr Leu Gly Tyr Ser Gln Gln Gln Gln Glu Lys Ile Lys Pro Lys
            60                  65                  70

gtg cgt tcg aca gtg gcg cag cac cac gag gca ctg gtg ggc cat ggg 5394
Val Arg Ser Thr Val Ala Gln His His Glu Ala Leu Val Gly His Gly
        75                  80                  85

ttt aca cac gcg cac atc gtt gcg ctc agc caa cac ccg gca gcg tta 5442
Phe Thr His Ala His Ile Val Ala Leu Ser Gln His Pro Ala Ala Leu
    90                  95                  100

ggg acc gtc gct gtc acg tat cag cac ata atc acg gcg ttg cca gag 5490
Gly Thr Val Ala Val Thr Tyr Gln His Ile Ile Thr Ala Leu Pro Glu
105                 110                 115                 120

gcg aca cac gaa gac atc gtt ggc gtc ggc aaa cag tgg tcc ggc gca 5538
Ala Thr His Glu Asp Ile Val Gly Val Gly Lys Gln Trp Ser Gly Ala
                125                 130                 135

cgc gcc ctg gag gcc ttg ctc acg gat gcg ggg gag ttg aga ggt ccg 5586
Arg Ala Leu Glu Ala Leu Leu Thr Asp Ala Gly Glu Leu Arg Gly Pro
            140                 145                 150

ccg tta cag ttg gac aca ggc caa ctt gtg aag att gca aaa cgt ggc 5634
Pro Leu Gln Leu Asp Thr Gly Gln Leu Val Lys Ile Ala Lys Arg Gly
        155                 160                 165

ggc gtg acc gca atg gag gca gtg cat gca tcg cgc aat gcg ctc acg 5682
Gly Val Thr Ala Met Glu Ala Val His Ala Ser Arg Asn Ala Leu Thr
    170                 175                 180

gga gca ccc ctc aac ctg acc ccg gac cag gtg gtt gca atc gcg tca 5730
Gly Ala Pro Leu Asn Leu Thr Pro Asp Gln Val Val Ala Ile Ala Ser
185                 190                 195                 200

cac gat ggg gga aag cag gcc cta gaa acc gtt cag cga ctc ctg ccc 5778
His Asp Gly Gly Lys Gln Ala Leu Glu Thr Val Gln Arg Leu Leu Pro
                205                 210                 215

gtc ctg tgc cag gac cac ggc ctg acc ccc gaa cag gtt gtc gct att 5826
Val Leu Cys Gln Asp His Gly Leu Thr Pro Glu Gln Val Val Ala Ile
            220                 225                 230

gct agt aac ggc gga ggc aaa cag gcg ctg gaa aca gtt cag cgc ctc 5874
Ala Ser Asn Gly Gly Gly Lys Gln Ala Leu Glu Thr Val Gln Arg Leu
        235                 240                 245

ttg ccg gtc ttg tgt cag gcc cac ggc ctg acc ccg gac cag gtg gtt 5922
Leu Pro Val Leu Cys Gln Ala His Gly Leu Thr Pro Asp Gln Val Val
    250                 255                 260

gca atc gcg tca cac gat ggg gga aag cag gcc cta gaa acc gtt cag 5970
Ala Ile Ala Ser His Asp Gly Gly Lys Gln Ala Leu Glu Thr Val Gln
265                 270                 275                 280

cga ctc ctg ccc gtc ctg tgc cag gcc cac ggc ctg acc cca gcc cag 6018
Arg Leu Leu Pro Val Leu Cys Gln Ala His Gly Leu Thr Pro Ala Gln
                285                 290                 295

gtt gtg gcc atc gcc agc aac ata ggt ggc aag cag gcc ctc gaa acc 6066
Val Val Ala Ile Ala Ser Asn Ile Gly Gly Lys Gln Ala Leu Glu Thr
            300                 305                 310

gtc cag aga ctg tta ccg gtt ctc tgc cag gac cac ggc ctg acc ccc 6114
Val Gln Arg Leu Leu Pro Val Leu Cys Gln Asp His Gly Leu Thr Pro
        315                 320                 325

gac cag gtt gtc gct att gct agt aac ggc gga ggc aaa cag gcg ctg 6162
Asp Gln Val Val Ala Ile Ala Ser Asn Gly Gly Gly Lys Gln Ala Leu
    330                 335                 340

gaa aca gtt cag cgc ctc ttg ccg gtc ttg tgt cag gac cac ggc ctg 6210
Glu Thr Val Gln Arg Leu Leu Pro Val Leu Cys Gln Asp His Gly Leu
345                 350                 355                 360

acc ccg gaa cag gtg gtt gca atc gcg tca cac gat ggg gga aag cag 6258
Thr Pro Glu Gln Val Val Ala Ile Ala Ser His Asp Gly Gly Lys Gln
                365                 370                 375

gcc cta gaa acc gtt cag cga ctc ctg ccc gtc ctg tgc cag gcc cac 6306
Ala Leu Glu Thr Val Gln Arg Leu Leu Pro Val Leu Cys Gln Ala His
            380                 385                 390

ggc ctg acc ccc gac cag gtt gtc gct att gct agt aac ggc gga ggc 6354
Gly Leu Thr Pro Asp Gln Val Val Ala Ile Ala Ser Asn Gly Gly Gly

395                 400                 405

aaa cag gcg ctg gaa aca gtt cag cgc ctc ttg ccg gtc ttg tgt cag          6402
Lys Gln Ala Leu Glu Thr Val Gln Arg Leu Leu Pro Val Leu Cys Gln
    410                 415                 420

gcc cac ggc ctg acc ccg gcc cag gtg gtt gca atc gcg tca cac gat          6450
Ala His Gly Leu Thr Pro Ala Gln Val Val Ala Ile Ala Ser His Asp
425                 430                 435                 440

ggg gga aag cag gcc cta gaa acc gtt cag cga ctc ctg ccc gtc ctg          6498
Gly Gly Lys Gln Ala Leu Glu Thr Val Gln Arg Leu Leu Pro Val Leu
                445                 450                 455

tgc cag gac cac ggc ctg acc ccg gac cag gtg gtt gca atc gcg tca          6546
Cys Gln Asp His Gly Leu Thr Pro Asp Gln Val Val Ala Ile Ala Ser
            460                 465                 470

cac gat ggg gga aag cag gcc cta gaa acc gtt cag cga ctc ctg ccc          6594
His Asp Gly Gly Lys Gln Ala Leu Glu Thr Val Gln Arg Leu Leu Pro
        475                 480                 485

gtc ctg tgc cag gac cac ggc ctg acc ccg gaa cag gtg gtt gca atc          6642
Val Leu Cys Gln Asp His Gly Leu Thr Pro Glu Gln Val Val Ala Ile
    490                 495                 500

gcg tca cac gat ggg gga aag cag gcc cta gaa acc gtt cag cga ctc          6690
Ala Ser His Asp Gly Gly Lys Gln Ala Leu Glu Thr Val Gln Arg Leu
505                 510                 515                 520

ctg ccc gtc ctg tgc cag gcc cac ggc ctg acc cca gac caa gtt gtc          6738
Leu Pro Val Leu Cys Gln Ala His Gly Leu Thr Pro Asp Gln Val Val
                525                 530                 535

gcg att gca agc aac aac gga ggc aaa caa gcc tta gaa aca gtc cag          6786
Ala Ile Ala Ser Asn Asn Gly Gly Lys Gln Ala Leu Glu Thr Val Gln
            540                 545                 550

aga ttg ttg cct gtg ctg tgc caa gcc cac ggc ctg acc ccc gcc cag          6834
Arg Leu Leu Pro Val Leu Cys Gln Ala His Gly Leu Thr Pro Ala Gln
        555                 560                 565

gtt gtc gct att gct agt aac ggc gga ggc aaa cag gcg ctg gaa aca          6882
Val Val Ala Ile Ala Ser Asn Gly Gly Gly Lys Gln Ala Leu Glu Thr
    570                 575                 580

gtt cag cgc ctc ttg ccg gtc ttg tgt cag gac cac ggc ctg acc cca          6930
Val Gln Arg Leu Leu Pro Val Leu Cys Gln Asp His Gly Leu Thr Pro
585                 590                 595                 600

gac caa gtt gtc gcg att gca agc aac aac gga ggc aaa caa gcc tta          6978
Asp Gln Val Val Ala Ile Ala Ser Asn Asn Gly Gly Lys Gln Ala Leu
                605                 610                 615

gaa aca gtc cag aga ttg ttg ccg gtg ctg tgc caa gac cac ggc ctg          7026
Glu Thr Val Gln Arg Leu Leu Pro Val Leu Cys Gln Asp His Gly Leu
            620                 625                 630

acc cca gaa cag gtt gtg gcc atc gcc agc aac ata ggt ggc aag cag          7074
Thr Pro Glu Gln Val Val Ala Ile Ala Ser Asn Ile Gly Gly Lys Gln
        635                 640                 645

gcc ctc gaa acc gtc cag aga ctg tta ccg gtt ctc tgc cag gcc cac          7122

Ala Leu Glu Thr Val Gln Arg Leu Leu Pro Val Leu Cys Gln Ala His
    650                 655                 660

ggc ctg acc cca gac caa gtt gtc gcg att gca agc aac aac gga ggc      7170
Gly Leu Thr Pro Asp Gln Val Val Ala Ile Ala Ser Asn Asn Gly Gly
665                 670                 675                 680

aaa caa gcc tta gaa aca gtc cag aga ttg ttg cct gtg ctg tgc caa      7218
Lys Gln Ala Leu Glu Thr Val Gln Arg Leu Leu Pro Val Leu Cys Gln
                685                 690                 695

gcc cac ggc ctg acc ccg gcc cag gtg gtt gca atc gcg tca cac gat      7266
Ala His Gly Leu Thr Pro Ala Gln Val Val Ala Ile Ala Ser His Asp
            700                 705                 710

ggg gga aag cag gcc cta gaa acc gtt cag cga ctc ctg ccc gtc ctg      7314
Gly Gly Lys Gln Ala Leu Glu Thr Val Gln Arg Leu Leu Pro Val Leu
        715                 720                 725

tgc cag gac cac ggc ctg acg cct gag cag gta gtg gct att gca tcc      7362
Cys Gln Asp His Gly Leu Thr Pro Glu Gln Val Val Ala Ile Ala Ser
    730                 735                 740

aac gga ggg ggc aga ccc gca ctg gag tca atc gtg gcc cag ctt tcg      7410
Asn Gly Gly Gly Arg Pro Ala Leu Glu Ser Ile Val Ala Gln Leu Ser
745                 750                 755                 760

agg ccg gac ccc gcg ctg gcc gca ctc act aat gat cat ctt gta gcg      7458
Arg Pro Asp Pro Ala Leu Ala Ala Leu Thr Asn Asp His Leu Val Ala
                765                 770                 775

ctg gcc tgc ctc ggc gga cgt cct gcc atg gat gca gtg aaa aag gga      7506
Leu Ala Cys Leu Gly Gly Arg Pro Ala Met Asp Ala Val Lys Lys Gly
            780                 785                 790

ttg ccg cac gcg ccg gaa ttg atc aga tcc cag cta gtg aaa tct gaa      7554
Leu Pro His Ala Pro Glu Leu Ile Arg Ser Gln Leu Val Lys Ser Glu
        795                 800                 805

ttg gaa gag aag aaa tct gaa ctt aga cat aaa ttg aaa tat gtg cca      7602
Leu Glu Glu Lys Lys Ser Glu Leu Arg His Lys Leu Lys Tyr Val Pro
    810                 815                 820

cat gaa tat att gaa ttg att gaa atc gca aga aat tca act cag gat      7650
His Glu Tyr Ile Glu Leu Ile Glu Ile Ala Arg Asn Ser Thr Gln Asp
825                 830                 835                 840

aga atc ctt gaa atg aag gtg atg gag ttc ttt atg aag gtt tat ggt      7698
Arg Ile Leu Glu Met Lys Val Met Glu Phe Phe Met Lys Val Tyr Gly
                845                 850                 855

tat cgt ggt aaa cat ttg ggt gga tca agg aaa cca gac gga gca att      7746
Tyr Arg Gly Lys His Leu Gly Gly Ser Arg Lys Pro Asp Gly Ala Ile
            860                 865                 870

tat act gtc gga tct cct att gat tac ggt gtg atc gtt gat act aag      7794
Tyr Thr Val Gly Ser Pro Ile Asp Tyr Gly Val Ile Val Asp Thr Lys
        875                 880                 885

gca tat tca gga ggt tat aat ctt cca att ggt caa gca gat gaa atg      7842
Ala Tyr Ser Gly Gly Tyr Asn Leu Pro Ile Gly Gln Ala Asp Glu Met
    890                 895                 900

caa aga tat gtc gaa gag aat caa aca aga aac aag cat atc aac cct     7890
Gln Arg Tyr Val Glu Glu Asn Gln Thr Arg Asn Lys His Ile Asn Pro
905                 910                 915                 920

aat gaa tgg tgg aaa gtc tat cca tct tca gta aca gaa ttt aag ttc     7938
Asn Glu Trp Trp Lys Val Tyr Pro Ser Ser Val Thr Glu Phe Lys Phe
                925                 930                 935

ttg ttt gtg agt ggt cat ttc aaa gga aac tac aaa gct cag ctt aca     7986
Leu Phe Val Ser Gly His Phe Lys Gly Asn Tyr Lys Ala Gln Leu Thr
            940                 945                 950

aga ttg aat cat atc act aat tgt aat gga gct gtt ctt agt gta gaa     8034
Arg Leu Asn His Ile Thr Asn Cys Asn Gly Ala Val Leu Ser Val Glu
        955                 960                 965

gag ctt ttg att ggt gga gaa atg att aaa gct ggt aca ttg aca ctt     8082
Glu Leu Leu Ile Gly Gly Glu Met Ile Lys Ala Gly Thr Leu Thr Leu
    970                 975                 980

gag gaa gtg aga agg aaa ttt aat aac ggt gag ata aac ttt taa         8127
Glu Glu Val Arg Arg Lys Phe Asn Asn Gly Glu Ile Asn Phe
985                 990                 995

aaaatcagcc tcgactgtgc cttctagttg cc                                 8159


<210>  4
<211>  998
<212>  PRT
<213>  Artificial Sequence

<220>
<223>  Synthetic Construct

<400>  4

Met Ala Ser Ser Pro Pro Lys Lys Lys Arg Lys Val Ala Ala Ala Asp
1               5                   10                  15

Tyr Lys Asp Asp Asp Asp Lys Ser Trp Lys Asp Ala Ser Gly Trp Ser
            20                  25                  30

Arg Met His Ala Ala Pro Arg Arg Arg Ala Ala Gln Pro Ser Asp Ala
        35                  40                  45

Ser Pro Ala Ala Gln Val Asp Leu Arg Thr Leu Gly Tyr Ser Gln Gln
    50                  55                  60

Gln Gln Glu Lys Ile Lys Pro Lys Val Arg Ser Thr Val Ala Gln His
65                  70                  75                  80

His Glu Ala Leu Val Gly His Gly Phe Thr His Ala His Ile Val Ala
                85                  90                  95

Leu Ser Gln His Pro Ala Ala Leu Gly Thr Val Ala Val Thr Tyr Gln
            100                 105                 110

His Ile Ile Thr Ala Leu Pro Glu Ala Thr His Glu Asp Ile Val Gly
        115                 120                 125

Val Gly Lys Gln Trp Ser Gly Ala Arg Ala Leu Glu Ala Leu Leu Thr
    130                 135                 140

Asp Ala Gly Glu Leu Arg Gly Pro Pro Leu Gln Leu Asp Thr Gly Gln
145                 150                 155                 160

Leu Val Lys Ile Ala Lys Arg Gly Gly Val Thr Ala Met Glu Ala Val
                165                 170                 175

His Ala Ser Arg Asn Ala Leu Thr Gly Ala Pro Leu Asn Leu Thr Pro
            180                 185                 190

Asp Gln Val Val Ala Ile Ala Ser His Asp Gly Gly Lys Gln Ala Leu
        195                 200                 205

Glu Thr Val Gln Arg Leu Leu Pro Val Leu Cys Gln Asp His Gly Leu
    210                 215                 220

Thr Pro Glu Gln Val Val Ala Ile Ala Ser Asn Gly Gly Gly Lys Gln
225                 230                 235                 240

Ala Leu Glu Thr Val Gln Arg Leu Leu Pro Val Leu Cys Gln Ala His
                245                 250                 255

Gly Leu Thr Pro Asp Gln Val Val Ala Ile Ala Ser His Asp Gly Gly
            260                 265                 270

Lys Gln Ala Leu Glu Thr Val Gln Arg Leu Leu Pro Val Leu Cys Gln
        275                 280                 285

Ala His Gly Leu Thr Pro Ala Gln Val Val Ala Ile Ala Ser Asn Ile
    290                 295                 300

Gly Gly Lys Gln Ala Leu Glu Thr Val Gln Arg Leu Leu Pro Val Leu
305                 310                 315                 320

Cys Gln Asp His Gly Leu Thr Pro Asp Gln Val Val Ala Ile Ala Ser
                325                 330                 335

Asn Gly Gly Gly Lys Gln Ala Leu Glu Thr Val Gln Arg Leu Leu Pro
            340                 345                 350

Val Leu Cys Gln Asp His Gly Leu Thr Pro Glu Gln Val Val Ala Ile
        355                 360                 365

Ala Ser His Asp Gly Gly Lys Gln Ala Leu Glu Thr Val Gln Arg Leu
    370                 375                 380

Leu Pro Val Leu Cys Gln Ala His Gly Leu Thr Pro Asp Gln Val Val
385                 390                 395                 400

Ala Ile Ala Ser Asn Gly Gly Gly Lys Gln Ala Leu Glu Thr Val Gln
                405                 410                 415

Arg Leu Leu Pro Val Leu Cys Gln Ala His Gly Leu Thr Pro Ala Gln
            420                 425                 430

Val Val Ala Ile Ala Ser His Asp Gly Gly Lys Gln Ala Leu Glu Thr
        435                 440                 445

Val Gln Arg Leu Leu Pro Val Leu Cys Gln Asp His Gly Leu Thr Pro
    450                 455                 460

Asp Gln Val Val Ala Ile Ala Ser His Asp Gly Gly Lys Gln Ala Leu
465                 470                 475                 480

Glu Thr Val Gln Arg Leu Leu Pro Val Leu Cys Gln Asp His Gly Leu
                485                 490                 495

Thr Pro Glu Gln Val Val Ala Ile Ala Ser His Asp Gly Gly Lys Gln
            500                 505                 510

Ala Leu Glu Thr Val Gln Arg Leu Leu Pro Val Leu Cys Gln Ala His
        515                 520                 525

Gly Leu Thr Pro Asp Gln Val Val Ala Ile Ala Ser Asn Asn Gly Gly
    530                 535                 540

Lys Gln Ala Leu Glu Thr Val Gln Arg Leu Leu Pro Val Leu Cys Gln
545                 550                 555                 560

Ala His Gly Leu Thr Pro Ala Gln Val Val Ala Ile Ala Ser Asn Gly
                565                 570                 575

Gly Gly Lys Gln Ala Leu Glu Thr Val Gln Arg Leu Leu Pro Val Leu
            580                 585                 590

Cys Gln Asp His Gly Leu Thr Pro Asp Gln Val Val Ala Ile Ala Ser
        595                 600                 605

Asn Asn Gly Gly Lys Gln Ala Leu Glu Thr Val Gln Arg Leu Leu Pro
    610                 615                 620

Val Leu Cys Gln Asp His Gly Leu Thr Pro Glu Gln Val Val Ala Ile
625                 630                 635                 640

Ala Ser Asn Ile Gly Gly Lys Gln Ala Leu Glu Thr Val Gln Arg Leu
                645                 650                 655

Leu Pro Val Leu Cys Gln Ala His Gly Leu Thr Pro Asp Gln Val Val
            660                 665                 670

Ala Ile Ala Ser Asn Asn Gly Gly Lys Gln Ala Leu Glu Thr Val Gln
        675                 680                 685

Arg Leu Leu Pro Val Leu Cys Gln Ala His Gly Leu Thr Pro Ala Gln
    690                 695                 700

Val Val Ala Ile Ala Ser His Asp Gly Gly Lys Gln Ala Leu Glu Thr
705                 710                 715                 720

Val Gln Arg Leu Leu Pro Val Leu Cys Gln Asp His Gly Leu Thr Pro
                725                 730                 735

Glu Gln Val Val Ala Ile Ala Ser Asn Gly Gly Gly Arg Pro Ala Leu
            740                 745                 750

Glu Ser Ile Val Ala Gln Leu Ser Arg Pro Asp Pro Ala Leu Ala Ala
        755                 760                 765

Leu Thr Asn Asp His Leu Val Ala Leu Ala Cys Leu Gly Gly Arg Pro
    770                 775                 780

Ala Met Asp Ala Val Lys Lys Gly Leu Pro His Ala Pro Glu Leu Ile
785                 790                 795                 800

Arg Ser Gln Leu Val Lys Ser Glu Leu Glu Glu Lys Lys Ser Glu Leu
                805                 810                 815

Arg His Lys Leu Lys Tyr Val Pro His Glu Tyr Ile Glu Leu Ile Glu
            820                 825                 830

Ile Ala Arg Asn Ser Thr Gln Asp Arg Ile Leu Glu Met Lys Val Met
        835                 840                 845

Glu Phe Phe Met Lys Val Tyr Gly Tyr Arg Gly Lys His Leu Gly Gly
    850                 855                 860

Ser Arg Lys Pro Asp Gly Ala Ile Tyr Thr Val Gly Ser Pro Ile Asp
865                 870                 875                 880

Tyr Gly Val Ile Val Asp Thr Lys Ala Tyr Ser Gly Gly Tyr Asn Leu
                885                 890                 895

Pro Ile Gly Gln Ala Asp Glu Met Gln Arg Tyr Val Glu Glu Asn Gln
            900                 905                 910

Thr Arg Asn Lys His Ile Asn Pro Asn Glu Trp Trp Lys Val Tyr Pro
        915                 920                 925

Ser Ser Val Thr Glu Phe Lys Phe Leu Phe Val Ser Gly His Phe Lys
    930                 935                 940

Gly Asn Tyr Lys Ala Gln Leu Thr Arg Leu Asn His Ile Thr Asn Cys
945                 950                 955                 960

Asn Gly Ala Val Leu Ser Val Glu Glu Leu Leu Ile Gly Gly Glu Met
                965                 970                 975

Ile Lys Ala Gly Thr Leu Thr Leu Glu Glu Val Arg Arg Lys Phe Asn
            980                 985                 990

Asn Gly Glu Ile Asn Phe
        995

<210>  5
<211>  4860
<212>  DNA
<213>  Artificial Sequence

<220>
<223>  donor vector

<220>
<221>  CDS
<222>  (98)..(817)

<400>  5
cgccattctg cctggggacg tcggagcaag cttgatttag gtgacactat agaatacaag      60

ctacttgttc tttttgcagg atcccatcga tgccacc atg gtg agc aag ggc gag      115
                                         Met Val Ser Lys Gly Glu
                                         1               5

gag ctg ttc acc ggg gtg gtg ccc atc ctg gtc gag ctg gac ggc gac      163
Glu Leu Phe Thr Gly Val Val Pro Ile Leu Val Glu Leu Asp Gly Asp
            10                  15                  20

gta aac ggc cac aag ttc agc gtg tcc ggc gag ggc gag ggc gat gcc      211
Val Asn Gly His Lys Phe Ser Val Ser Gly Glu Gly Glu Gly Asp Ala

25                  30                  35

acc tac ggc aag ctg acc ctg aag ttc atc tgc acc acc ggc aag ctg      259
Thr Tyr Gly Lys Leu Thr Leu Lys Phe Ile Cys Thr Thr Gly Lys Leu
    40                  45                  50

ccc gtg ccc tgg ccc acc ctc gtg acc acc ctg acc tac ggc gtg cag      307
Pro Val Pro Trp Pro Thr Leu Val Thr Thr Leu Thr Tyr Gly Val Gln
55                  60                  65                  70

tgc ttc agc cgc tac ccc gac cac atg aag cag cac gac ttc ttc aag      355
Cys Phe Ser Arg Tyr Pro Asp His Met Lys Gln His Asp Phe Phe Lys
                75                  80                  85

tcc gcc atg ccc gaa ggc tac gtc cag gag cgc acc atc ttc ttc aag      403
Ser Ala Met Pro Glu Gly Tyr Val Gln Glu Arg Thr Ile Phe Phe Lys
            90                  95                  100

gac gac ggc aac tac aag acc cgc gcc gag gtg aag ttc gag ggc gac      451
Asp Asp Gly Asn Tyr Lys Thr Arg Ala Glu Val Lys Phe Glu Gly Asp
        105                 110                 115

acc ctg gtg aac cgc atc gag ctg aag ggc atc gac ttc aag gag gac      499
Thr Leu Val Asn Arg Ile Glu Leu Lys Gly Ile Asp Phe Lys Glu Asp
    120                 125                 130

ggc aac atc ctg ggg cac aag ctg gag tac aac tac aac agc cac aac      547
Gly Asn Ile Leu Gly His Lys Leu Glu Tyr Asn Tyr Asn Ser His Asn
135                 140                 145                 150

gtc tat atc atg gcc gac aag cag aag aac ggc atc aag gtg aac ttc      595
Val Tyr Ile Met Ala Asp Lys Gln Lys Asn Gly Ile Lys Val Asn Phe
                155                 160                 165

aag atc cgc cac aac atc gag gac ggc agc gtg cag ctc gcc gac cac      643
Lys Ile Arg His Asn Ile Glu Asp Gly Ser Val Gln Leu Ala Asp His
            170                 175                 180

tac cag cag aac acc ccc atc ggc gac ggc ccc gtg ctg ctg ccc gac      691
Tyr Gln Gln Asn Thr Pro Ile Gly Asp Gly Pro Val Leu Leu Pro Asp
        185                 190                 195

aac cac tac ctg agc acc cag tcc gcc ctg agc aaa gac ccc aac gag      739
Asn His Tyr Leu Ser Thr Gln Ser Ala Leu Ser Lys Asp Pro Asn Glu
    200                 205                 210

aag cgc gat cac atg gtc ctg ctg gag ttc gtg acc gcc gcc ggg atc      787
Lys Arg Asp His Met Val Leu Leu Glu Phe Val Thr Ala Ala Gly Ile
215                 220                 225                 230

act ctc ggc atg gac gag ctg tac aag taa tctagaacta tagtgagtcg      837
Thr Leu Gly Met Asp Glu Leu Tyr Lys
                235

tattacgtag atccagacat gataagatac attgatgagt ttggacaaac cacaactaga      897
atgcagtgaa aaaaatgctt tatttgtgaa atttgtgatg ctattgcttt atttgtaacc      957
attataagct gcaataaaca agttaacaac aacaattgca ttcattttat gtttcaggtt     1017
cagggggagg tgtgggaggt tttttaattc gcggcgcgcc gcggcgccaa tgcattgggc     1077
ccggtaccca gcttttgttc cctttagtga gggttaatac ttcttgctgc actgggaatt     1137
cagaaaacat gagagctcac gggagatgag tgcgcgcttg gcgtaatcat ggtcatagct     1197
gtttcctgtg tgaaattgtt atccgctcac aattccacac aacatacgag ccggaagcat     1257
aaagtgtaaa gcctggggtg cctaatgagt gagctaactc acattaattg cgttgcgctc     1317
actgcccgct ttccagtcgg gaaacctgtc gtgccagctg cattaatgaa tcggccaacg     1377

cgcggggaga ggcggtttgc gtattgggcg ctcttccgct tcctcgctca ctgactcgct 1437
gcgctcggtc gttcggctgc ggcgagcggt atcagctcac tcaaaggcgg taatacggtt 1497
atccacagaa tcaggggata acgcaggaaa gaacatgtga gcaaaaggcc agcaaaaggc 1557
caggaaccgt aaaaaggccg cgttgctggc gtttttccat aggctccgcc cccctgacga 1617
gcatcacaaa aatcgacgct caagtcagag gtggcgaaac ccgacaggac tataaagata 1677
ccaggcgttt ccccctggaa gctccctcgt gcgctctcct gttccgaccc tgccgcttac 1737
cggatacctg tccgcctttc tcccttcggg aagcgtggcg ctttctcata gctcacgctg 1797
taggtatctc agttcggtgt aggtcgttcg ctccaagctg ggctgtgtgc acgaaccccc 1857
cgttcagccc gaccgctgcg ccttatccgg taactatcgt cttgagtcca acccggtaag 1917
acacgactta tcgccactgg cagcagccac tggtaacagg attagcagag cgaggtatgt 1977
aggcggtgct acagagttct tgaagtggtg gcctaactac ggctacacta gaaggacagt 2037
atttggtatc tgcgctctgc tgaagccagt taccttcgga aaaagagttg gtagctcttg 2097
atccggcaaa caaaccaccg ctggtagcgg tggttttttt gtttgcaagc agcagattac 2157
gcgcagaaaa aaaggatctc aagaagatcc tttgatcttt tctacggggt ctgacgctca 2217
gtggaacgaa aactcacgtt aagggatttt ggtcatgaga ttatcaaaaa ggatcttcac 2277
ctagatcctt ttaaattaaa aatgaagttt taaatcaatc taaagtatat atgagtaaac 2337
ttggtctgac agttaccaat gcttaatcag tgaggcacct atctcagcga tctgtctatt 2397
tcgttcatcc atagttgcct gactccccgt cgtgtagata actacgatac gggagggctt 2457
accatctggc cccagtgctg caatgatacc gcgagaccca cgctcaccgg ctccagattt 2517
atcagcaata aaccagccag ccggaagggc cgagcgcaga agtggtcctg caactttatc 2577
cgcctccatc cagtctatta attgttgccg ggaagctaga gtaagtagtt cgccagttaa 2637
tagtttgcgc aacgttgttg ccattgctac aggcatcgtg gtgtcacgct cgtcgtttgg 2697
tatggcttca ttcagctccg gttcccaacg atcaaggcga gttacatgat cccccatgtt 2757
gtgcaaaaaa gcggttagct ccttcggtcc tccgatcgtt gtcagaagta agttggccgc 2817
agtgttatca ctcatggtta tggcagcact gcataattct cttactgtca tgccatccgt 2877
aagatgcttt tctgtgactg gtgagtactc aaccaagtca ttctgagaat agtgtatgcg 2937
gcgaccgagt tgctcttgcc cggcgtcaat acgggataat accgcgccac atagcagaac 2997
tttaaaagtg ctcatcattg gaaaacgttc ttcggggcga aaactctcaa ggatcttacc 3057
gctgttgaga tccagttcga tgtaacccac tcgtgcaccc aactgatctt cagcatcttt 3117
tactttcacc agcgtttctg ggtgagcaaa aacaggaagg caaaatgccg caaaaaaggg 3177
aataagggcg acacggaaat gttgaatact catactcttc ctttttcaat attattgaag 3237
catttatcag ggttattgtc tcatgagcgg atacatattt gaatgtattt agaaaaataa 3297
acaaataggg gttccgcgca catttccccg aaaagtgcca cctaaattgt aagcgttaat 3357
attttgttaa aattcgcgtt aaatttttgt taaatcagct cattttttaa ccaataggcc 3417
gaaatcggca aaatccctta taaatcaaaa gaatagaccg agatagggtt gagtgttgtt 3477
ccagtttgga acaagagtcc actattaaag aacgtggact ccaacgtcaa agggcgaaaa 3537
accgtctatc agggcgatgg cccactacgt gaaccatcac cctaatcaag ttttttgggg 3597
tcgaggtgcc gtaaagcact aaatcggaac cctaaaggga gcccccgatt tagagcttga 3657
cggggaaagc cggcgaacgt ggcgagaaag gaagggaaga aagcgaaagg agcgggcgct 3717
agggcgctgg caagtgtagc ggtcacgctg cgcgtaacca ccacacccgc cgcgcttaat 3777
gcgccgctac agggcgcgtc ccattcgcca ttcaggctgc gcaactgttg ggaagggcga 3837
tcggtgcggg cctcttcgct attacgccag tcgatcgacc atagccaatt caatatggcg 3897
tatatggact catgccaatt caatatggtg gatctggacc tgtgccaatt caatatggcg 3957
tatatggact cgtgccaatt caatatggtg gatctggacc ccagccaatt caatatggcg 4017
gacttggcac catgccaatt caatatggcg gacttggcac tgtgccaact ggggaggggt 4077
ctacttggca cggtgccaag tttgaggagg ggtcttggcc ctgtgccaag tccgccatat 4137
tgaattggca tggtgccaat aatggcggcc atattggcta tatgccagga tcaatatata 4197
ggcaatatcc aatatggccc tatgccaata tggctattgg ccaggttcaa tactatgtat 4257
tggccctatg ccatatagta ttccatatat gggttttcct attgacgtag atagcccctc 4317
ccaatgggcg gtcccatata ccatatatgg ggcttcctaa taccgcccat agccactccc 4377
ccattgacgt caatggtctc tatatatggt ctttcctatt gacgtcatat gggcggtcct 4437
attgacgtat atggcgcctc ccccattgac gtcaattacg gtaaatggcc cgcctggctc 4497
aatgcccatt gacgtcaata ggaccaccca ccattgacgt caatgggatg gctcattgcc 4557
cattcatatc cgttctcacg ccccctattg acgtcaatga cggtaaatgg cccacttggc 4617
agtacatcaa tatctattaa tagtaacttg gcaagtacat tactattggc aagtacgcca 4677
agggtacatt ggcagtactc ccattgacgt caatggcggt aaatggcccg cgatggctgc 4737
caagtacatc cccattgacg tcaatgggga ggggcaatga cgcaaatggg cgttccattg 4797
acgtaaatgg gcggtaggcg tgcctaatgg gaggtctata taagcaatgc tcgtttaggg 4857
aac 4860

<210> 6
<211> 239
<212> PRT

<213>  Artificial Sequence

<220>
<223>  Synthetic Construct

<400>  6

Met Val Ser Lys Gly Glu Glu Leu Phe Thr Gly Val Val Pro Ile Leu
1               5                   10                  15

Val Glu Leu Asp Gly Asp Val Asn Gly His Lys Phe Ser Val Ser Gly
            20                  25                  30

Glu Gly Glu Gly Asp Ala Thr Tyr Gly Lys Leu Thr Leu Lys Phe Ile
        35                  40                  45

Cys Thr Thr Gly Lys Leu Pro Val Pro Trp Pro Thr Leu Val Thr Thr
    50                  55                  60

Leu Thr Tyr Gly Val Gln Cys Phe Ser Arg Tyr Pro Asp His Met Lys
65                  70                  75                  80

Gln His Asp Phe Phe Lys Ser Ala Met Pro Glu Gly Tyr Val Gln Glu
                85                  90                  95

Arg Thr Ile Phe Phe Lys Asp Asp Gly Asn Tyr Lys Thr Arg Ala Glu
            100                 105                 110

Val Lys Phe Glu Gly Asp Thr Leu Val Asn Arg Ile Glu Leu Lys Gly
        115                 120                 125

Ile Asp Phe Lys Glu Asp Gly Asn Ile Leu Gly His Lys Leu Glu Tyr
    130                 135                 140

Asn Tyr Asn Ser His Asn Val Tyr Ile Met Ala Asp Lys Gln Lys Asn
145                 150                 155                 160

Gly Ile Lys Val Asn Phe Lys Ile Arg His Asn Ile Glu Asp Gly Ser
                165                 170                 175

Val Gln Leu Ala Asp His Tyr Gln Gln Asn Thr Pro Ile Gly Asp Gly
            180                 185                 190

Pro Val Leu Leu Pro Asp Asn His Tyr Leu Ser Thr Gln Ser Ala Leu
        195                 200                 205

Ser Lys Asp Pro Asn Glu Lys Arg Asp His Met Val Leu Leu Glu Phe
    210                 215                 220

Val Thr Ala Ala Gly Ile Thr Leu Gly Met Asp Glu Leu Tyr Lys
225                 230                 235


<210>  7
<211>  45
<212>  DNA
<213>  Artificial Sequence

<220>
<223>  Xltyr-CMVEGFP-F

<400>  7
aacatgagag ctcacgggag atgagtgcgc gcttggcgta atcat                    45

<210>  8
<211>  47
<212>  DNA
<213>  Artificial Sequence

<220>
<223>  Xltyr-CMVEGFP-R

<400>  8
ttctgaattc ccagtgcagc aagaagtatt aaccctcact aaaggga                47


<210>  9
<211>  25
<212>  DNA
<213>  Artificial Sequence

<220>
<223>  tyr-genomic F

<400>  9
ggagaggatg gcctctggag agata                                        25


<210>  10
<211>  24
<212>  DNA
<213>  Artificial Sequence

<220>
<223>  tyr-genomic R

<400>  10
ggtgggatgg attcctccca gaag                                         24


<210>  11
<211>  25
<212>  DNA
<213>  Artificial Sequence

<220>
<223>  pCS2-F

<400>  11
ataagataca ttgatgagtt tggac                                        25


<210>  12
<211>  25
<212>  DNA
<213>  Artificial Sequence

<220>
<223>  pCS2-R

<400>  12
atgcagctgg cacgacaggt ttccc                                        25


<210>  13

<211>  25
<212>  DNA
<213>  Artificial Sequence

<220>
<223>  oligonucleotide 13

<400>  13
caccgctctc acaggccacc cccca                                              25


<210>  14
<211>  25
<212>  DNA
<213>  Artificial Sequence

<220>
<223>  oligonucleotide 14

<400>  14
aaactggggg gtggcctgtg agagc                                              25


<210>  15
<211>  25
<212>  DNA
<213>  Artificial Sequence

<220>
<223>  oligonucleotide 15

<400>  15
caccgtggat ccgtggggtg gcccc                                              25


<210>  16
<211>  25
<212>  DNA
<213>  Artificial Sequence

<220>
<223>  oligonucleotide 16

<400>  16
aaacggggcc accccacgga tccac                                              25


<210>  17
<211>  24
<212>  DNA
<213>  Artificial Sequence

<220>
<223>  oligonucleotide 17

<400>  17
caccggtgcc tgaccaaggt gccc                                               24


<210>  18
<211>  24
<212>  DNA
<213>  Artificial Sequence

<220>
<223>  oligonucleotide 18

<400>  18
aaacgggcac cttggtcagg cacc                                              24


<210>  19
<211>  28
<212>  DNA
<213>  Artificial Sequence

<220>
<223>  primer 19

<400>  19
acaccaagac agacatctct gtcccttg                                          28


<210>  20
<211>  22
<212>  DNA
<213>  Artificial Sequence

<220>
<223>  primer 20
<400>  20
atccgtatcc aatgtgggga ac                                                22


<210>  21
<211>  22
<212>  DNA
<213>  Artificial Sequence

<220>
<223>  primer 21

<400>  21
ccgcaacctc cccttctacg ag                                                22


<210>  22
<211>  23
<212>  DNA
<213>  Artificial Sequence

<220>
<223>  primer 22

<400>  22
tcagcaggtc aaggggagga atg                                               23


<210>  23
<211>  5966
<212>  DNA
<213>  Artificial Sequence

<220>
<223>  donor vector

<400> 23
ctcatgacca aaatccctta acgtgagtta cgcgcgcgtc gttccactga gcgtcagacc 60
ccgtagaaaa gatcaaagga tcttcttgag atcctttttt tctgcgcgta atctgctgct 120
tgcaaacaaa aaaaccaccg ctaccagcgg tggtttgttt gccggatcaa gagctaccaa 180
ctctttttcc gaaggtaact ggcttcagca gagcgcagat accaaatact gttcttctag 240
tgtagccgta gttagcccac cacttcaaga actctgtagc accgcctaca tacctcgctc 300
tgctaatcct gttaccagtg gctgctgcca gtggcgataa gtcgtgtctt accgggttgg 360
actcaagacg atagttaccg gataaggcgc agcggtcggg ctgaacgggg ggttcgtgca 420
cacagcccag cttggagcga acgacctaca ccgaactgag atacctacag cgtgagctat 480
gagaaagcgc cacgcttccc gaagggagaa aggcggacag gtatccggta agcggcaggg 540
tcggaacagg agagcgcacg agggagcttc cagggggaaa cgcctggtat ctttatagtc 600
ctgtcgggtt tcgccacctc tgacttgagc gtcgattttt gtgatgctcg tcaggggggc 660
ggagcctatg gaaaaacgcc agcaacgcgg cctttttacg gttcctggcc ttttgctggc 720
cttttgctca catgttcttt cctgcgttat cccctgattc tgtggataac cgtattaccg 780
cctttgagtg agctgatacc gctcgccgca gccgaacgac cgagcgcagc gagtcagtga 840
gcgaggaagc ggaaggcgag agtagggaac tgccaggcat caaactaagc agaaggcccc 900
tgacggatgg cctttttgcg tttctacaaa ctctttctgt gttgtaaaac gacggccagt 960
cttaagctcg ggccccctgg gcggttctga taacgagtaa tcgttaatcc gcaaataacg 1020
taaaaacccg cttcggcggg tttttttatg gggggagttt agggaaagag catttgtcag 1080
aatatttaag ggcgcctgtc actttgcttg atatatgaga attatttaac cttataaatg 1140
agaaaaaagc aacgcacttt aaataagata cgttgctttt tcgattgatg aacacctata 1200
attaaactat tcatctatta tttatgattt tttgtatata caatatttct agtttgttaa 1260
agagaattaa gaaaataaat ctcgaaaata ataaagggaa aatcagtttt tgatatcaaa 1320
attatacatg tcaacgataa tacaaaatat aatacaaact ataagatgtt atcagtattt 1380
attatcattt agaataaatt ttgtgtcgcc cttaattgtg agcggataac aattacgagc 1440
ttcatgcaca gtggcgttga cattgattat tgactagtta ttaatagtaa tcaattacgg 1500
ggtcattagt tcatagccca tatatggagt tccgcgttac atacccgggg ccaccccacg 1560
gatccatggt gagtaaggga gaggaagata atatggcctc ccttcccgct acgcacgaac 1620
tccacatctt cgggtcaatc aacggtgttg acttcgacat ggtgggccag ggcaccggca 1680
atcccaatga cggatacgaa gaactcaatt tgaagagtac aaagggcgat ctccaattct 1740
caccttggat tctggttccc cacattggat acggatttca tcagtacctg ccgtaccccg 1800
atgggatgag cccatttcag gctgcaatgg tagatggtag cggttaccaa gtacaccgaa 1860
ctatgcaatt tgaggacggt gcctcactga cagtgaacta tcggtatact tacgaaggaa 1920
gccacatcaa gggagaggca caggtcaaag gaaccggatt tccagccgac gggccagtca 1980
tgacaaactc cctgaccgcc gcagattggt gccgcagcaa aaagacctat ccaaatgaca 2040
agaccattat ctcgacattc aaatggagct acaccaccgg aaacggcaaa cgctatcggt 2100
ctaccgccag gacaacctac acatttgcaa aacctatggc cgcaaactat ctgaaaaacc 2160
agccgatgta tgtgttccga aagacggaat taaaacactc gaaaacagaa ctaaacttta 2220
aagagtggca gaaagccttt accgacgtaa tgggcatgga cgagctgtat aagggaagcg 2280
gagagggcag aggaagtctg ctaacatgcg gtgacgtcga ggagaatcct ggacctatga 2340
ccgagtacaa gcccacggtg cgcctcgcca cccgcgacga cgtccccggg gccgtacgca 2400
ccctcgccgc cgcgttcgcc gactaccccg ccacgcgcca caccgtcgac ccggaccgcc 2460
acatcgagcg ggtcaccgag ctgcaagaac tcttcctcac gcgcgtcggg ctcgacatcg 2520
gcaaggtgtg ggtcgcggac gacggcgccg cggtggcggt ctggaccacg ccggagagcg 2580
tcgaagcggg ggcggtgttc gccgagatcg gcccgcgcat ggccgagttg agcggttccc 2640
ggctggccgc gcagcaacag atggaaggcc tcctggcgcc gcaccggccc aaggagcccg 2700
cgtggttcct ggccaccgtc ggcgtctcgc ccgaccacca gggcaagggt ctgggcagcg 2760
ccgtcgtgct ccccggagtg gaggcggccg agcgcgccgg ggtgcccgcc ttcctggaga 2820
cctccgcgcc ccgcaacctc cccttctacg agcggctcgg cttcaccgtc accgccgacg 2880
tcgaggtgcc cgaaggaccg cgcacctggt gcatgacccg caagcccggt gcctgaccaa 2940
ggtgcccggg tctagaatgc tgatgggcta gcaaaatcag cctcgactgt gccttctagt 3000
tgccagccat ctgttgtttg cccctccccc gtgccttcct tgaccctgga aggtgccact 3060
cccactgtcc tttcctaata aaatgaggaa attgcatcac aacactcaac cctatctcgg 3120
tctattcttt tgatttataa gggattttgc cgatttcggc ctattggtta aaaaatgagc 3180
tgatttaaca aaaatttaac gcgaattaat tctgtggaat gtgtgtcagt tagggtgtgg 3240
aaagtcccca ggctccccag caggcagaag tatgcaaagc atgcatctca attagtcagc 3300
aaccaggtgt ggaaagtccc caggctcccc agcaggcaga agtatgcaaa gcatgcatct 3360
caattagtca gcaaccatag tcccgcccct aactccgccc atcccgcccc taactccgcc 3420
cagttccgcc cattctccgc cccatggctg actaattttt tttatttatg cagaggccga 3480
ggccgcctct gcctctgagc tattccagaa gtagtgagga ggcttttttg gaggcctagg 3540
cttttgcaaa aagctcccgg gagcttgtat atccattttc ggatctgatc agcacgtgat 3600
gaaaaagcct gaactcaccg cgacgtctgt cgagaagttt ctgatcgaaa agttcgacag 3660
cgtttccgac ctgatgcagc tctcggaggg cgaagaatct cgtgctttca gcttcgatgt 3720

aggagggcgt ggatatgtcc tgcgggtaaa tagctgcgcc gatggtttct acaaagatcg 3780
ttatgtttat cggcactttg catcggccgc gctcccgatt ccggaagtgc ttgacattgg 3840
ggaattcagc gagagcctga cctattgcat ctcccgccgt gcacagggtg tcacgttgca 3900
agacctgcct gaaaccgaac tgcccgctgt tctgcagccg gtcgcggagg ccatggatgc 3960
gatcgctgcg gccgatctta gccagacgag cgggttcggc ccattcggac cgcaaggaat 4020
cggtcaatac actacatggc gtgatttcat atgcgcgatt gctgatcccc atgtgtatca 4080
ctggcaaact gtgatggacg acaccgtcag tgcgtccgtc gcgcaggctc tcgatgagct 4140
gatgctttgg gccgaggact gccccgaagt ccggcacctc gtgcacgcgg atttcggctc 4200
caacaatgtc ctgacggaca atggccgcat aacagcggtc attgactgga gcgaggcgat 4260
gttcggggat tcccaatacg aggtcgccaa catcttcttc tggaggccgt ggttggcttg 4320
tatggagcag cagacgcgct acttcgagcg gaggcatccg gagcttgcag gatcgccgcg 4380
gctccgggcg tatatgctcc gcattggtct tgaccaactc tatcagagct tggttgacgg 4440
caatttcgat gatgcagctt gggcgcaggg tcgatgcgac gcaatcgtcc gatccggagc 4500
cgggactgtc gggcgtacac aaatcgcccg cagaagcgcg gccgtctgga ccgatggctg 4560
tgtagaagta ctcgccgata gtggaaaccg acgccccagc actcgtccga gggcaaagga 4620
atagcacgtg ctacgagatt tcgattccac cgccgccttc tatgaaaggt tgggcttcgg 4680
aatcgttttc cgggacgccg gctggatgat cctccagcgc ggggatctca tgctggagtt 4740
cttcgcccac cccaacttgt ttattgcagc ttataatggt tacaaataaa gcaatagcat 4800
cacaaatttc acaaataaag catttttttc actgcattct agttgtggtt tgtccaaact 4860
catcaatgta tcttatcatg tctgtatacc gtcgacctct agctagagct tggcgtaatc 4920
atggtcatta ccaatgctta atcagtgagg cacctatctc agcgatctgt ctatttcgtt 4980
catccatagt tgcctgactc cccgtcgtgt agataactac gatacgggag ggcttaccat 5040
ctggccccag cgctgcgatg ataccgcgag aaccacgctc accggctccg gatttatcag 5100
caataaacca gccagccgga agggccgagc gcagaagtgg tcctgcaact ttatccgcct 5160
ccatccagtc tattaattgt tgccgggaag ctagagtaag tagttcgcca gttaatagtt 5220
tgcgcaacgt tgttgccatc gctacaggca tcgtggtgtc acgctcgtcg tttggtatgg 5280
cttcattcag ctccggttcc caacgatcaa ggcgagttac atgatccccc atgttgtgca 5340
aaaaagcggt tagctccttc ggtcctccga tcgttgtcag aagtaagttg gccgcagtgt 5400
tatcactcat ggttatggca gcactgcata attctcttac tgtcatgcca tccgtaagat 5460
gcttttctgt gactggtgag tactcaacca agtcattctg agaatagtgt atgcggcgac 5520
cgagttgctc ttgcccggcg tcaatacggg ataataccgc gccacatagc agaactttaa 5580
aagtgctcat cattggaaaa cgttcttcgg ggcgaaaact ctcaaggatc ttaccgctgt 5640
tgagatccag ttcgatgtaa cccactcgtg cacccaactg atcttcagca tcttttactt 5700
tcaccagcgt ttctgggtga gcaaaaacag gaaggcaaaa tgccgcaaaa aagggaataa 5760
gggcgacacg gaaatgttga atactcatat tcttcctttt tcaatattat tgaagcattt 5820
atcagggtta ttgtctcatg agcggataca tatttgaatg tatttagaaa aataaacaaa 5880
taggggtcag tgttacaacc aattaaccaa ttctgaacat tatcgcgagc ccatttatac 5940
ctgaatatgg ctcataacac cccttg 5966

<210> 24
<211> 50
<212> DNA
<213> Xenopus laevis

<400> 24
acttcttgct gcactgggaa catgagattc agaagctcac gggagatgag 50

<210> 25
<211> 52
<212> DNA
<213> Artificial Sequence

<220>
<223> TALEN target sequence in donor vector

<400> 25
acttcttgct gcactgggaa ttcagaaaac atgagagctc acgggagatg ag 52

<210> 26
<211> 43

<212>  DNA
<213>  Artificial Sequence

<220>
<223>  5′ junction sequence

<400>  26
acttcttgct gcactgggaa catgagagct cacgggagat gag                43


<210>  27
<211>  44
<212>  DNA
<213>  Artificial Sequence

<220>
<223>  3′ junction sequence

<400>  27
acttcttgct gcactgggaa attgagaagc tcacgggaga tgag               44


<210>  28
<211>  51
<212>  DNA
<213>  Unknown

<220>
<223>  TALEN target sequence in gene X


<220>
<221>  misc_feature
<222>  (2)..(50)
<223>  n is a, c, g, or t

<400>  28
tnnnnnnnnn nnnnnnnnnn nnnnnnnnnn nnnnnnnnnn nnnnnnnnnn a       51


<210>  29
<211>  51
<212>  DNA
<213>  Unknown

<220>
<223>  TALEN target sequence in gene X


<220>
<221>  misc_feature
<222>  (2)..(50)
<223>  n is a, c, g, or t

<400>  29
tnnnnnnnnn nnnnnnnnnn nnnnnnnnnn nnnnnnnnnn nnnnnnnnnn a       51


<210>  30
<211>  51
<212>  DNA
<213>  Artificial Sequence

<220>
<223>  TALEN target sequence in donor vector


<220>
<221>  misc_feature
<222>  (2)..(50)
<223>  n is a, c, g, or t

<400>  30
tnnnnnnnnn nnnnnnnnnn nnnnnnnnnn nnnnnnnnnn nnnnnnnnnn a                51


<210>  31
<211>  51
<212>  DNA
<213>  Artificial Sequence

<220>
<223>  TALEN target sequence in donor vector


<220>
<221>  misc_feature
<222>  (2)..(50)
<223>  n is a, c, g, or t

<400>  31
tnnnnnnnnn nnnnnnnnnn nnnnnnnnnn nnnnnnnnnn nnnnnnnnnn a                51


<210>  32
<211>  51
<212>  DNA
<213>  Artificial Sequence

<220>
<223>  TALEN target sequence in donor vector


<220>
<221>  misc_feature
<222>  (2)..(50)
<223>  n is a, c, g, or t

<400>  32
tnnnnnnnnn nnnnnnnnnn nnnnnnnnnn nnnnnnnnnn nnnnnnnnnn a                51


<210>  33
<211>  51
<212>  DNA
<213>  Artificial Sequence

<220>
<223>  TALEN target sequence in donor vector


<220>
<221>  misc_feature
<222>  (2)..(50)
<223>  n is a, c, g, or t

<400>  33
tnnnnnnnnn nnnnnnnnnn nnnnnnnnnn nnnnnnnnnn nnnnnnnnnn a                51

<210>  34
<211>  43
<212>  DNA
<213>  Artificial Sequence

<220>
<223>  Resulted sequence

<220>
<221>  misc_feature
<222>  (2)..(42)
<223>  n is a, c, g, or t

<400>  34
tnnnnnnnnn nnnnnnnnnn nnnnnnnnnn nnnnnnnnnn nna                         43

<210>  35
<211>  43
<212>  DNA
<213>  Artificial Sequence

<220>
<223>  Resulted sequence

<220>
<221>  misc_feature
<222>  (2)..(42)
<223>  n is a, c, g, or t

<400>  35
tnnnnnnnnn nnnnnnnnnn nnnnnnnnnn nnnnnnnnnn nna                         43

<210>  36
<211>  43
<212>  DNA
<213>  Artificial Sequence

<220>
<223>  Resulted sequence

<220>
<221>  misc_feature
<222>  (2)..(42)
<223>  n is a, c, g, or t

<400>  36
tnnnnnnnnn nnnnnnnnnn nnnnnnnnnn nnnnnnnnnn nna                         43

<210>  37
<211>  43
<212>  DNA
<213>  Artificial Sequence

<220>
<223>  Resulted sequence


<220>
<221>  misc_feature
<222>  (2)..(42)
<223>  n is a, c, g, or t

<400>  37
tnnnnnnnnn nnnnnnnnnn nnnnnnnnnn nnnnnnnnnn nna                          43


<210>  38
<211>  27
<212>  DNA
<213>  Unknown

<220>
<223>  gRNA target sequence in genomic sequence

<400>  38
gcatgcatga tgcatgcagt aggagta                                            27


<210>  39
<211>  27
<212>  DNA
<213>  Unknown

<220>
<223>  Complementary sequence of gRNA target sequence in genomic sequence

<400>  39
tactcctact gcatgcatca tgcatgc                                            27


<210>  40
<211>  25
<212>  DNA
<213>  Artificial Sequence

<220>
<223>  Complementary sequence of gRNA target sequence in donor vector

<400>  40
tgcctgtaat gcatgcgagc tagcc                                              25


<210>  41
<211>  25
<212>  DNA
<213>  Artificial Sequence

<220>
<223>  gRNA target sequence in donor vector

<400>  41
ggctagctcg catgcattac aggca                                              25


<210>  42
<211>  25

<212>  DNA
<213>  Artificial Sequence

<220>
<223>  gRNA target sequence in donor vector

<400>  42
acctgccata gtaggagctt aggat                                          25


<210>  43
<211>  25
<212>  DNA
<213>  Artificial Sequence

<220>
<223>  Complementary sequence of gRNA target sequence in donor vector

<400>  43
atcctaagct cctactatcc gaggt                                          25


<210>  44
<211>  36
<212>  DNA
<213>  Unknown

<220>
<223>  gRNA target sequence in gene X


<220>
<221>  misc_feature
<222>  (1)..(18)
<223>  n is a, c, g, or t

<220>
<221>  misc_feature
<222>  (21)..(26)
<223>  n is a, c, g, or t

<220>
<221>  misc_feature
<222>  (29)..(36)
<223>  n is a, c, g, or t

<400>  44
nnnnnnnnnn nnnnnnnngg nnnnnnggnn nnnnnn                              36


<210>  45
<211>  36
<212>  DNA
<213>  Unknown

<220>
<223>  Complementary sequence of gRNA target sequence in gene X


<220>
<221>  misc_feature
<222>  (1)..(8)
<223>  n is a, c, g, or t

<220>
<221>  misc_feature
<222>  (11)..(16)
<223>  n is a, c, g, or t

<220>
<221>  misc_feature
<222>  (19)..(36)
<223>  n is a, c, g, or t

<400>  45
nnnnnnnncc nnnnnnccnn nnnnnnnnnn nnnnnn                                36


<210>  46
<211>  36
<212>  DNA
<213>  Artificial Sequence

<220>
<223>  gRNA target sequence in donor vector


<220>
<221>  misc_feature
<222>  (1)..(18)
<223>  n is a, c, g, or t

<220>
<221>  misc_feature
<222>  (21)..(26)
<223>  n is a, c, g, or t

<220>
<221>  misc_feature
<222>  (29)..(36)
<223>  n is a, c, g, or t

<400>  46
nnnnnnnnnn nnnnnnnngg nnnnnnggnn nnnnnn                                36


<210>  47
<211>  36
<212>  DNA
<213>  Artificial Sequence

<220>
<223>  Complementary sequence of gRNA target sequence in donor vector


<220>
<221>  misc_feature
<222>  (1)..(8)
<223>  n is a, c, g, or t

<220>
<221>  misc_feature
<222>  (11)..(16)
<223>  n is a, c, g, or t

<220>

<221>  misc_feature
<222>  (19)..(36)
<223>  n is a, c, g, or t

<400>  47
nnnnnnnncc nnnnnnccnn nnnnnnnnnn nnnnnn                                36


<210>  48
<211>  30
<212>  DNA
<213>  Artificial Sequence

<220>
<223>  Resulted sequence


<220>
<221>  misc_feature
<222>  (1)..(18)
<223>  n is a, c, g, or t

<220>
<221>  misc_feature
<222>  (21)..(30)
<223>  n is a, c, g, or t

<400>  48
nnnnnnnnnn nnnnnnnngg nnnnnnnnnn                                       30


<210>  49
<211>  30
<212>  DNA
<213>  Artificial Sequence

<220>
<223>  Resulted sequence


<220>
<221>  misc_feature
<222>  (1)..(10)
<223>  n is a, c, g, or t

<220>
<221>  misc_feature
<222>  (13)..(30)
<223>  n is a, c, g, or t

<400>  49
nnnnnnnnnn ccnnnnnnnn nnnnnnnnnn                                       30


<210>  50
<211>  30
<212>  DNA
<213>  Artificial Sequence

<220>
<223>  Resulted sequence

<220>
<221>  misc_feature
<222>  (1)..(18)
<223>  n is a, c, g, or t

<220>
<221>  misc_feature
<222>  (21)..(30)
<223>  n is a, c, g, or t

<400>  50
nnnnnnnnnn nnnnnnnngg nnnnnnnnnn                                        30


<210>  51
<211>  30
<212>  DNA
<213>  Artificial Sequence

<220>
<223>  Resulted sequence


<220>
<221>  misc_feature
<222>  (1)..(10)
<223>  n is a, c, g, or t

<220>
<221>  misc_feature
<222>  (13)..(30)
<223>  n is a, c, g, or t

<400>  51
nnnnnnnnnn ccnnnnnnnn nnnnnnnnnn                                        30


<210>  52
<211>  36
<212>  DNA
<213>  Unknown

<220>
<223>  gRNA target sequence in gene X


<220>
<221>  misc_feature
<222>  (1)..(24)
<223>  n is a, c, g, or t

<220>
<221>  misc_feature
<222>  (29)..(36)
<223>  n is a, c, g, or t

<400>  52
nnnnnnnnnn nnnnnnnnnn nnnnccggnn nnnnnn                                 36


<210>  53
<211>  36
<212>  DNA

<213>  Unknown

<220>
<223>  Complementary sequence of gRNA target sequence in gene X


<220>
<221>  misc_feature
<222>  (1)..(8)
<223>  n is a, c, g, or t

<220>
<221>  misc_feature
<222>  (13)..(36)
<223>  n is a, c, g, or t

<400>  53
nnnnnnnncc ggnnnnnnnn nnnnnnnnnn nnnnnn                                36


<210>  54
<211>  36
<212>  DNA
<213>  Artificial Sequence

<220>
<223>  Complementary sequence of gRNA target sequence in donor vector


<220>
<221>  misc_feature
<222>  (1)..(8)
<223>  n is a, c, g, or t

<220>
<221>  misc_feature
<222>  (13)..(36)
<223>  n is a, c, g, or t

<400>  54
nnnnnnnncc ggnnnnnnnn nnnnnnnnnn nnnnnn                                36


<210>  55
<211>  36
<212>  DNA
<213>  Artificial Sequence

<220>
<223>  gRNA target sequence in donor vector


<220>
<221>  misc_feature
<222>  (1)..(24)
<223>  n is a, c, g, or t

<220>
<221>  misc_feature
<222>  (29)..(36)
<223>  n is a, c, g, or t

<400>  55

nnnnnnnnnn nnnnnnnnnn nnnnccggnn nnnnnn                                36

<210>  56
<211>  30
<212>  DNA
<213>  Artificial Sequence

<220>
<223>  Resulted sequence

<220>
<221>  misc_feature
<222>  (1)..(30)
<223>  n is a, c, g, or t

<400>  56
nnnnnnnnnn nnnnnnnnnn nnnnnnnnnn                                        30

<210>  57
<211>  30
<212>  DNA
<213>  Artificial Sequence

<220>
<223>  Resulted sequence

<220>
<221>  misc_feature
<222>  (1)..(30)
<223>  n is a, c, g, or t

<400>  57
nnnnnnnnnn nnnnnnnnnn nnnnnnnnnn                                        30

<210>  58
<211>  30
<212>  DNA
<213>  Artificial Sequence

<220>
<223>  Resulted sequence

<220>
<221>  misc_feature
<222>  (1)..(16)
<223>  n is a, c, g, or t

<220>
<221>  misc_feature
<222>  (21)..(30)
<223>  n is a, c, g, or t

<400>  58
nnnnnnnnnn nnnnnnccgg nnnnnnnnnn                                        30

<210>  59

<211>  30
<212>  DNA
<213>  Artificial Sequence

<220>
<223>  Resulted sequence


<220>
<221>  misc_feature
<222>  (1)..(10)
<223>  n is a, c, g, or t

<220>
<221>  misc_feature
<222>  (15)..(30)
<223>  n is a, c, g, or t

<400>  59
nnnnnnnnnn ccggnnnnnn nnnnnnnnnn                                        30


<210>  60
<211>  62
<212>  DNA
<213>  Unknown

<220>
<223>  FokI-dCas9 target sequence in gene X


<220>
<221>  misc_feature
<222>  (1)..(60)
<223>  n is a, c, g, or t

<400>  60
nnnnnnnnnn nnnnnnnnnn nnnnnnnnnn nnnnnnnnnn nnnnnnnnnn nnnnnnnnnn      60

gg                                                                      62


<210>  61
<211>  62
<212>  DNA
<213>  Unknown

<220>
<223>  FokI-dCas9 target sequence in gene X


<220>
<221>  misc_feature
<222>  (1)..(60)
<223>  n is a, c, g, or t

<400>  61
nnnnnnnnnn nnnnnnnnnn nnnnnnnnnn nnnnnnnnnn nnnnnnnnnn nnnnnnnnnn      60

cc                                                                      62


<210>  62

<211>  62
<212>  DNA
<213>  Artificial Sequence

<220>
<223>  FokI-dCas9 target sequence in donor vector


<220>
<221>  misc_feature
<222>  (1)..(60)
<223>  n is a, c, g, or t

<400>  62
nnnnnnnnnn nnnnnnnnnn nnnnnnnnnn nnnnnnnnnn nnnnnnnnnn nnnnnnnnnn      60

gg                                                                      62


<210>  63
<211>  62
<212>  DNA
<213>  Artificial Sequence

<220>
<223>  FokI-dCas9 target sequence in donor vector


<220>
<221>  misc_feature
<222>  (1)..(60)
<223>  n is a, c, g, or t

<400>  63
nnnnnnnnnn nnnnnnnnnn nnnnnnnnnn nnnnnnnnnn nnnnnnnnnn nnnnnnnnnn      60

cc                                                                      62


<210>  64
<211>  54
<212>  DNA
<213>  Artificial Sequence

<220>
<223>  Resulted sequence


<220>
<221>  misc_feature
<222>  (1)..(52)
<223>  n is a, c, g, or t

<400>  64
nnnnnnnnnn nnnnnnnnnn nnnnnnnnnn nnnnnnnnnn nnnnnnnnnn nngg            54


<210>  65
<211>  54
<212>  DNA
<213>  Artificial Sequence

<220>

<223>  Resulted sequence

<220>
<221>  misc_feature
<222>  (1)..(52)
<223>  n is a, c, g, or t

<400>  65
nnnnnnnnnn nnnnnnnnnn nnnnnnnnnn nnnnnnnnnn nnnnnnnnnn nncc          54

<210>  66
<211>  54
<212>  DNA
<213>  Artificial Sequence

<220>
<223>  Resulted sequence

<220>
<221>  misc_feature
<222>  (1)..(52)
<223>  n is a, c, g, or t

<400>  66
nnnnnnnnnn nnnnnnnnnn nnnnnnnnnn nnnnnnnnnn nnnnnnnnnn nngg          54

<210>  67
<211>  54
<212>  DNA
<213>  Artificial Sequence

<220>
<223>  Resulted sequence

<220>
<221>  misc_feature
<222>  (1)..(52)
<223>  n is a, c, g, or t

<400>  67
nnnnnnnnnn nnnnnnnnnn nnnnnnnnnn nnnnnnnnnn nnnnnnnnnn nncc          54

<210>  68
<211>  55
<212>  DNA
<213>  Artificial Sequence

<220>
<223>  5' Expected sequence

<400>  68
accggtactt cttgctgcac tgggaacatg agagctcacg ggagatgagt gcgcg          55

<210>  69
<211>  55
<212>  DNA

<213>  Artificial Sequence

<220>
<223>  5′ Resulted sequence

<400>  69
accggtactt cttgctgcac tgggaacatg agagctcacg ggagatgagt gcgcg          55


<210>  70
<211>  55
<212>  DNA
<213>  Artificial Sequence

<220>
<223>  3′ Expected sequence

<400>  70
gttaatactt cttgctgcac tgggaattca gaagctcacg ggagatgaga atttt          55


<210>  71
<211>  55
<212>  DNA
<213>  Artificial Sequence

<220>
<223>  3′ Resulted sequence

<400>  71
gttaatactt cttgctgcac tgggaattca gaagctcacg ggagatgaga atttt          55


<210>  72
<211>  98
<212>  DNA
<213>  Artificial Sequence

<220>
<223>  3′ Resulted sequence

<400>  72
gttaatactt cttgctgcac tgggaattca gagtaccctg gcaccggtac ttcttgctgc     60

actgggaacc tgatgagctc acgggagatg agaatttt                             98


<210>  73
<211>  55
<212>  DNA
<213>  Artificial Sequence

<220>
<223>  5′ Expected sequence

<400>  73
accggtactt cttgctgcac tgggaacatg agagctcacg ggagatgagt gcgcg          55


<210>  74
<211>  45
<212>  DNA
<213>  Artificial Sequence

<220>
<223>  5' Resulted sequence

<400>  74
accggtactt cttgctgcac tgggaacatg ggagatgagt gcgcg                45


<210>  75
<211>  60
<212>  DNA
<213>  Artificial Sequence

<220>
<223>  5' Resulted sequence

<400>  75
accggtactt cttgctgcac tgggaacatg aagagagctc acgggagatg agtgcgcgct      60


<210>  76
<211>  55
<212>  DNA
<213>  Artificial Sequence

<220>
<223>  3' Expected sequence

<400>  76
gttaatactt cttgctgcac tgggaattca gaagctcacg ggagatgaga atttt          55


<210>  77
<211>  55
<212>  DNA
<213>  Artificial Sequence

<220>
<223>  3' Resulted sequence

<400>  77
gttaatactt cttgctgcac tgggaattca gaagctcacg ggagatgaga atttt          55


<210>  78
<211>  40
<212>  DNA
<213>  Homo sapiens

<400>  78
cttcctctca caggccaccc cccaaggtga agaactgaag                          40


<210>  79
<211>  40
<212>  DNA
<213>  Homo sapiens

<400>  79
cttcagttct tcaccttggg gggtggcctg tgagaggaag                          40


<210>  80

<211>  30
<212>  DNA
<213>  Artificial Sequence

<220>
<223>  Complementary sequence of gRNA target sequence in donor vector

<400>  80
cccggggcca ccccacggat ccatggtgag                                        30


<210>  81
<211>  30
<212>  DNA
<213>  Artificial Sequence

<220>
<223>  gRNA target sequence in donor vector

<400>  81
ctcaccatgg atccgtgggg tggccccggg                                        30


<210>  82
<211>  30
<212>  DNA
<213>  Artificial Sequence

<220>
<223>  gRNA target sequence in donor vector

<400>  82
caagcccggt gcctgaccaa ggtgcccggg                                        30


<210>  83
<211>  30
<212>  DNA
<213>  Artificial Sequence

<220>
<223>  Complementary sequence of gRNA target sequence in donor vector

<400>  83
cccgggcacc ttggtcaggc accgggcttg                                        30


<210>  84
<211>  30
<212>  DNA
<213>  Artificial Sequence

<220>
<223>  Resulted sequence

<400>  84
tcacaggcca ccccacggat ccatggtgag                                        30


<210>  85
<211>  30
<212>  DNA
<213>  Artificial Sequence

<220>
<223>  Resulted sequence

<400>  85
ctcaccatgg atccgtgggg tggcctgtga                                        30


<210>  86
<211>  30
<212>  DNA
<213>  Artificial Sequence

<220>
<223>  Resulted sequence

<400>  86
caagcccggt gcctgaccaa ggtgaagaac                                        30


<210>  87
<211>  30
<212>  DNA
<213>  Artificial Sequence

<220>
<223>  Resulted sequence

<400>  87
gttcttcacc ttggtcaggc accgggcttg                                        30


<210>  88
<211>  30
<212>  DNA
<213>  Artificial Sequence

<220>
<223>  5' Expected sequence

<400>  88
tcacaggcca ccccacggat ccatggtgag                                        30


<210>  89
<211>  30
<212>  DNA
<213>  Artificial Sequence

<220>
<223>  5' Resulted sequence

<400>  89
tcacaggcca ccccacggat ccatggtgag                                        30


<210>  90
<211>  21
<212>  DNA
<213>  Artificial Sequence

<220>
<223>  5' Resulted sequence

<400>  90
tcacaggcca cccccggtga g                                                21

<210>  91
<211>  39
<212>  DNA
<213>  Artificial Sequence

<220>
<223>  5' Resulted sequence

<400>  91
tcacaggcca cccctgccac cccacggatc catggtgag                             39

<210>  92
<211>  30
<212>  DNA
<213>  Artificial Sequence

<220>
<223>  3' Expected sequence

<400>  92
caagcccggt gcctgaccaa ggtgaagaac                                       30

<210>  93
<211>  23
<212>  DNA
<213>  Artificial Sequence

<220>
<223>  3' Resulted sequence

<400>  93
caagcccggc caaggtgaag aac                                              23

<210>  94
<211>  25
<212>  DNA
<213>  Artificial Sequence

<220>
<223>  3' Resulted sequence

<400>  94
caagcccggt gccaaggtga agaac                                            25

<210>  95
<211>  29
<212>  DNA
<213>  Artificial Sequence

<220>
<223>  3' Resulted sequence

<400>  95
caagcccggt gcctcccaag gtgaagaac                                        29

<210>  96
<211>  30
<212>  DNA
<213>  Artificial Sequence

<220>
<223>  3′ Resulted sequence

<400>  96
caagcccggt gcctgcccaa ggtgaagaac                                        30

## Description

### Technical Field

The present invention relates to a method for inserting a desired nucleic acid into a predetermined site in a nucleic acid contained in a cell, a vector for the method, a kit for the method, and a cell obtained by the method. Further, the present invention relates to an organism comprising a cell containing a desired nucleic acid and a method for producing the organism.

### Background Art

TALENs (TALE Nucleases), ZFNs (Zinc Finger Nucleases), and the like are known as polypeptides including a plurality of nuclease subunits formed of DNA binding domains and DNA cleavage domains (Patent Literatures 1 to 4 and Non-Patent Literature 1). As for these artificial nucleases, a plurality of adjacent DNA cleavage domains form multimers at each binding site of the DNA binding domains, and thereby catalyze double strand break of DNAs. Each of the DNA binding domains contains repeats of a plurality of DNA binding modules. Each of the DNA binding modules recognizes a specific base pair in the DNA strand. Accordingly, a specific nucleotide sequence can be specifically cleaved by appropriately designing a DNA binding module. Other known nucleases which specifically cleave the specific nucleotide sequence are an RNA-guided nuclease such as a CRISPR/Cas system (Non-Patent Literature 2) and an RNA-guided FokI nuclease with a FokI nuclease fused to the CRISPR/Cas system (FokI-dCas9) (Non-Patent Literature 3). Various genetic modifications such as gene deletion and insertion on a genomic DNA and mutation introduction are performed using errors and recombination during repair of breaks by these nucleases (refer to Patent Literatures 5 to 6 and Non-Patent Literature 4).

As methods for inserting a desired nucleic acid into a cell using an artificial nuclease, the methods described in Non-Patent Literatures 5 to 8 are known. Non-Patent Literature 5 describes a method for inserting a foreign DNA by homologous recombination using TALENs. Non-Patent Literature 6 describes a method for inserting a foreign DNA by homologous recombination using ZFNs. However, the vector used for homologous recombination is long-stranded and cannot be easily produced. Depending on the cells and organisms, the homologous recombination efficiency is sometimes low. Therefore, these methods can be used only for limited cells and organisms. In order to obtain a modified organism that stably has a cell with a desired nucleic acid inserted therein, it is effective to obtain an adult organism by introducing a target nucleic acid into an animal embryo and differentiating the embryo. However, the homologous recombination efficiency is low in the animal embryo, and thus these methods are inefficient. A known technique for introducing a foreign DNA into animal embryos is ssODN-mediated gene modification. In this technique, it is only possible to introduce a short DNA with about several 10 bp.

The method described in Non-Patent Literature 7 or 8 is a method for inserting a nucleic acid into a cell by using an artificial nuclease without using homologous recombination. Non-Patent Literature 7 discloses a method for inserting a foreign DNA by cleaving a nucleic acid in a cell and a foreign DNA to be inserted using the ZFNs and TALENs, and joining the cleaved sites of the nucleic acid and the foreign DNA by the action of non-homologous end joining (NHEJ). However, the method described in Non-Patent Literature 7 does not control the direction of the nucleic acid to be inserted, and the junction of the nucleic acid to be inserted is not accurate. In the method described in Non-Patent Literature 8, a single-stranded end formed from the nucleic acid in the cell by nuclease cleavage is joined to a single-stranded end formed from the foreign DNA by annealing them, in order to achieve the control of direction and accurate joining. However, the method described in Non-Patent Literature 8 requires use of heterodimeric ZFNs and heterodimeric TALENs in order to prevent a DNA after insertion from being cleaved again, and a highly-active homodimeric artificial nuclease cannot be used in this method. The method described in Non-Patent Literature 8 is not used to insert the desired nucleic acid into animal embryos. Further, in the method described in Non-Patent Literature 8, the single-stranded end is frequently annealed to a wrong site, and a cell in which a nucleic acid is accurately inserted is not frequently obtained. In this regard, Non-Patent Literatures 5 to 8 do not describe a method of using an RNA-guided nuclease such as a CRISPR/Cas system or an RNA-guided FokI nuclease such as FokI-dCas9.

### Citation List

### Patent Literatures

Patent Literature 1: PCT International Publication No. WO 2011-072246
Patent Literature 2: PCT International Publication No. WO 2011-154393
Patent Literature 3: PCT International Publication No. WO 2011-159369
Patent Literature 4: PCT International Publication No. WO 2012-093833
Patent Literature 5: Japanese Patent Application National Publication (Laid-Open) No. 2013-513389
Patent Literature 6: Japanese Patent Application National Publication (Laid-Open) No. 2013-529083

### Non-Patent Literatures

Non-Patent Literature 1: Nat Rev Genet. 2010 Sep; 11 (9): 636-46.
Non-Patent Literature 2: Nat Protoc. 2013 Nov; 8 (11): 2281-308.
Non-Patent Literature 3: Nat Biotechnol. 2014 Jun; 32 (6): 569-76.
Non-Patent Literature 4: Cell. 2011 Jul 22; 146 (2): 318-31.
Non-Patent Literature 5: Nat Biotechnol. 2011 Jul 7; 29 (8): 731-4.
Non-Patent Literature 6: Nat Biotechnol. 2009 Sep; 27 (9): 851-7.
Non-Patent Literature 7: Biotechnol Bioeng. 2013 Mar; 110 (3): 871-80.
Non-Patent Literature 8: Genome Res. 2013 Mar; 23 (3): 539-46.

### Summary of Invention

### Problems to be Solved by the Invention

Therefore, an object of the present invention includes to provide a method for inserting a desired nucleic acid into a predetermined site of a nucleic acid in each cell of various organisms accurately and easily without requiring any complicated step such as production of a long-stranded vector, the method also enables insertion of a relatively long-stranded nucleic acid and can be used in combination with the homodimeric nuclease including a DNA cleavage domain, the RNA-guided nuclease or the RNA-guided FokI nuclease.

### Means for Solving the Problems

The present inventors focused on a region formed of a first nucleotide sequence and a region formed of a second nucleotide sequence which sandwich a predetermined site in which a nucleic acid is to be inserted, and designed a nuclease that specifically cleaves a moiety including these regions included in a nucleic acid in a cell. Further, the present inventors designed a vector including a region formed of a first nucleotide sequence, a desired nucleic acid to be inserted and a region formed of a second nucleotide sequence in the stated order in the 5'-end to 3'-end direction. Then, the present inventors introduced the designed vector into the cell, allowed the nuclease to act on the cell, and thereby effected cleavage of the predetermined site in the nucleic acid in the cell. Further, they allowed the nuclease to act on the vector, resulting in production of a nucleic acid fragment including the region formed of the first nucleotide sequence, the desired nucleic acid and the region formed of the second nucleotide sequence in the stated order in the 5'-end to 3'-end direction. As a result, in the cell, the first nucleotide sequence in the nucleic acid in the cell and the first nucleotide sequence in the vector were joined by microhomology-mediated end joining (MMEJ), and the second nucleotide sequence in the nucleic acid in the cell and the second nucleotide sequence in the vector were joined by MMEJ. Accordingly, the desired nucleic acid was accurately inserted into the predetermined site of the nucleic acid of the cell. It was possible to perform the insertion step on relatively long-stranded nucleic acids of several kb or more. The used nuclease specifically cleaves the moiety including the region formed of the first nucleotide sequence and the region formed of the second nucleotide sequence in the nucleic acid in the cell before insertion. However, the linked nucleic acid does not include a part of the moiety because of insertion of the desired nucleic acid. Thus, the nucleic acid was not cleaved again by the nuclease present in the cell and was stably maintained, and insertion of the desired nucleic acid occurred at high frequency.

According to the method, the sequences are joined by microhomology-mediated end joining which functions in many cells. Consequently, a desired nucleic acid can be accurately inserted at high frequency into cells at the developmental stage or the like with low homologous recombination efficiency. The method can be applied to a wide range of organisms and cells. Further, according to the method, a vector for introducing a nuclease and a vector for inserting a nucleic acid can be simultaneously inserted into a cell and thus the operation is simple. Furthermore, according to the method, changes in the nucleic acid moiety in the cell due to microhomology-mediated end joining prevent the inserted nucleic acid from being cleaved again. As the DNA cleavage domain included in the nuclease, a highly active homodimeric domain can also be used, and a wide range of experimental materials can be selected.

That is, according to a first aspect of the present invention, there is provided a vector for inserting a desired nucleic acid into a predetermined site in a nucleic acid contained in a cell by a nuclease,
wherein the nucleic acid contained in the cell includes a region formed of a first nucleotide sequence, the predetermined site, and a region formed of a second nucleotide sequence in the stated order in a 5'-end to 3'-end direction,
wherein the nuclease specifically cleaves a moiety including the region formed of the first nucleotide sequence and the region formed of the second nucleotide sequence included in the cell, and
wherein the vector includes a region formed of a first nucleotide sequence, the desired nucleic acid, and a region formed of a second nucleotide sequence in the stated order in a 5'-end to 3'-end direction.

That is, according to a second aspect of the present invention, there is provided a vector for inserting a desired nucleic acid into a predetermined site in a nucleic acid contained in a cell by a nuclease including a first DNA binding domain and a second DNA binding domain,
wherein the nucleic acid contained in the cell includes a region formed of a first nucleotide sequence, the predetermined site, and a region formed of a second nucleotide sequence in the stated order in a 5'-end to 3'-end direction,
wherein the region formed of the first nucleotide sequence, the predetermined site and the region formed of the second nucleotide sequence in the nucleic acid contained in the cell are each located between a region formed of a nucleotide sequence recognized by the first DNA binding domain and a region formed of a nucleotide sequence recognized by the second DNA binding domain,
wherein the vector includes a region formed of a first nucleotide sequence, the desired nucleic acid, and a region formed of a second nucleotide sequence in the stated order in a 5'-end to 3'-end direction,
wherein the region formed of the first nucleotide sequence and the region formed of the second nucleotide sequence in the vector are each located between a region formed of a nucleotide sequence recognized by the first DNA binding domain and a region formed of a nucleotide sequence recognized by the second DNA binding domain, and
wherein the vector produces a nucleic acid fragment including the region formed of the first nucleotide sequence, the desired nucleic acid, and the region formed of the second nucleotide sequence in the stated order in the 5'-end to 3'-end direction by the nuclease.

Further, according to a third aspect of the present invention, there is provided the vector according to the first or second aspect, wherein the first nucleotide sequence in the nucleic acid contained in the cell and the first nucleotide sequence in the vector are joined by microhomology-mediated end joining (MMEJ), and the second nucleotide sequence in the nucleic acid contained in the cell and the second nucleotide sequence in the vector are joined by MMEJ, whereby the desired nucleic acid is inserted.

Further, according to a fourth aspect of the present invention, there is provided the vector according to the second aspect, wherein the nuclease is a homodimeric nuclease and the vector is a circular vector.

Further, according to a fifth aspect of the present invention, there is provided the vector according to the first aspect, wherein the nuclease is a Cas9 nuclease.

Further, according to a sixth aspect of the present invention, there is provided the vector according to the second aspect, wherein the nuclease is a TALEN.

Further, according to a seventh aspect of the present invention, there is provided a kit for inserting a desired nucleic acid into a predetermined site in a nucleic acid contained in a cell, comprising the vector according to any one of the first to sixth aspects and a vector for expressing a nuclease.

Further, according to an eighth aspect of the present invention, there is provided a method for inserting a desired nucleic acid into a predetermined site in a nucleic acid contained in a cell, including a step of introducing the vector according to any one of the first to sixth aspects and a vector for expressing a nuclease into a cell.

Further, according to a ninth aspect of the present invention, there is provided a cell obtained by the method according to the eighth aspect.

Further, according to a tenth aspect of the present invention, there is provided an organism comprising the cell according to the ninth aspect.

Further, according to an eleventh aspect of the present invention, there is provided a method for producing an organism comprising a desired nucleic acid, comprising a step of differentiating a cell obtained by the method according to the eighth aspect.

Further, according to a twelfth aspect of the present invention, there is provided an organism produced by the method according to the eleventh aspect.

The invention may also be understood by reference to the following clauses numbered 1-12.
1. A vector for inserting a desired nucleic acid into a predetermined site in a nucleic acid contained in a cell by a nuclease,
   wherein the nucleic acid contained in the cell includes a region formed of a first nucleotide sequence, the predetermined site, and a region formed of a second nucleotide sequence in the stated order in a 5'-end to 3'-end direction,
   wherein the nuclease specifically cleaves a moiety including the region formed of the first nucleotide sequence and the region formed of the second nucleotide sequence included in the cell, and
   wherein the vector includes a region formed of a first nucleotide sequence, the desired nucleic acid, and a region formed of a second nucleotide sequence in the stated order in a 5'-end to 3'-end direction.
2. A vector for inserting a desired nucleic acid into a predetermined site in a nucleic acid contained in a cell by a nuclease including a first DNA binding domain and a second DNA binding domain,
   wherein the nucleic acid contained in the cell includes a region formed of a first nucleotide sequence, the predetermined site, and a region formed of a second nucleotide sequence in the stated order in a 5'-end to 3'-end direction,
   wherein the region formed of the first nucleotide sequence, the predetermined site and the region formed of the second nucleotide sequence in the nucleic acid contained in the cell are each located between a region formed of a nucleotide sequence recognized by the first DNA binding domain and a region formed of a nucleotide sequence recognized by the second DNA binding domain,
   wherein the vector includes a region formed of a first nucleotide sequence, the desired nucleic acid, and a region formed of a second nucleotide sequence in the stated order in a 5'-end to 3'-end direction,
   wherein the region formed of the first nucleotide sequence and the region formed of the second nucleotide sequence in the vector are each located between a region formed of a nucleotide sequence recognized by the first DNA binding domain and a region formed of a nucleotide sequence recognized by the second DNA binding domain, and
   wherein the vector produces a nucleic acid fragment including the region formed of the first nucleotide sequence, the desired nucleic acid, and the region formed of the second nucleotide sequence in the stated order in the 5'-end to 3'-end direction by the nuclease.
3. The vector according to clause 1 or 2, wherein the first nucleotide sequence in the nucleic acid contained in the cell and the first nucleotide sequence in the vector are joined by microhomology-mediated end joining, and the second nucleotide sequence in the nucleic acid contained in the cell and the second nucleotide sequence in the vector are joined by microhomology-mediated end joining, whereby the desired nucleic acid is inserted.
4. The vector according to clause 2, wherein the nuclease is a homodimeric nuclease and the vector is a circular vector.
5. The vector according to clause 1, wherein the nuclease is a Cas9 nuclease.
6. The vector according to clause 2, wherein the nuclease is a TALEN.
7. A kit for inserting a desired nucleic acid into a predetermined site in a nucleic acid contained in a cell, comprising the vector according to any one of clauses 1 to 6 and a vector for expressing a nuclease.
8. A method for inserting a desired nucleic acid into a predetermined site in a nucleic acid contained in a cell, comprising a step of introducing the vector according to any one of clauses 1 to 6 and a vector for expressing a nuclease into a cell.
9. A cell obtained by the method according to clause 8.
10. An organism comprising the cell according to clause 9.
11. A method for producing an organism comprising a desired nucleic acid, comprising a step of differentiating a cell obtained by the method according to clause 8.
12. An organism produced by the method according to clause 11.

### Effects of the Invention

When the vector of the present invention is used, a desired nucleic acid can be accurately and easily inserted into a predetermined site of a nucleic acid in each cell of various organisms without requiring any complicated step such as production of a long-stranded vector, without depending on homologous recombination efficiency in cells or organisms, and without causing any frame shift. Relatively long-stranded nucleic acids of several kb or more can also be inserted. The method for inserting a nucleic acid using the vector of the present invention can be used in combination with a nuclease including a homodimeric DNA cleavage domain with high nuclease activity. Alternatively, the method for inserting a nucleic acid using the vector of the present invention can be used in combination with an RNA-guided nuclease such as a CRISPR/Cas system. Further, when the vector of the present invention is used, it is possible to accurately design a junction and to knock-in a functional domain with in-frame. Thus, when a nucleic acid containing a gene as a label is used, the organism subjected to target insertion can be easily identified by detecting expression of the gene. It is possible to easily obtain an organism with a desired nucleic acid inserted therein at high frequency. Further, the method for inserting a nucleic acid using the vector of the present invention can be used for undifferentiated cells such as animal embryos with low homologous recombination efficiency. Consequently, by inserting a desired nucleic acid into an undifferentiated cell using the vector of the present invention and differentiating the obtained undifferentiated cell, it is possible to easily obtain an adult organism that stably maintains the desired nucleic acid.

### Brief Description of Drawings

[Fig. 1] Fig. 1 is a schematic view illustrating target integration to a tyr locus in the case where the whole vector containing a desired nucleic acid is inserted using TALENs.
[Fig. 2] Fig. 2 is a schematic view illustrating the case where a part of the vector containing a desired nucleic acid is inserted using TALENs.
[Fig. 3] Fig. 3 is a schematic view of the design of the vector of the present invention using a CRISPR/Cas system.
[Fig. 4a] Fig. 4a is a schematic view of a case where the whole vector containing a desired nucleic acid is inserted using a CRISPR/Cas system.
[Fig. 4b] Fig. 4b is a schematic view of a case where the whole vector containing a desired nucleic acid is inserted using a CRISPR/Cas system.
Fig. 5 is a schematic view of a case where the whole vector containing a desired nucleic acid is inserted using a FokI-dCas9.
[Fig. 6] Fig. 6 illustrates phenotype of each embryonic into which TALENs and a vector for target integration (TAL-PITCh vector) have been microinjected. Figs. 6 illustrates bright field images (upper row) and GFP fluorescence images (lower row) of TALEN R + vector-injected embryos (negative control group; A) and TALEN mix + vector-injected embryos (experimental group; B).
[Fig. 7] Fig. 7 illustrates percentages of phenotypes in the negative control group and the experimental group. The phenotypes are classified into four groups (Full, Half, Mosaic and Non), except for abnormal embryo (gray, Abnormal). The number of individuals is shown at the top of each graph.
[Fig. 8] Fig. 8 illustrates detection of the introduction of the donor vector (TAL-PITCh vector) into a target gene locus. The lower views are photographs of electrophoresis of PCR products using primer sets at the upstream and downstream of a target sequence of tyrTALEN, and the sides of the vector. The upper view illustrates the positions of the primers. Each of the arrows in the lower views indicate a band that shows integration of each vector. The numeric characters correspond to individual numbers of Fig. 6.

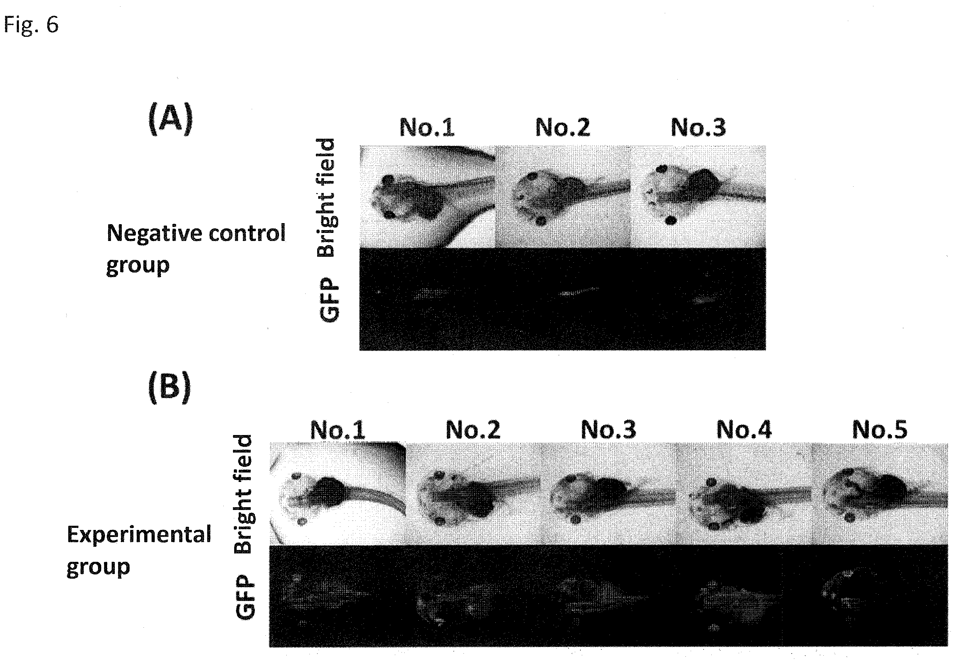

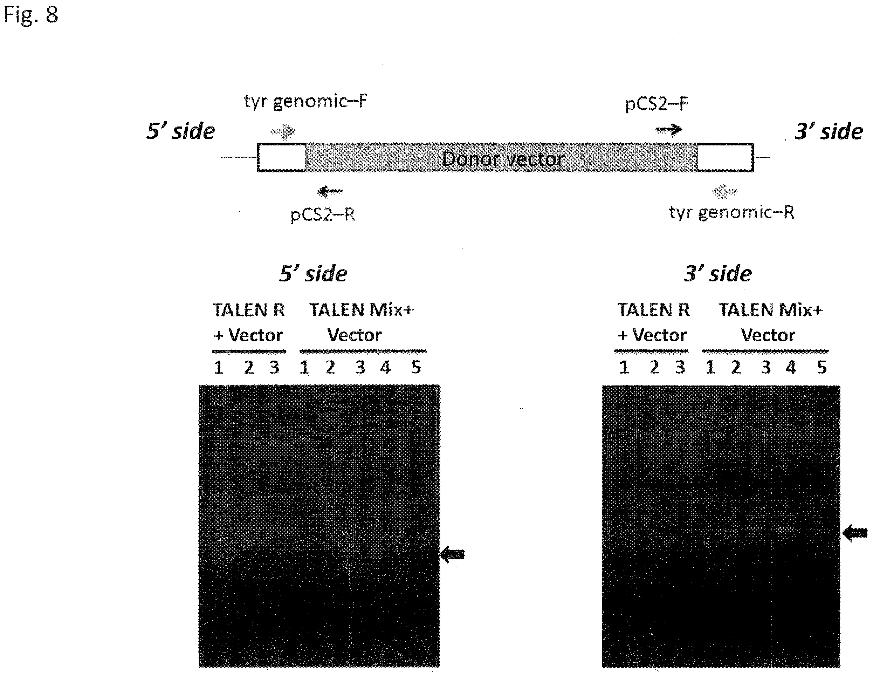

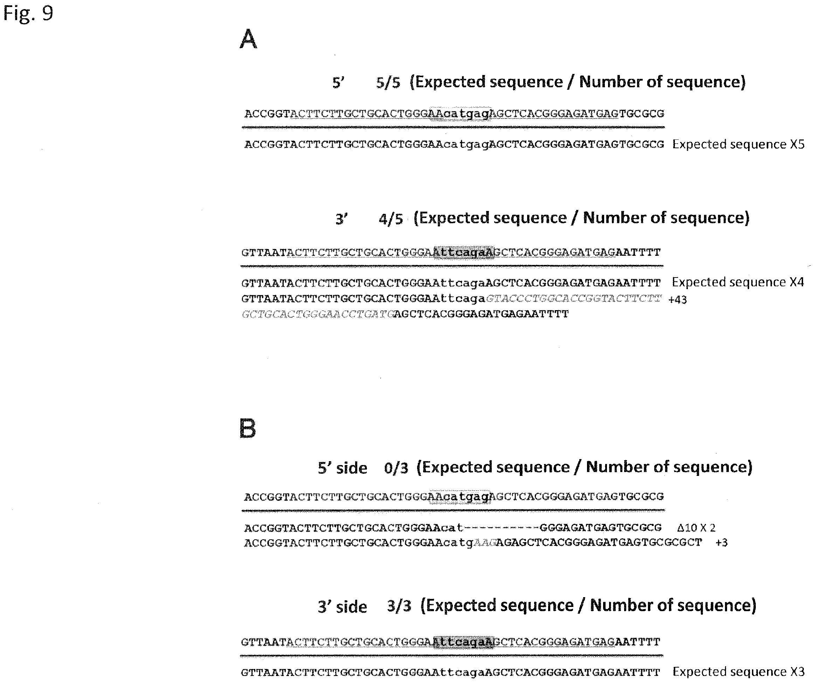

[Fig. 9] Figs. 9A and 9B illustrate sequence analysis of the junction between the insertion site and the donor vector (TAL-PITCh vector). The results of sequencing of PCR products (at the 5'-side and the 3'-side in Fig. 8) derived from Nos. 3 and 4 (Fig. 6) are shown. Sequences expected in MMEJ-dependent introduction are shown in the upper row. TALEN target sequences are underlined. Boxes near the center represent a spacer surrounding sequence shortened by MMEJ at the 5'-side and a spacer surrounding sequence shortened by MMEJ at the 3'-side, respectively. Each deletion is indicated by a dashed line (-), and each insertion is indicated by italics.
[Fig. 10] Fig. 10 is a schematic view of target integration to an FBL locus of a HEK293T cell using a CRISPR/Cas system.
[Fig. 11A] Figs. 11A and 11B illustrate the full length sequence of a donor vector (CRIS-PITCh vector). A mNeonGreen coding sequence is indicated in green, a 2A peptide coding sequence is indicated in purple and a puromycin resistance gene coding sequence is indicated in blue. A gRNA target sequence at the 5'-side and a gRNA target sequence at the 3'-side are underlined.
[Fig. 11B] Fig. 11B is a continuation of Fig. 11A.
[Fig. 12] Fig. 12 is a mNeonGreen fluorescence image showing a phenotype of a HEK293T cell in which a vector expressing three types of gRNAs and Cas9 and a donor vector (CRIS-PITCh vector) have been co-introduced.
[Fig. 13] Fig. 13 illustrates sequence analysis of the junction between the insertion site and the donor vector (CRIS-PITCh vector). The sequences expected in MMEJ-dependent introduction are shown in the upper row. Each deletion is indicated by a dashed line (-), each insertion is indicated by a double underline, and each substitution is indicated by an underline.

### Modes for Carrying Out the Invention

The vector provided by the first aspect of the present invention is a vector for inserting a desired nucleic acid into a predetermined site in a nucleic acid contained in a cell. Examples of the nucleic acid contained in the cell include genomic DNA in a cell. Examples of the cell origin include human; non-human mammals such as cow, miniature pig, pig, sheep, goat, rabbit, dog, cat, guinea pig, hamster, mouse, rat and monkey; birds; fish such as zebrafish; amphibia such as frog; reptiles; insects such as drosophila; and crustacea. Examples of the cell origin include plants such as *Arabidopsis thaliana.* The cell may be a cultured cell. The cell may be an immature cell, such as a pluripotent stem cell including an embryonic stem cell (ES cell) and an induced pluripotent stem cell (iPS cell), capable of differentiating into a more mature tissue cell. The embryonic stem cell and induced pluripotent stem cell can infinitely increase, and are useful as supply sources for a large amount of functional cells.

The cell into which the vector of the first aspect of the present invention is inserted includes a nucleic acid including a region formed of a first nucleotide sequence, a predetermined site in which a nucleic acid is to be inserted and a region formed of a second nucleotide sequence in the stated order in the 5'-end to 3'-end direction. The first nucleotide sequence and the second nucleotide sequence are expedient terms showing a relationship with the sequence included in the vector to be inserted. The first and second nucleotide sequences may be adjacent to the predetermined site directly or through a region consisting of a specific base sequence. When the first and second nucleotide sequences are adjacent to the predetermined site through the region consisting of a specific base sequence, the specific base sequence is preferably from 1 to 7 bases in length and more preferably from 1 to 3 bases in length. The first nucleotide sequence is preferably from 3 to 10 bases in length, more preferably from 4 to 8 bases in length, and even more preferably from 5 to 7 bases in length. The second nucleotide sequence is preferably from 3 to 10 bases in length, more preferably from 4 to 8 bases in length, and even more preferably from 5 to 7 bases in length.

The vector provided by the first aspect of the present invention is a vector for inserting a desired nucleic acid into a predetermined site in a nucleic acid contained in a cell using a nuclease. In the first aspect of the present invention, the nuclease specifically cleaves the moiety including the region formed of the first nucleotide sequence and the region formed of the second nucleotide sequence in the cell. Such a nuclease is, for example, a nuclease including a first DNA binding domain and a second DNA binding domain. This nuclease will be described in the section herein in which the vector provided by the second aspect of the present invention is described. Examples of another nuclease which performs the specific cleavage as described above include RNA-guided nucleases such as nucleases based on the CRISPR/Cas system. In the CRISPR/Cas system, a moiety called "PAM" is essential to cleave a double strand by the Cas9 nuclease. Examples of the Cas9 nuclease include SpCas9 derived from *Streptococcus pyogenes* and StCas9 derived from *Streptococcus thermophilus.* The PAM of SpCas9 is a "5'-NGG-3'" sequence (N represents any nucleotide) and a position where the double strand is cleaved is located at a position 3 bases upstream (at the 5'-end) of the PAM. A guide RNA (gRNA) in the CRISPR/Cas system recognizes a base sequence located at the 5'-side of the position where the double strand is cleaved. Then, the position where the double strand is cleaved in the CRISPR/Cas system corresponds to the predetermined site for inserting the desired nucleic acid in the nucleic acid contained in the cell. The region formed of the first nucleotide sequence and the region formed of the second nucleotide sequence are present at both ends of the predetermined site. Accordingly, the CRISPR/Cas system using the gRNA which recognizes the base sequence located at the 5'-end of the PAM contained in a nucleic acid in a cell can specifically cleave the moiety including the region formed of the first nucleotide sequence and the region formed of the second nucleotide sequence.

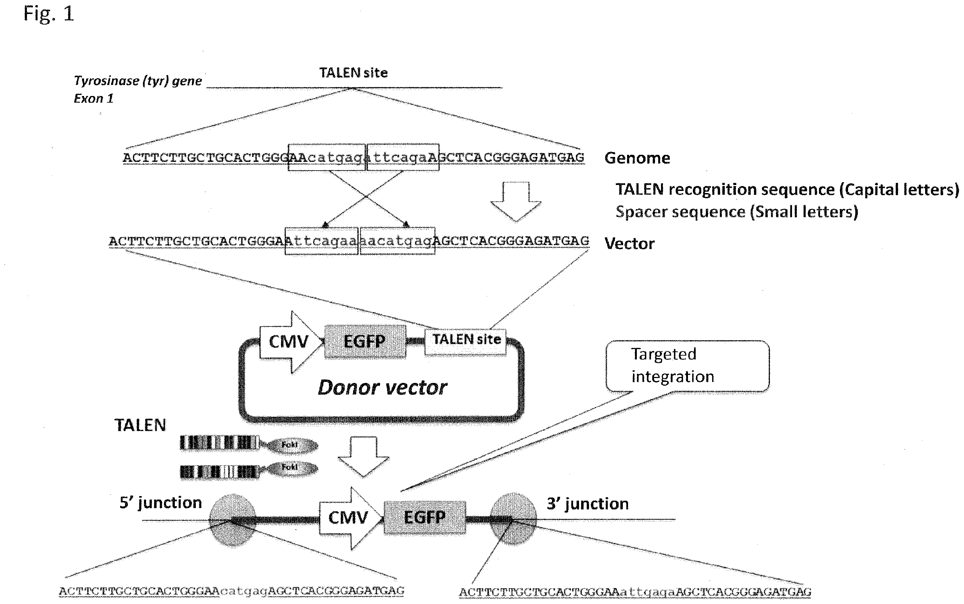

The vector provided by the first aspect of the present invention includes a region formed of a first nucleotide sequence, a desired nucleic acid to be inserted into a cell and a region formed of a second nucleotide sequence in the stated order in the 5'-end to 3'-end direction. The region formed of the first nucleotide sequence included in the vector is the same as the region formed of the first nucleotide sequence in the nucleic acid contained in the cell. The region formed of the second nucleotide sequence included in the vector is the same as the region formed of the second nucleotide sequence in the nucleic acid contained in the cell. A relationship between the first and second nucleotide sequences included in the vector and the first and second nucleotide sequences in the nucleic acid contained in the cell will be described using Fig. 1 as an example. "AAcatgag" contained in the TALEN site of Fig. 1 is a first nucleotide sequence. "AA" in the first nucleotide sequence is an overlap between the nucleotide sequence recognized by the first DNA binding domain and the first nucleotide sequence. "attcagaA" contained in the TALEN site of Fig. 1 is a second nucleotide sequence. The capital letter A of the second nucleotide sequence represents an overlap between the second nucleotide sequence and the nucleotide sequence recognized by the second DNA binding domain. On the other hand, "Attcagaa" contained in the donor vector of Fig. 1 is a second nucleotide sequence. The capital letter A included in the second nucleotide sequence represents an overlap between the nucleotide sequence recognized by the first DNA binding domain and the first nucleotide sequence. "aacatgaa" contained in the donor vector of Fig. 1 is a first nucleotide sequence. A sequence encoding CMV and EGFP contained in the donor vector of Fig. 1 is a desired nucleic acid to be inserted into a cell. As illustrated in the schematic view of the donor vector of Fig. 1, the donor vector will be described by defining the region formed of the first nucleotide sequence (aacatgag) as the starting point. The donor vector of Fig. 1 includes a region formed of a first nucleotide sequence, a desired nucleic acid to be inserted into a cell and a region formed of the second nucleotide sequence in the stated order in the 5'-end to 3'-end direction. The donor vector of Fig. 1 will be described in comparison to the TALEN site of Fig. 1. In the TALEN site, the 3'-end of the first nucleotide sequence is adjacent to or in contact with the 5'-end of the second nucleotide sequence. On the other hand, in the donor vector, the 3'-end of the second nucleotide sequence is adjacent to or in contact with the 5'-end of the first nucleotide sequence. In this regard, in an example of Fig. 1, a positional relationship between the first nucleotide sequence and the second nucleotide sequence in the nucleic acid in the cell is reversed, compared to a positional relationship between the first nucleotide sequence and the second nucleotide sequence in the vector. Such a relationship results from the fact that the donor vector of Fig. 1 is a circular vector and the nuclease cleaves a moiety including the region formed of the first nucleotide sequence and the region formed of the second nucleotide sequence in the vector. Thus, the vector of the first aspect of the present invention is preferably a circular vector. In the case where the vector of the first aspect of the present invention is a circular vector, the 3'-end of the second nucleotide sequence and the 5'-end of the first nucleotide sequence which are contained in the vector of the first aspect of the present invention are preferably adjacent or directly linked to each other. In the case where the vector of the first aspect of the present invention is a circular vector and the second nucleotide sequence is adjacent to the first nucleotide sequence, the 3'-end of the second nucleotide sequence is separated from the 5'-end of the first nucleotide sequence preferably by a region of from 1 to 7 bases in length, more preferably from 1 to 5 bases in length, and even more preferably from 1 to 3 bases in length.

The vector provided by the second aspect of the present invention is a vector for inserting a desired nucleic acid using a nuclease including a first DNA binding domain and a second DNA binding domain.

Examples of the origin of a DNA binding domain include TALEs (transcription activator-like effectors) of plant pathogen Xanthomonas and Zinc fingers. Preferably, the DNA binding domain continuously includes one or more DNA binding modules that specifically recognize base pairs from the N-terminus. One DNA binding module specifically recognizes one base pair. Therefore, the first DNA binding domain and the second DNA binding domain each recognize a region formed of a specific nucleotide sequence. The nucleotide sequence recognized by the first DNA binding domain and the nucleotide sequence recognized by the second DNA binding domain may be the same as or different from each other. The number of DNA binding modules included in the DNA binding domain is preferably from 8 to 40, more preferably from 12 to 25, and even more preferably from 15 to 20, from the viewpoint of compatibility between the level of nuclease activity and the level of DNA sequence recognition specificity of the DNA cleavage domain. The DNA binding module is, for example, a TAL effector repeat. Examples of the length of a DNA binding module include a length of from 20 to 45, a length of from 30 to 38, a length of from 32 to 36 and a length of 34. All the DNA binding modules included in the DNA binding domain are preferably identical in length. The first DNA binding domain and the second DNA binding domain are preferably identical in origin and characteristics.

In the case where the RNA-guided FokI nuclease (FokI-dCas9) is used, the FokI-dCas9 forming a complex with a gRNA corresponds to the nuclease including the DNA binding domain in the second aspect. The dCas9 is a Cas9 whose catalytic activity is inactivated. The dCas9 is guided by a gRNA recognizing a base sequence located near the site in which a double strand is cleaved, and is linked to a nucleic acid. That is, the dCas9 forming a complex with a gRNA corresponds to the DNA binding domain in the second aspect.

The nuclease including the first DNA binding domain and the second DNA binding domain preferably includes a first nuclease subunit including a first DNA binding domain and a first DNA cleavage domain and a second nuclease subunit including a second DNA binding domain and a second DNA cleavage domain.

Preferably, the first DNA cleavage domain and the second DNA cleavage domain approach each other to form a multimer after each of the first DNA binding domain and the second DNA binding domain is linked to a DNA, and acquires an improved nuclease activity. The DNA cleavage domain is, for example, a DNA cleavage domain derived from a restriction enzyme FokI. The DNA cleavage domain may be a heterodimeric DNA cleavage domain or may be a homodimeric DNA cleavage domain. When the first DNA cleavage domain and the second DNA cleavage domain approach each other, a multimer is formed and an improved nuclease activity is obtained. However, In the case where neither the multimer is formed nor the improved nuclease activity is obtained even if the first DNA cleavage domain and the first DNA cleavage domain approach each other, and neither the multimer is formed nor the improved nuclease activity is obtained even if the second DNA cleavage domain and the second DNA cleavage domain approach each other, each of the first DNA cleavage domain and the second DNA cleavage domain is a heterodimeric DNA cleavage domain. In the case where a multimer is formed and the nuclease activity is improved when the first DNA cleavage domain and the first DNA cleavage domain approach each other, the first DNA cleavage domain is a homodimeric DNA cleavage domain. In the case of using the homodimeric DNA cleavage domain, a high nuclease activity is generally obtained. The first DNA cleavage domain and the second DNA cleavage domain are preferably identical in origin and characteristics.

In the case of using a TALEN, the first DNA binding domain and the first DNA cleavage domain in the first nuclease subunit are linked by a polypeptide consisting of from 20 to 70 amino acids, from 25 to 65 amino acids or from 30 to 60 amino acids, preferably from 35 to 55 amino acids, more preferably from 40 to 50 amino acids, even more preferably from 45 to 49 amino acids, and most preferably 47 amino acids. In the case of using ZFN, the first DNA binding domain and the first DNA cleavage domain in the first nuclease subunit are linked by a polypeptide consisting of from 0 to 20 amino acids or from 2 to 10 amino acids, preferably from 3 to 9 amino acids, more preferably from 4 to 8 amino acids and even more preferably from 5 to 7 amino acids. In the case of using FokI-dCas9, the dCas9 and FokI in the first nuclease subunit are linked by a polypeptide consisting of from 1 to 20 amino acids, from 1 to 15 amino acids or from 1 to 10 amino acids, preferably from 2 to 8 amino acids, more preferably from 3 to 7 amino acids, even more preferably from 4 to 6 amino acids, and most preferably 5 amino acids. The same holds for the second nuclease subunit. The first nuclease subunit linked by such a length of polypeptide has high specificity to the length of the moiety including the region formed of the first nucleotide sequence and the region formed of the second nucleotide sequence, and specifically cleaves a spacer region having a specific length. Thus, the nucleic acid is not frequently inserted into a site outside the target site by nonspecific cleavage, and the nucleic acid joined by microhomology-mediated end joining as described later is not frequently cleaved again. This is preferable.

In the nucleic acid contained in the cell into which the vector provided by the second aspect of the present invention is to be inserted, the region formed of the first nucleotide sequence is located between the region formed of the nucleotide sequence recognized by the first DNA binding domain and the region formed of the nucleotide sequence recognized by the second DNA binding domain. Further, the region formed of the second nucleotide sequence is located between the region formed of the nucleotide sequence recognized by the first DNA binding domain and the region formed of the nucleotide sequence recognized by the second DNA binding domain. Further, the predetermined site is located between the region formed of the nucleotide sequence recognized by the first DNA binding domain and the region formed of the nucleotide sequence recognized by the second DNA binding domain. In the nucleic acid, a combination of two nucleotide sequences recognized by the DNA binding domain surrounding the region formed of the first nucleotide sequence may be different from a combination of two nucleotide sequences recognized by the DNA binding domain surrounding the region formed of the second nucleotide sequence. In this case, different nucleases are used as the nuclease for cleaving around the region formed of the first nucleotide sequence and the nuclease for cleaving around the region formed of the second nucleotide sequence. In the nucleic acid contained in the cell, the region formed of the nucleotide sequence recognized by the first DNA binding domain and the region formed of the nucleotide sequence recognized by the second DNA binding domain are separated by a region formed of a nucleotide sequence of preferably from 5 to 40 bases in length, more preferably from 10 to 30 bases in length, and even more preferably from 12 to 20 bases in length. The base length of the region separating both the regions may be the same as or different from the total of the base length of the first nucleotide sequence and the base length of the second nucleotide sequence. For example, in the nucleic acid contained in the cell, in the case where the following conditions are satisfied: the 3'-end of the first nucleotide sequence is directly in contact with the 5'-end of the second nucleotide sequence, there is no overlap between the nucleotide sequence recognized by the first DNA binding domain and the first nucleotide sequence, and there is no overlap between the second nucleotide sequence and the nucleotide sequence recognized by the second DNA binding domain, the base length of the region separating both the regions is the same as the total of the base length of the first nucleotide sequence and the base length of the second nucleotide sequence. However, in the case where one or more items selected from these conditions are not satisfied, the base length of the region separating both the regions is different from the total of the base length of the first nucleotide sequence and the base length of the second nucleotide sequence. The region formed of the first nucleotide sequence in the nucleic acid contained in the cell may partially overlap the region formed of the nucleotide sequence recognized by the first DNA binding domain. Further, the region formed of the second nucleotide sequence in the nucleic acid contained in the cell may partially overlap the region formed of the nucleotide sequence recognized by the second DNA binding domain. In the case where there is a partial overlap, the overlapping moiety consists of a nucleotide sequence of preferably from 1 to 6 bases in length, more preferably from 1 to 5 bases in length, and even more preferably from 2 to 4 bases in length. In the case where there is a partial overlap, the length of a moiety which separates two regions recognized by the DNA binding domain and includes the region formed of the first nucleotide sequence and the region formed of the second nucleotide sequence is greatly reduced by microhomology-mediated end joining as described later. Thus, the linked nucleic acid is hardly cleaved again and the inserted nucleic acid is more stably maintained. This is preferable.

In the vector provided by the second aspect of the present invention, the region formed of the first nucleotide sequence and the region formed of the second nucleotide sequence are each located between the region formed of the nucleotide sequence recognized by the first DNA binding domain and the region formed of the nucleotide sequence recognized by the second DNA binding domain. In the vector, a combination of two nucleotide sequences recognized by the DNA binding domain surrounding the region formed of the first nucleotide sequence may be different from a combination of two nucleotide sequences recognized by the DNA binding domain surrounding the region formed of the second nucleotide sequence. In this case, different nucleases are used as the nuclease for cleaving around the region formed of the first nucleotide sequence and the nuclease for cleaving around the region formed of the second nucleotide sequence. In the vector, the region formed of the nucleotide sequence recognized by the first DNA binding domain may be present at the 5'-end or the 3'-end as compared to the region formed of the nucleotide sequence recognized by the second DNA binding domain. However, in the vector, the nucleotide sequence that is located at the 3'-end of the first nuclease sequence and recognized by the first DNA binding domain or the second DNA binding domain is preferably different from the sequence that is located at the 3'-end of the second nucleotide sequence in the nucleic acid contained in the cell and recognized by the first DNA binding domain or the second DNA binding domain. Further, in the vector, the nucleotide sequence that is located at the 5'-end of the second nuclease sequence and recognized by the first DNA binding domain or the second DNA binding domain is preferably different from the sequence that is located at the 5'-end of the first nucleotide sequence in the nucleic acid contained in the cell and recognized by the first DNA binding domain or the second DNA binding domain. In these cases, the frequency of cleavage occurring again after insertion of a desired nucleic acid can be further reduced by using a nuclease including a heterodimeric DNA cleavage domain in combination. In the vector, one site may be cleaved, or two or more sites may be cleaved by one or more nucleases containing a first DNA binding domain and a second DNA binding domain. The vector cleaved at two sites is, for example, a vector including a region formed of a nucleotide sequence recognized by a first DNA binding domain, a region formed of a first nucleotide sequence, a region formed of a nucleotide sequence recognized by a second DNA binding domain, a desired nucleic acid to be inserted into a cell, the region formed of the nucleotide sequence recognized by the first DNA binding domain, a region formed of a second nucleotide sequence, and the region formed of the nucleotide sequence recognized by the second DNA binding domain in the stated order in the 5'-end to 3'-end direction. In the case of using the vector cleaved at two sites, unnecessary nucleic acids contained in the vector can be removed by nuclease cleavage. Consequently, it is possible to more safely obtain a desired cell containing no unnecessary nucleic acids.

In the vector provided by the second aspect of the present invention, the region that separates the region formed of the nucleotide sequence recognized by the first DNA binding domain from the region formed of the nucleotide sequence recognized by the second DNA binding domain and that includes the region formed of the first nucleotide sequence or the region formed of the second nucleotide sequence consists of a nucleotide sequence of preferably from 5 to 40 bases in length, more preferably from 10 to 30 bases in length, and even more preferably from 12 to 20 bases in length. In the case where the region that separates the region formed of the nucleotide sequence recognized by the first DNA binding domain from the region formed of the nucleotide sequence recognized by the second DNA binding domain includes both the first nucleotide sequence and the second nucleotide sequence, the base length of the region separating both the regions is the same or almost the same as the total of the base length of the first nucleotide sequence and the base length of the second nucleotide sequence. As described above, the first nucleotide sequence is preferably from 3 to 10 bases in length, more preferably from 4 to 8 bases in length, and even more preferably from 5 to 7 bases in length. As described above, the second nucleotide sequence is preferably from 3 to 10 bases in length, more preferably from 4 to 8 bases in length, and even more preferably from 5 to 7 bases in length. In the case where there is an overlap between the region formed of the first nucleotide sequence or the second nucleotide sequence and the region formed of the nucleotide sequence recognized by the DNA binding domain, the case where there is an overlap between the region formed of the first nucleotide sequence and the region formed of the second nucleotide sequence or the case where the region formed of the first nucleotide sequence is not directly linked to the region formed of the second nucleotide sequence, the base length of the region separating both the regions is not the same but almost the same as the total of the base length of the first nucleotide sequence and the base length of the second nucleotide sequence.

In the vector provided by the first or second aspect of the present invention, for example, the first nucleotide sequence in the nucleic acid contained in the cell and the first nucleotide sequence in the vector are joined by microhomology-mediated end joining (MMEJ), and the second nucleotide sequence in the nucleic acid contained in the cell and the second nucleotide sequence in the vector are joined by MMEJ, whereby the desired nucleic acid is inserted into a predetermined site in the nucleic acid contained in the cell.

In the vector provided by the second aspect of the present invention, for example, the nuclease is a homodimeric nuclease and the vector is a circular vector.

In the vector provided by the first aspect of the present invention, for example, the nuclease is an RNA-guided nuclease such as a nuclease based on the CRISPR/Cas system. Preferably, the nuclease is a Cas9 nuclease.

In the vector provided by the second aspect of the present invention, the nuclease is preferably a ZFN, a TALEN or FokI-dCas9, and more preferably a TALEN. The ZFN, TALEN or FokI-dCas9 may be homodimeric or heterodimeric. The nuclease is preferably a homodimeric ZFN, TALEN or FokI-dCas9, and more preferably a homodimeric TALEN.

The nucleases also include their mutants. Such a mutant may be any mutant as long as it exhibits the activity of the nuclease. The mutant is, for example, a nuclease containing the amino acid sequence in which several amino acids, such as 1, 2, 3, 4, 5, 6, 7, 8, 9, 10, 15, 20, 25, 30, 35, 40, 45 or 50 amino acids are substituted, deleted and/or added in the amino acid sequence of the nuclease.

A desired nucleic acid contained in the vector provided by the present invention is, for example, from 10 to 10000 bases in length and may be several kilo bases in length. The desired nucleic acid may also contain a nucleic acid encoding a gene. The gene encoded can be any gene. Examples thereof include genes encoding an enzyme converting a chemiluminescence substrate such as alkaline phosphatase, peroxidase, chloramphenicol acetyltransferase and β-galactosidase. The desired nucleic acid may contain a nucleic acid encoding a gene capable of detecting the expression level by the light signal. In this case, the presence or absence of the light signal in the cell after vector introduction is detected so that the success or failure of the insertion can be easily confirmed, and the efficiency and frequency of obtaining a cell having a desired nucleic acid inserted therein are improved. Examples of the gene capable of detecting the expression level by the light signal include genes encoding a fluorescent protein such as a green fluorescent protein (GFP), a humanized Renilla green fluorescent protein (hrGFP), an enhanced green fluorescent protein (eGFP), a yellowish green fluorescent protein (mNeonGreen), an enhanced blue fluorescent protein (eBFP), an enhanced cyan fluorescent protein (eCFP), an enhanced yellow fluorescent protein (eYFP) and a red fluorescent protein (RFP or DsRed); and genes encoding a bioluminescence protein such as firefly luciferase and Renilla luciferase.

In the vector provided by the present invention, it is preferable that the region formed of the first nucleotide sequence is directly adjacent to the desired nucleic acid. Further, it is preferable that the desired nucleic acid is directly adjacent to the region formed of the second nucleotide sequence. In the case where the desired nucleic acid contains a functional factor such as a gene, the first and second nucleotide sequences included in the vector may encode a part of the functional factor.

The vector provided by the present invention may be a circular vector or a linear vector. The vector provided by the present invention is preferably a circular vector. Examples of the vector of the present invention include a plasmid vector, a cosmid vector, a viral vector and an artificial chromosome vector. Examples of the artificial chromosome vector include yeast artificial chromosome vector (YAC), bacterial artificial chromosome vector (BAC), P1 artificial chromosome vector (PAC), mouse artificial chromosome vector (MAC) and human artificial chromosome vector (HAC). Examples of the component of the vector include a nucleic acid such as a DNA and an RNA; and a nucleic acid analog such as a GNA, an LNA, a BNA, a PNA and a TNA. The vector may be modified by components other than the nucleic acid, such as saccharides.

According to the seventh aspect, the present invention provides a kit for inserting a desired nucleic acid into a predetermined site in a nucleic acid contained in a cell. The kit according to the seventh aspect of the present invention comprises the vector according to any one of the first to sixth aspects. The kit according to the seventh aspect of the present invention further comprises a vector for expressing a nuclease. The vector for expressing a nuclease is, for example, a vector for expressing a nuclease including a first DNA binding domain and a second DNA binding domain. Examples of the vector for expressing a nuclease include a plasmid vector, a cosmid vector, a viral vector and an artificial chromosome vector. The vector for expressing a nuclease is, for example, a vector set comprising a first vector that contains a gene encoding a first nuclease subunit including a first DNA binding domain and a first DNA cleavage domain and a second vector that contains a gene encoding a second nuclease subunit including a second DNA binding domain and a second DNA cleavage domain. Another example is a vector including both of the gene encoding the first nuclease subunit and the gene encoding the second nuclease subunit. The first and second vectors may be present in different nucleic acid fragments or identical nucleic acid fragments. In the case where different nucleases are used as the nuclease for cleaving around the region formed of the first nucleotide sequence and the nuclease for cleaving around the region formed of the second nucleotide sequence, the kit of the seventh aspect of the present invention comprises a plurality of the vector sets including first and second vectors. In the case of using the nuclease based on the CRISPR/Cas system as a nuclease, the kit of the seventh aspect of the present invention may comprise: a vector for expressing a gRNA and a nuclease for cleaving around the region formed of the first nucleotide sequence in the vector of the first aspect of the present invention; a vector for expressing a gRNA and a nuclease for cleaving around the region formed of the second nucleotide sequence in the vector of the first aspect of the present invention; and a a vector for expressing gRNA and a nuclease for cleaving a predetermined site in a nucleic acid contained in a cell. The vector for expressing a nuclease based on the CRISPR/Cas system may contain a vector for expressing a gRNA and a vector for expressing Cas9 per one cleavage site. The vector for expressing a gRNA and Cas9 may contain both a gene encoding a gRNA and a gene encoding Cas9. Alternatively, the vector may be a vector set including a vector containing the gene encoding a gRNA and a vector containing the gene encoding Cas9. A plurality of vectors having different functions may be present in identical nucleic acid fragments or may be present in different nucleic acid fragments.

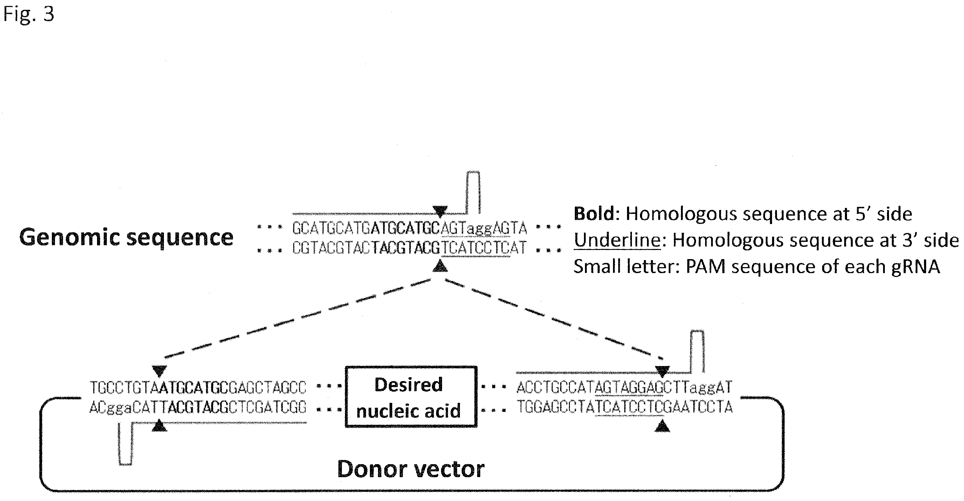

According to the eighth aspect, the present invention provides a method for inserting a desired nucleic acid into a predetermined site in a nucleic acid contained in a cell. The method according to the eighth aspect of the present invention comprises a step of introducing the vector according to any one of the first to sixth aspects of the present invention and the vector for expressing a nuclease into a cell. The vector for expressing a nuclease is, for example, a vector for expressing a nuclease including a first DNA binding domain and a second DNA binding domain as described above. Another example is a vector set including a first vector that contains a gene encoding a first nuclease subunit including a first DNA binding domain and a first DNA cleavage domain and a second vector that contains a gene encoding a second nuclease subunit including a second DNA binding domain and a second DNA cleavage domain. These vectors may be introduced into cells by allowing the vectors to be in contact with *ex vivo* cultured cells, or by administering the vectors into the living body and allowing the vectors to be indirectly in contact with cells present in the living body. These vectors can be introduced into the cells simultaneously or separately. In the case where these vectors are introduced separately into the cells, for example, a vector for expressing a nuclease may be previously introduced into a cell to produce a stable expression cell line or inducible expression cell line of the nuclease, and then, the vector according to any one of the first to sixth aspects of the present invention may be introduced into the produced stable expression cell line or inducible expression cell line. When the step of introduction into a cell is performed, a nuclease (such as the nuclease including the first DNA binding domain and the second DNA binding domain) functions in the cell, resulting in a nucleic acid fragment including a region formed of a first nucleotide sequence, a desired nucleic acid to be inserted into a cell and a region formed of a second nucleotide sequence in the stated order in the 5'-end to 3'-end direction from the vector. The step results in cleavage of a predetermined site in a nucleic acid in a cell. Thereafter, in the cell, the first nucleotide sequence in the nucleic acid in the cell and the first nucleotide sequence in the vector are joined by microhomology-mediated end joining (MMEJ), and the second nucleotide sequence in the nucleic acid in the cell and the second nucleotide sequence in the vector are joined by MMEJ. As a result, a desired nucleic acid is accurately inserted into a predetermined site of a nucleic acid of a cell. In the case of using the vector of the first aspect of the present invention, the nuclease for combination use specifically cleaves the moiety including the region formed of the first nucleotide sequence and the region formed of the second nucleotide sequence in the nucleic acid in the cell before insertion. However, the linked nucleic acid does not contain the moiety because of insertion of the desired nucleic acid. For example, in the case of using the nuclease based on the CRISPR/Cas system, all the gRNA target sequences lose the PAM sequence and the sequence of 3 bases adjacent to the PAM sequence after linkage. Thus, the nucleic acid is not cleaved again by the nuclease present in the cell and is stably retained. Insertion of the desired nucleic acid occurs at high frequency. In this regard, a combination of the vector of the first aspect of the present invention and the CRISPR/Cas system such that the linked nucleic acid loses the PAM sequence or the base adjacent to the PAM sequence can be appropriately designed with reference to the first and second nucleotide sequences included in both the vector and the nucleic acid in the cell as well as the sequences adjacent to these nucleotide sequences. An example of the design is illustrated in a schematic view in Fig. 3. In the case of using the vector of the second aspect of the present invention, the spacer region separating two DNA binding domains in the linked nucleic acid is shorter than that before linkage. Thus, the nucleic acid is not cleaved again by the nuclease present in the cell and is stably retained. Insertion of a desired nucleic acid occurs at high frequency. In this regard, the cleavage activity of the nuclease including a plurality of DNA binding domains depends on the length of the spacer region sandwiched between the regions recognized by the DNA binding domains. The nuclease specifically cleaves a spacer region having a specific length. In the linked nucleic acid, the spacer region separating two DNA binding domains consists of a nucleotide sequence of preferably from 1 to 20 bases in length, more preferably from 2 to 15 bases in length, and even more preferably from 3 to 10 bases in length.

In the present invention, the vector for inserting a desired nucleic acid into a predetermined site in a nucleic acid contained in a cell and the vector for expressing a nuclease may be identical to or different from each other. In the case of using the nuclease based on the CRISPR/Cas system, the vector for inserting a desired nucleic acid into a predetermined site in a nucleic acid contained in a cell, the vector for expressing a nuclease and the vector for expressing a gRNA may be identical to or different from one another.

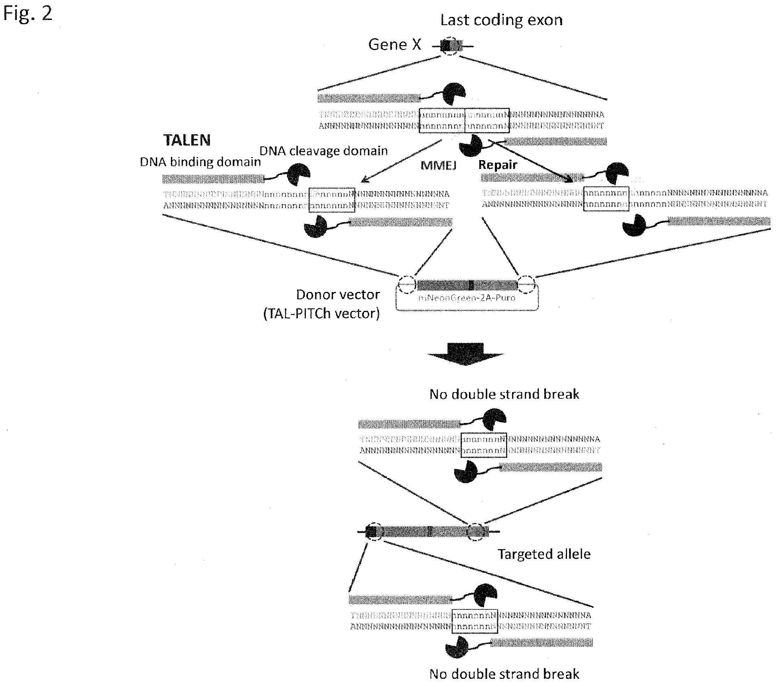

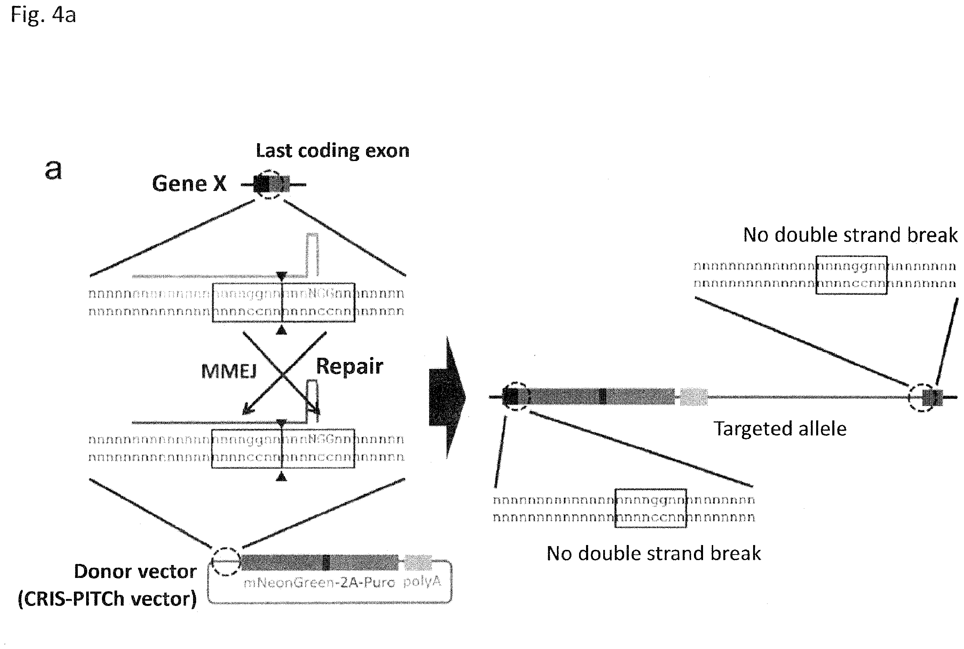

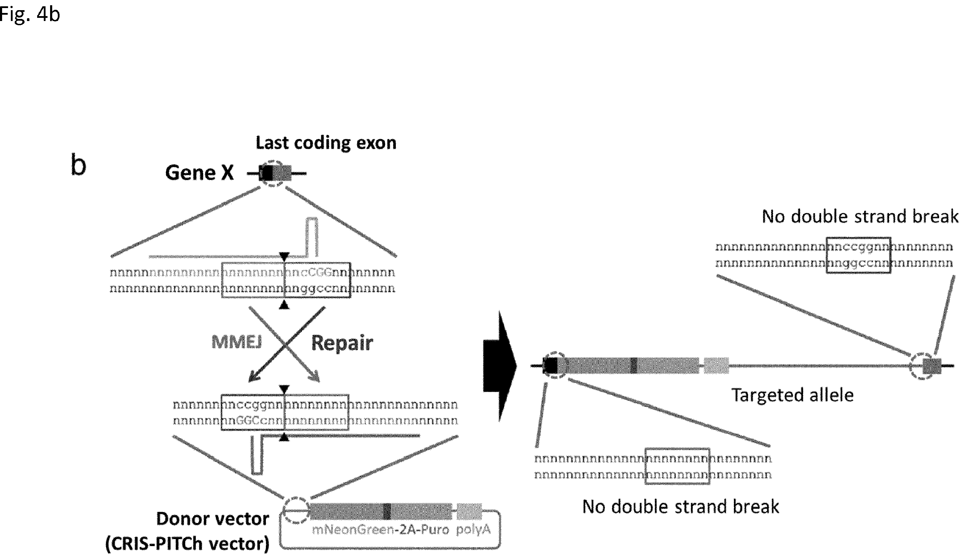

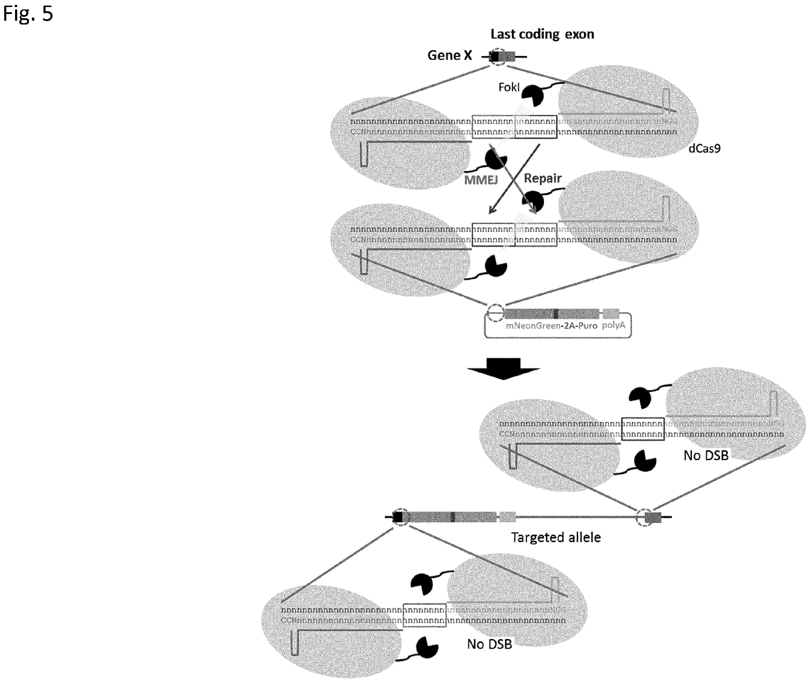

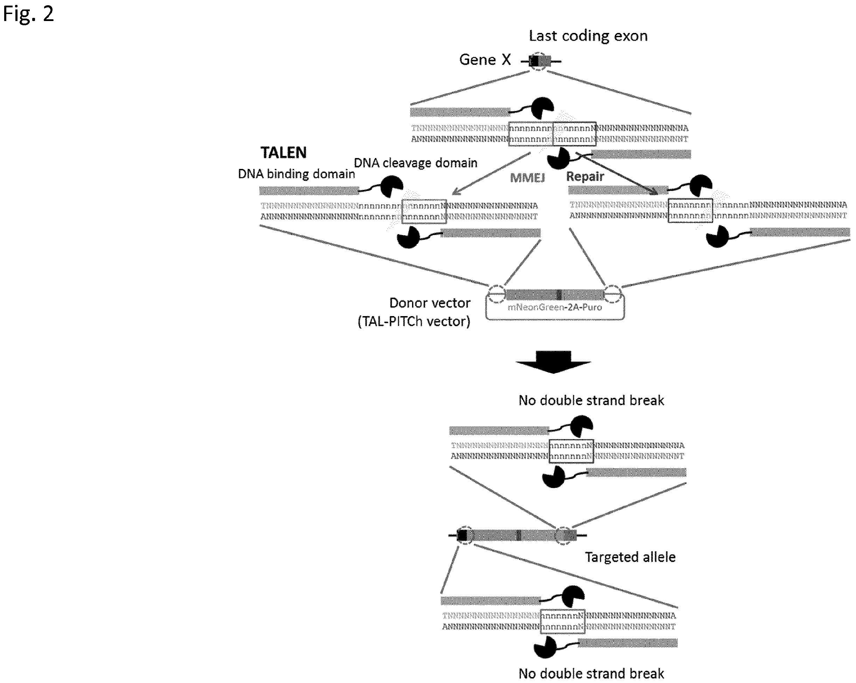

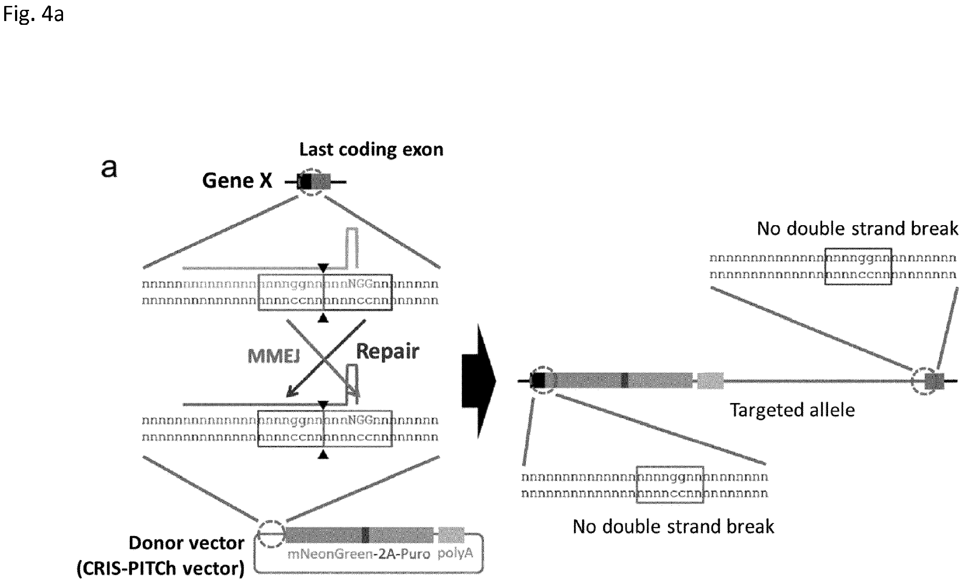

In the case where a desired nucleic acid is inserted using the vector of the present invention, a part of the vector containing a desired nucleic acid may be inserted into a predetermined site in a nucleic acid contained in a cell. Alternatively, the whole vector containing a desired nucleic acid may be inserted into a predetermined site in a nucleic acid contained in a cell. Fig. 1 is a schematic view of a case where the whole vector containing a desired nucleic acid is inserted using TALENs. Fig. 2 is a schematic view illustrating the case where a part of the vector containing a desired nucleic acid is inserted using TALENs. Fig. 3 is a schematic view of a case where a part of the vector containing a desired nucleic acid is inserted into a predetermined site in a nucleic acid contained in a cell using the CRISPR/Cas system. Figs. 4A and 4B are each a schematic view of a case where the whole vector containing a desired nucleic acid is inserted using the CRISPR/Cas system. Fig. 5 is a schematic view of a case where the whole vector containing a desired nucleic acid is inserted using FokI-dCas9.

According to the ninth aspect, the present invention provides a cell obtained by the method according to the eighth aspect of the present invention. The cell of the ninth aspect of the present invention can be obtained by performing the introduction step in the method of the eighth aspect and then selecting the cell with the nucleic acid inserted. For example, in the case where the nucleic acid to be inserted contains a gene encoding a specific reporter protein, selection of cells can be easily performed at high frequency by detecting the expression of the reporter protein and selecting the amount of the detected expression as an indicator.

According to the tenth aspect, the present invention provides an organism comprising the cell of the ninth aspect of the present invention. In the method of the eighth aspect of the present invention, in the case of administering the vectors into the living body and allowing the vectors to be indirectly in contact with cells present in the living body, the organism of the tenth aspect of the present invention is obtained.

According to the eleventh aspect, the present invention provides a method for producing an organism comprising a desired nucleic acid, comprising a step of differentiating a cell obtained by the method according to the eighth aspect of the present invention. In the method of the eighth aspect of the present invention, a cell comprising a desired nucleic acid is obtained by allowing a vector to be in contact with an ex vivo cultured cell, and differentiating the obtained cell to form an adult organism comprising a desired nucleic acid.

According to the twelfth aspect, the present invention provides an organism produced by the method according to the eleventh aspect of the present invention. The produced organism comprises a desired nucleic acid in a predetermined site of a nucleic acid contained in a cell in the organism, and can be used in various applications such as analysis of the functions of biological substances (e.g., genes, proteins, lipids and saccharides) depending on the function of the desired nucleic acid.

### Examples

Hereinafter, the present invention will be more specifically described with reference to examples, but the present invention is not limited thereto.

### Example 1

### Target Integration with TALEN

In this example, an expression cassette of a fluorescent protein gene was introduced (target integration) into Exon1 of a tyrosinase (tyr) gene of *Xenopus laevis* using the TALEN and the donor vector (TAL-PITCh vector).

### 1-1. Construction of TALEN:

The TALEN plasmid was constructed in the following manner. A vector constructed by In-Fusion cloning (Clontech Laboratories, Inc.) using pFUS_B6 vector (Addgene) as a template was mixed with a plasmid having a single DNA binding domain. By a Golden Gate reaction, 4 DNA binding domains were linked together (STEP1 plasmid). Thereafter, a vector constructed by In-Fusion cloning (Clontech Laboratories, Inc.) using pcDNA-TAL-NC2 vector (Addgene) as a template was mixed with the STEP1 plasmid. A TALEN plasmid was obtained by the second Golden Gate reaction. The full length sequence of the plasmid is shown in SEQ ID NOs: 1 and 2 (Left_TALEN) and SEQ ID NOs: 3 and 4 (Right_TALEN) of the Sequence Listing.

### 1-2. Construction of Donor Vector for Target Integration (TAL-PITCh Vector):

A plasmid having a modified TALEN sequence in which the first half (first nucleotide sequence) of the spacer of the tyrTALEN target sequence was replaced with the second half (second nucleotide sequence) thereof was constructed (Fig. 1). Inverse PCR was performed with a primer set that adds the above sequence (Xltyr-CMVEGFP-F+Xltyr-CMVEGFP-R; the sequence is shown in Table 1 as described later) using a pCS2/EGFP plasmid with GFP inserted into the ClaI and XbaI sites of pCS2+ as a template. Then, DpnI (New England Biolabs) was added to the PCR reaction solution and the template plasmid was digested. The purified reaction solution was subjected to self ligation, followed by subcloning. A plasmid was prepared from the clone in which accurate insertion was confirmed by sequence analysis and the plasmid was used as a donor vector (The sequence is shown in SEQ ID NOs: 5 and 6 of the Sequence Listing. In SEQ ID NO: 5, the nucleotide sequences 98 to 817 represent an ORF sequence of EGFP. This sequence is inserted into the ClaI/XbaI site of pCS2+. In SEQ ID NO: 5, the nucleotide sequences 1116 to 1167 represent a sequence recognized by the modified TALEN.).

### 1-3. Microinjection into Xenopus Laevis:

On the day preceding the experiment, human pituitary gonadotrophin (ASKA Pharmaceutical Co., Ltd.) was administered to the male Xenopus laevis and the female Xenopus laevis. The administered units were 150 units (for male) and 600 units (for female). On the next day, several drops of sperm suspension was added to the collected eggs and the eggs were artificially inseminated. After about 20 minutes, a 3% cysteine solution was added to allow the fertilized eggs to be dejellied. Then, the resulting eggs were washed several times with 0.1xMMR (ringer solution for amphibians) and transferred into 5% Ficoll/0.3xMMR. The tyrosinase TALEN mRNA mix (Left, Right 250 pg each) and donor vector (100 pg) constructed in the sections 1-1 and 1-2 were co-introduced into the fertilized eggs by the microinjection method (experimental group). As a negative control, only the TALEN mRNA Right (250 pg) and donor vector (100 pg) were co-introduced. Embryos were cultured at 20°C and transferred into 0.1xMMR at the blastula stage to facilitate their development.

### 1-4. Detection of Target Integration:

The embryos (at the tadpole stage) into which the TALEN and vector were co-introduced were observed under a fluorescence stereoscopic microscope and the presence or absence of GFP fluorescence was determined. A genomic DNA for each individual was extracted from the embryos of the control and experimental groups. The introduction of the donor vector into the target site was determined by PCR. The junctions between the genome and the 5'- or 3'-side of the vector were amplified by PCR using the primer set designed at the upstream and downstream of the TALEN target sequence and the vector side. The primer set of tyr-genomic-F and pCS2-R was used for the 5'-side, and the primer set of tyr-genomic-R and pCS2-F was used for the 3'-side (the sequence is shown in Table 1 as described later). After agarose electrophoresis confirmation, a band of the target size was cut out and subcloned into pBluescript SK. The inserted sequence was amplified by colony PCR, followed by analysis by direct sequencing. The sequencing was performed using CEQ-8000 (Beckman Coulter, Inc.).

### Result:

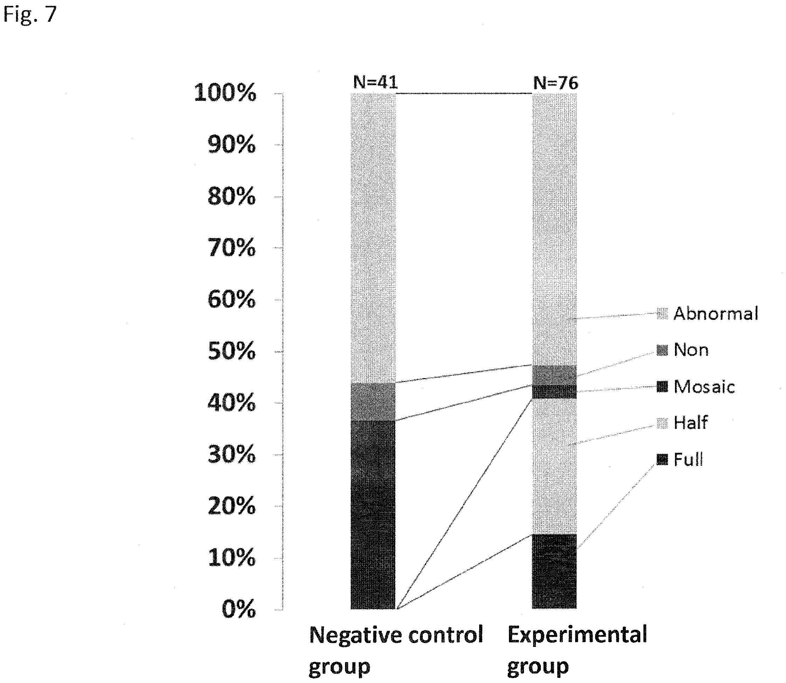

As for the embryos (at the tadpole stage) into which the donor vector was introduced, Figs. 6A and 6B shows phenotypes of the experimental group (TALEN mix + vector-injected embryo) and the negative control group (TALEN R + vector-injected embryo). In the experimental group, the tyr gene was broken and thus an albino phenotype was exhibited in the retinal pigment epithelium and melanophores. Additionally, many individuals generating strong GFP fluorescence throughout the body were observed (Fig. 6B) . No albino was observed in the negative control group. Individuals generating mosaic GFP fluorescence were partially observed (Fig. 6A). The ratio of the phenotypes in the experimental group and the negative control group was classified into four groups: Full: the individuals in which GFP fluorescence is observed in the whole body; Half: the individuals in which half of the right or left side has fluorescence; Mosaic: the individuals with mosaic fluorescence; and Non: the individuals in which GFP fluorescence is not observed (Fig. 7). The individuals of Full and Half were not observed in the negative control group, meanwhile, about 20% of the survived individuals exhibited phenotypes of Full and about 50% of the survived individuals exhibited phenotypes of Half in the experimental group.

Subsequently, a genomic DNA was respectively extracted from 5 tadpoles exhibiting phenotypes of Full and 3 individuals of the negative control group observed in Fig. 6, followed by genotyping. In order to confirm the inserted portion on the genome and the junction of the vector, the junctions between the target site and the 5'- or 3'-side of the donor vector were amplified by PCR using the primer set designed at the upstream and downstream of the tyrTALEN target sequence and the vector side (Fig. 8). The PCR products were subjected to electrophoresis and bands having an estimated size were confirmed in the experimental group Nos. 1, 3 and 4 (at the 5'-side) and the experimental group Nos. 2, 3 and 4 (at the 3'-side) (Fig. 8, indicated by arrows). On the other hand, no PCR product was confirmed in the negative control group. Then, in order to examine the sequence of the junctions, the PCR products at the 5'- and 3'-sides detected in Nos. 3 and 4 were subcloned, followed by sequence analysis. As a result, the sequence expected in the case of being joined by MMEJ was confirmed at a ratio of 100% (5/5 clone) in the junction at the 5'-side in No. 3, meanwhile, the sequence expected was confirmed at a ratio of 80% (4/5 clone) in the junction at the 3'-side (Fig. 9A). The sequence with 10 bases deleted or 3 bases inserted was confirmed in the junction at the 5'-side in No. 4, meanwhile, the sequence expected was confirmed at a ratio of 100% (3/3) in the junction at the 3'-side (Fig. 9B).

The sequences of the primers used in the sections 1-1 to 1-4 are shown in Table 1 below.

**[Table 1]**

| SEQ ID NO: | Primer name | Sequence (from 5' to 3') |
|---|---|---|
| 7 | Xltyr-CMVEGFP-F | AACATGAGAGCTCACGGAGATGAGTGCGCGCTTG GCGTAATCAT |
| 8 | Xltyr-CMVEGFP-R | TTCTGAATTCCCAGTGCAGCAAGAAGTATTAACCC TCACTAAAGGGA |
| 9 | tyr-genomic-F | GGAGAGGATGGCCTCTGGAGAGATA |
| 10 | tyr-genomic-R | GGTGGGATGGATTCCTCCCAGAAG |
| 11 | pCS2-F | ATAAGATACATTGATGAGTTTGGAC |
| 12 | pCS2-R | ATGCAGCTGGCACGACAGGTTTCCC |

### Example 2

### Target Integration into HEK293T Cell Using CRISPR/Cas9 System

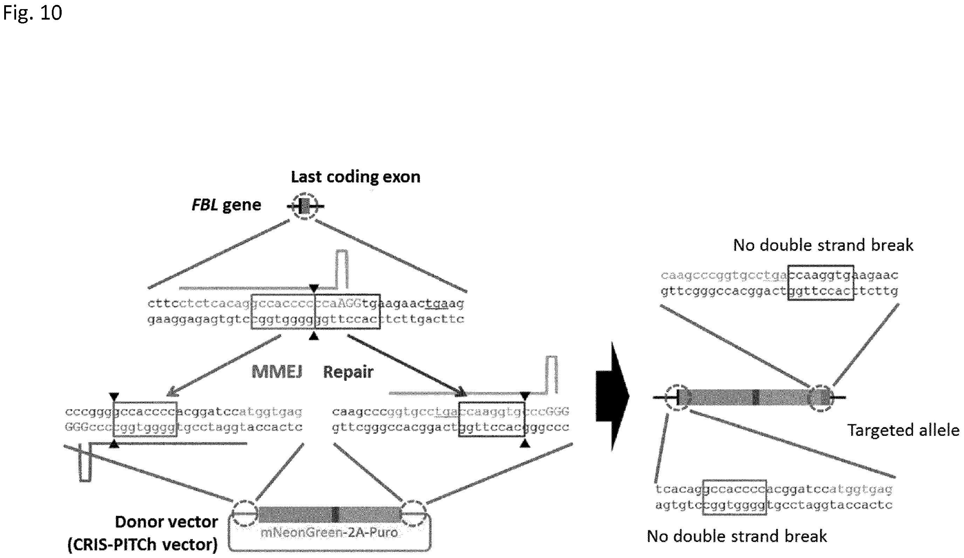

In this example, a fluorescent protein gene expression cassette was introduced (target integration) into the last coding exon of fibrillarin (FBL) gene in a HEK293T cell using the CRISPR/Cas9 system. The outline of this example is illustrated in Fig. 10. Briefly, the vector expressing three types of gRNAs indicated in orange, red and green in Fig. 10 and Cas9 and the donor vector (CRIS-PITCh vector) were co-introduced into the HEK293T cell and the resulting cell was selected by puromycin. Thereafter, DNA sequencing and fluorescent observation were carried out.

### 2-1. Construction of Vector Expressing gRNA and Cas9:

A vector simultaneously expressing three types of gRNAs, and Cas9 was constructed as described in SCIENTIFIC REPORTS 2014 Jun 23; 4: 5400. doi: 10.1038/srep05400. Briefly, the pX330 vector (Addgene; Plasmid 42230) was modified so that a plurality of gRNA expression cassettes could be linked by a Golden Gate reaction. The annealed synthetic oligonucleotides were inserted into three types of modified pX330 vectors. Specifically, oligonucleotides 13 and 14 were annealed to each other to produce a synthetic oligonucleotide for forming a genome cleavage gRNA (indicated in orange in Fig. 10). Further, oligonucleotides 15 and 16 were annealed to each other to produce a synthetic oligonucleotide for forming a genome cleavage gRNA at the 5'-side of the donor vector (indicated in red in Fig. 10). Further, oligonucleotides 17 and 18 were annealed to each other to produce a synthetic oligonucleotide for forming a genome cleavage gRNA at the 3'-side of the donor vector (indicated in green in Fig. 10). Each of the produced synthetic oligonucleotides was inserted into each of the plasmids and then the vectors were integrated by a Golden Gate reaction, and a vector simultaneously expressing three types of gRNAs, and Cas9 was obtained.

### 2-2. Construction of Donor Vector for Target Integration (CRIS-PITCh Vector):

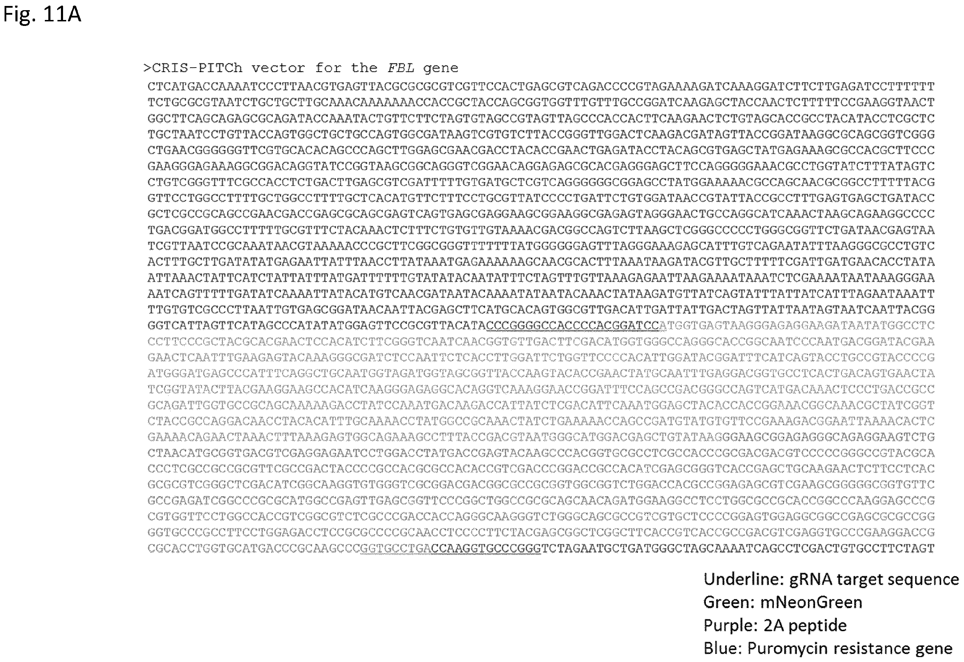

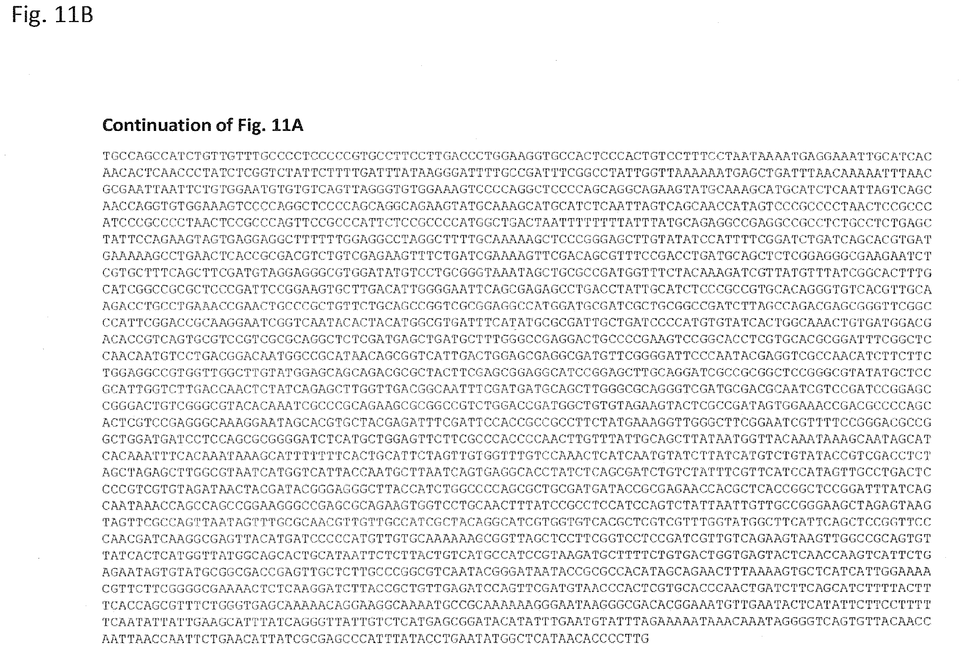

The CRIS-PITCh vector was constructed in the following manner. While a CMV promoter on the vector based on pCMV (Stratagene) was removed, In-Fusion cloning was used to construct a vector such that the gRNA target sequence at the 5'-side, the mNeonGreen coding sequence, the 2A peptide coding sequence, the puromycin resistance gene coding sequence and the gRNA target sequence at the 3'-side were aligned in this order. Figs. 11A and 11B show the full length sequence (SEQ ID NO: 23) of the constructed vector. In Figs. 11A and 11B, the mNeonGreen coding sequence is indicated in green (nucleotides 1566 to 2273 of SEQ ID NO: 23), the 2A peptide coding sequence is indicated in purple (nucleotides 2274 to 2336 of SEQ ID NO: 23), and the puromycin resistance gene coding sequence is indicated in blue (nucleotides 2337 to 2936 of SEQ ID NO: 23). The gRNA target sequences at the 5'- and 3'-sides are underlined.

### 2-3. Introduction into HEK293T Cells:

Introduction into HEK293T cells was performed in the following manner. HEK293T cells were cultured in 10% fetal bovine serum-containing Dulbecco's modified Eagle's medium (DMEM). The cultured cells were seeded at a density of 1 x 10⁵ cells per well on a 6-well plate on the day before the introduction of plasmids. In the introduction of plasmids, 400 ng of a vector expressing a gRNA and Cas9 and 200 ng of a CRIS-PITCh vector were introduced using Lipofectamine LTX (Life Technologies). After the introduction of plasmids, the cells were cultured in a drug-free medium for 3 days and then cultured in a culture medium containing 1 µg/mL of puromycin for 6 days. Thereafter, the cultured cells were single-cell cloned on a 96-well plate by limiting dilution.

### 2-4. Detection of Target Integration:

The HEK293T cell into which the vector expressing a gRNA and Cas9 and the CRIS-PITCh vector were co-introduced was observed using a confocal laser scanning microscope, and the presence or absence of fluorescence was determined. Then, the genomic DNA was extracted from a clone of puromycin resistant cells and the introduction of the donor vector into the target site was confirmed. The junctions between the genome and the 5'- or 3'-side of the vector were amplified by PCR using the primer set designed at the upstream and downstream of the CRISPR target sequence. The primer set of primers 19 and 20 was used for the 5'-side, and the primer set of primers 21 and 22 was used for the 3'-side (the sequence is shown in Table 2 as described later). After agarose electrophoresis confirmation, a band of the target size was cut out and analyzed by direct sequencing. The sequencing was performed using ABI 3130xl Genetic analyzer (Life Technologies).

### Result:

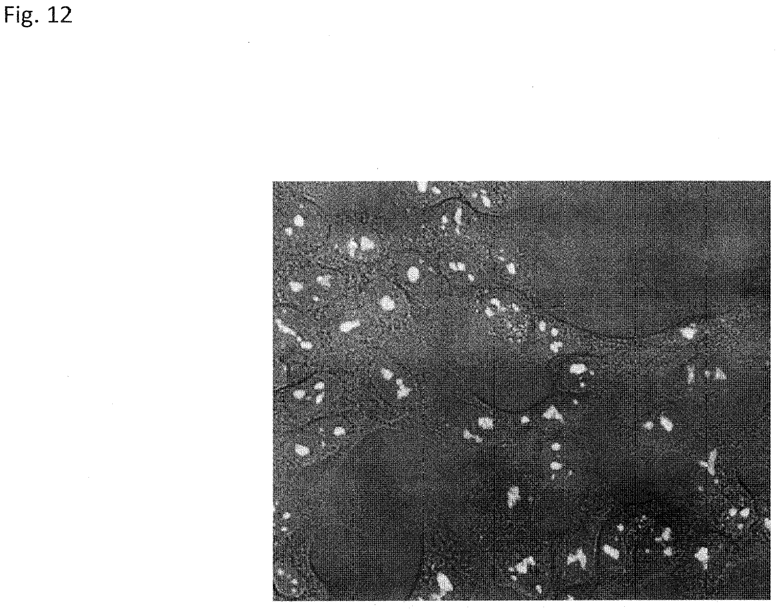

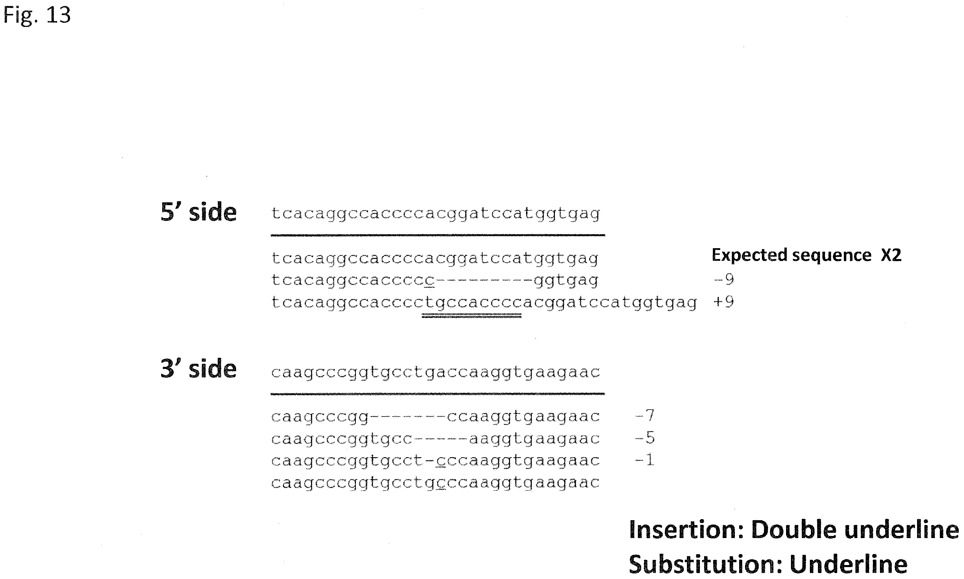

The result observed with the confocal laser scanning microscope is shown in Fig. 12. FBL is a protein specific to nucleoli. Accordingly, in the case where the target integration of the fluorescent protein gene to the FBL gene is successful, the fluorescent protein is localized in the nucleoli. As shown in Fig. 12, a fluorescence image corresponding to the localization pattern (nucleoli) of the FBL protein was obtained. Subsequently, the sequences of the junctions between the genome and the 5'- or 3'-side of the introduced vector were examined. As a result, the sequence expected when the junction at the 5'-side was joined by MMEJ was present at a ratio of 50% (2/4 clone). The remaining two clones had 9 bases deleted or inserted (Fig. 13). The completely expected sequence in the junction at the 3'-side was present at 0% (0/4 clone), but the sequence in which only one base was substituted was present (1 clone) . In addition, it was confirmed that one clone had one base deleted, one clone had 5 bases deleted, and one clone had 7 bases deleted (Fig. 13). Similarly, when the fluorescent protein gene expression cassette was introduced into a β-actin (ACTB) locus of HCT116 cells using the CRISPR/Cas9 system (target integration), the same result as that of the target integration into the HEK293T cell was obtained.

The sequences of the oligonucleotides used in the sections 2-1 to 2-4 are shown in Table 2 below.

**[Table 2]**

| SEQ ID NO: | Name | Sequence (from 5' to 3') |
|---|---|---|
| 13 | Oligonucleotide 13 | CACCGCTCTCACAGGCCACCCCCCA |
| 14 | Oligonucleotide 14 | AAACTGGGGGGTGGCCTGTGAGAGC |
| 15 | Oligonucleotide 15 | CACCGTGGATCCGTGGGGTGGCCCC |
| 16 | Oligonucleotide 16 | AAACGGGGCCACCCCACGGATCCAC |
| 17 | Oligonucleotide 17 | CACCGGTGCCTGACCAAGGTGCCC |
| 18 | Oligonucleotide 18 | AAACGGGCACCTTGGTCAGGCACC |
| 19 | Primer 19 | ACACCAAGACAGACATCTCTGTCCCTTG |
| 20 | Primer 20 | ATCCGTATCCAATGTGGGGAAC |
| 21 | Primer 21 | CCGCAACCTCCCCTTCTACGAG |
| 22 | Primer 22 | TCAGCAGGTCAAGGGGAGGAATG |

## Claims

1. A vector for inserting a desired nucleic acid into a predetermined site in a nucleic acid contained in a cell by a nuclease,
wherein the nucleic acid contained in the cell includes a region formed of a first nucleotide sequence, the predetermined site, and a region formed of a second nucleotide sequence in the stated order in a 5'-end to 3'-end direction,
wherein the nuclease specifically cleaves a moiety including the region formed of the first nucleotide sequence and the region formed of the second nucleotide sequence included in the cell,
wherein the vector includes a region formed of a first nucleotide sequence, the desired nucleic acid, and a region formed of a second nucleotide sequence in the stated order in a 5'-end to 3'-end direction,
wherein the vector produces a nucleic acid fragment including the region formed of the first nucleotide sequence, the desired nucleic acid, and the region formed of the second nucleotide sequence in the stated order in the 5'-end to 3'-end direction by the nuclease, and
wherein the first nucleotide sequence in the nucleic acid contained in the cell and the first nucleotide sequence in the vector are joined by microhomology-mediated end joining, and the second nucleotide sequence in the nucleic acid contained in the cell and the second nucleotide sequence in the vector are joined by microhomology-mediated end joining, whereby the desired nucleic acid is inserted.

2. The vector according to claim 1,
wherein the nuclease includes a first DNA binding domain and a second DNA binding domain,
wherein the region formed of the first nucleotide sequence, the predetermined site and the region formed of the second nucleotide sequence in the nucleic acid contained in the cell are each located between a region formed of a nucleotide sequence recognized by the first DNA binding domain and a region formed of a nucleotide sequence recognized by the second DNA binding domain, and
wherein the region formed of the first nucleotide sequence and the region formed of the second nucleotide sequence in the vector are each located between the region formed of the nucleotide sequence recognized by the first DNA binding domain and the region formed of the nucleotide sequence recognized by the second DNA binding domain.

3. The vector according to claim 2, wherein the nuclease is a homodimeric nuclease and the vector is a circular vector.

4. The vector according to claim 1, wherein the nuclease is a Cas9 nuclease.

5. The vector according to claim 2, wherein the nuclease is a TALEN.

6. The vector according to any one of claims 1 to 5, wherein the first nucleotide sequence is from 3 to 10 bases in length and the second nucleotide sequence is from 3 to 10 bases in length.

7. A kit for inserting a desired nucleic acid into a predetermined site in a nucleic acid contained in a cell, comprising the vector according to any one of claims 1 to 6 and a vector for expressing a nuclease.
